# EUROPEAN PATENT APPLICATION

(11) **EP 4 516 300 A2**
(43) Date of publication of application: **05.03.2025**
(21) Application number: 25150456.9
(22) Date of filing: 15.06.2020
(51) Int. Cl.: A61K 31/345

(54) **RATIONAL THERAPEUTIC TARGETING OF ONCOGENIC IMMUNE SIGNALING STATES IN MYELOID MALIGNANCIES VIA THE UBIQUITIN CONJUGATING ENZYME UBE2N**

(30) Priority: 14.06.2019 US 201962861711 P
(62) Divisional of application: 20822638.1
(71) Applicant: Children's Hospital Medical Center, Cincinnati, OH 45229-3039 (US)
(72) Inventor: Starczynowski, Daniel, Cincinnati, OH, 45236 (US); Barreyro, Laura, Cincinnati, OH, 45236 (US); Seibel, William, Liberty Township, OH, 45011 (US)
(74) Representative: Forresters IP LLP

(57) **Abstract**

Methods and compositions disclosed herein generally relate to compositions and methods for suppressing hematopoietic stem and progenitor cells (HSPCs) and the treatment of diseases or disorders involving UBE2N, such as cancers, including disorders such as myelodysplastic syndrome (MDS) and acute myeloid leukemia (AML) and chronic inflammatory disorders. Particular aspects relate to treating, e.g. acute myelomonocytic leukemia (AML-M4) and acute monocytic leukemia (AML-M5). Particular aspects of the invention relate to determining an individual in need of treatment who can be treated with a UBE2N inhibitor, such as an individual having AML-M4 and/or AML-M5. The invention further relates to using a UBE2N inhibitor to treat a disease or disorder characterized by malignant hematopoietic cells, as well as other cancers, and chronic inflammatory disorders, and as immune checkpoint regulators.

## Description

### CROSS REFERENCE TO RELATED APPLICATION

The present application claims the benefit of priority under 35 U.S.C. § 119(e) to U.S. Provisional Application No. 62/861,711, RATIONAL THERAPEUTIC TARGETING OF ONCOGENIC IMMUNE SIGNALING STATES IN MYELOID MALIGNANCIES VIA THE UBIQUITIN CONJUGATING ENZYME UBE2N, filed on June 14, 2019, which is currently co-pending herewith and which is incorporated by reference in its entirety.

### FIELD OF THE INVENTION

The invention disclosed herein generally relates to modulating UBE2N-dependent signaling states in hematopoietic stem and progenitor cells (HSPCs), particularly to inhibiting UBE2N immune signaling in leukemic hematopoietic stem and HSPCs, and even more particularly to methods for treatment of diseases, such as hematopoietic cancer, solid tumors, and chronic inflammatory disorders, and other disorders, comprising modulating UBE2N-dependent immune signaling states by administration of UBE2N inhibitors (optionally in combination or adjunctively with other therapeutic agents) to a subject having such a disease.

### BACKGROUND

Acute myeloid leukemia (AML) is a heterogeneous hematopoietic malignancy characterized by ineffective hematopoiesis, and overproduction of immature myeloid cells (blasts) associated with a differentiation block. Despite significant effort, patients with AML continue to have poor outcomes, with a five-year relative survival of 25% [1].

AML originates in hematopoietic stem and progenitor cells (HSPC) that acquire genetic or epigenetic abnormalities [2, 3]. Current chemotherapy regimens and targeted therapies do not fully eradicate the leukemic HSPC [4], thus leading to disease relapse. Therefore, there is an urgent need to identify molecular features of leukemic HSPC that are amenable to therapeutic intervention, which can also apply to HSPCs implicated in myelodysplastic syndromes (MDS), solid tumors, and chronic inflammation.

### SUMMARY OF THE INVENTION

Embodiments of the invention are directed to methods of treating a subtype of acute myeloid leukemia (AML) responsive to UBE2N inhibition, the method including: identifying a subject having one or more subtype of AML responsive to UBE2N inhibition, wherein the AML subtype comprises acute myelomonocytic leukemia (AML-M4) and/or acute monocytic leukemia (AML-M5); and providing to the subject one or more administrations of one or more compositions comprising a UBE2N inhibitor; and wherein administration of the UBE2N inhibitor results in treating the subtype of acute myeloid leukemia (AML) responsive to UBE2N inhibition in the subject. In some embodiments, treating includes modulating UBE2N-mediated immune signaling.

In some embodiments, the AML subtype includes AML-M4. In some embodiments, the AML subtype includes AML-M5.

In some embodiments of the methods, the UBE2N inhibitor can be a small molecule. In some embodiments, the UBE2N inhibitor can be at least one selected from NSC697923 (2-(4-methylphenyl)sulfonyl-5-nitrofuran), UC-764864 (1-(4-ethylphenyl)-3-[(6-methyl-1H-benzimidazol-2-yl) sulfanyl]prop-2-en-1-one), UC-764865 (1-(4-methoxyphenyl)-3-[(6-methyl-1H-benzimidazol-2-yl) sulfanyl]prop-2-en-1-one), and UC-764865 (1-(4-methylphenyl)-3-[(6-methyl-1H-benzimidazol-2-yl) sulfanyl]prop-2-en-1-one), and pharmaceutically-acceptable salts, cocrystals, hydrates, solvates, optical isomers, geometric isomers, salts of isomers, prodrugs, and derivatives thereof.

In some embodiments, administration of the UBE2N inhibitor to the subject decreases the incidence of one or more symptoms associated with AMI,-M4 and/or AML-M5 or decreases one or more markers of viability of AMI,-M4 and/or AML-M5 cells. In some embodiments, the one or more symptoms associated with AML-M4 and/or AML-M5 can include decreasing marrow failure, immune dysfunction, transformation to overt leukemia, or a combination thereof in the subject, or wherein the marker of viability of AML-M4 and/or AML-M5 cells comprises survival over time, proliferation, growth, migration, formation of colonies, chromatic assembly, DNA binding, RNA metabolism, cell migration, cell adhesion, inflammation, or a combination of two or more thereof.

In some embodiments, the method further includes administration of a composition including a BCL2 inhibitor. In some embodiments, the BCL2 inhibitor can include venetoclax, or a salt, isomer, derivative or analog thereof. In some embodiments, the administration of a composition including a BCL2 inhibitor can occur concurrently with or after administration of the UBE2N inhibitor. In some embodiments of the methods, the subject has been treated previously with one or more BCL2 inhibitor. In some embodiments, administration of the UBE2N inhibitor resensitizes the subject to the BCL2 inhibitor and/or otherwise enhances the effectiveness of the administration of the BCL2 inhibitor.

In some embodiments, the method further includes administration of one or more chemotherapy, and/or one or more apoptotic agent, immune modulating agent, and/or epigenetic modifying agent. In some embodiments, the chemotherapy includes one or more selected from the group consisting of a taxane, a platinum-based agent, an anthracycline, an alkylating agent, a vinca alkaloid, an epothilone, a histone deacetylase inhibitor, a topoisomerase I and II inhibitor, a kinase inhibitor, a nucleotide analog, a precursor analog, a peptide antibiotic, and combinations thereof. In some embodiments, the method further includes administration of a CUL4-CRBN E3 ligase complex inhibitor. In some embodiments, the CUL4-CRBN E3 ligase complex inhibitor includes lenalidomide.

In some embodiments, at least one of the one or more administrations includes parenteral administration, a mucosal administration, intravenous administration, subcutaneous administration, topical administration, intradermal administration, oral administration, sublingual administration, intranasal administration, or intramuscular administration. In some embodiments, if there is more than one administration at least one composition used for at least one administration can be different from the composition of at least one other administration. In some embodiments, the compound of at least one of the one or more compositions can be administered to the subject in an amount of from about 0.005 mg/kg animal body weight to about 50 mg /kg animal body weight. In some embodiments, the subject is a mammal, preferably wherein the subject can be a human, a rodent, or a primate. In some embodiments, the subject can be enrolled in a clinical trial.

Further embodiments of the invention include methods of identifying a subject having acute myeloid leukemia (AML) suitable for treatment with a UBE2N inhibitor, the method including: determining whether the subject has one or more subtype of AML responsive to UBE2N inhibition, wherein the AML subtype includes acute myelomonocytic leukemia (AML-M4) and/or acute monocytic leukemia (AML-M5); assigning the subject to a first treatment cohort where the subject has an AML subtype including AMI,-M4 and/or AML-M5, wherein the first treatment cohort can be treatable by administration of an UBE2N inhibitor, or assigning the subject to a second treatment cohort where the subject does not have an AML subtype including AMI,-M4 and/or AML-M5, wherein the second treatment cohort can be not treatable, or can be less effectively treatable by administration of an UBE2N inhibitor. In some embodiments, determining whether the subject has one or more subtype of AML responsive to UBE2N inhibition includes obtaining a sample from the subject, and analyzing the sample to determine whether the subject has AML-M4 or AML-M5. In some embodiments, the AML subtype includes AML-M4. In some embodiments, the AML subtype includes AML-M5. In some embodiments, the methods further include treating the subject with a UBE2N inhibitor if the subject has an AML subtype including AMI,-M4 and/or AML-M5, or treating the subject with a therapy excluding a UBE2N inhibitor if the subject does not have an AML subtype including AML-M4 and/or AML-M5.

Further embodiments of the invention relate to methods of treating a chronic inflammatory condition responsive to UBE2N inhibition, the method including: identifying a subject having one or more chronic inflammatory condition responsive to UBE2N inhibition; and providing to the subject one or more administrations of one or more compositions including a UBE2N inhibitor; and wherein administration of the UBE2N inhibitor results in treating the chronic inflammatory condition responsive to UBE2N inhibition in the subject.

Further embodiments of the invention relate to methods of treating a hematologic malignancy and/or solid tumor responsive to UBE2N inhibition, the method including: identifying a subject having a hematologic malignancy and/or solid tumor responsive to UBE2N inhibition; and providing to the subject one or more administrations of one or more compositions including a UBE2N inhibitor; and wherein administration of the UBE2N inhibitor results in treating the hematologic malignancy and/or solid tumor responsive to UBE2N inhibition in the subject. In some embodiments, the disease or disorder includes diffuse large B cell lymphoma, neuroblastoma, breast cancer, metastatic colorectal cancer, hepatocarcinoma, ovarian cancer, breast cancer, cervical cancer, colorectal cancer, endometrial cancer, glioma, head and neck cancer, liver cancer, melanoma, ovarian cancer, pancreatic cancer, prostate cancer, renal cancer, stomach cancer, testicular cancer, thyroid cancer, or urothelial cancer, or a combination of two or more thereof.

### BRIEF DESCRIPTION OF THE DRAWINGS

Those of skill in the art will understand that the drawings, described below, are for illustrative purposes only. The drawings are not intended to limit the scope of the present teachings in any way.
Figure 1 depicts dysregulation of innate immune signaling in myeloid malignancies.
Figure 2 demonstrates that dysregulation of UBE2N-dependent innate immune pathways is associated with AML HSPC.
Figure 3 describes knockdown of UBE2N in leukemic and normal hematopoietic cells.
Figure 4 demonstrates that UBE2N expression is required for leukemic cell function.
Figure 5 depicts identification of UBE2N inhibitors.
Figure 6 depicts interaction of UC-764864/65 with UBE2N.
Figure 7 demonstrates that UC-764864 suppresses UBE2N enzymatic activity and innate immune signaling.
Figure 8 depicts proteomic analysis and evaluation of ubiquitin posttranslational modifications induced by UC-764864.
Figure 9 demonstrates that inhibition of UBE2N catalytic function suppresses AML *in vitro.*
Figure 10 depicts characterization of UC-764865.
Figure 11 demonstrates that inhibition of UBE2N catalytic function suppresses AML *in vivo.*
Figure 12 depicts *in vivo* pharmacokinetic properties and toxicity of UC-764865.
Figure 13 demonstrates that baseline oncogenic immune signaling states in AML confer sensitivity to inhibition of UBE2N catalytic function.
Figure 14 demonstrates the effects of UC-764864 on UBE2N-dependent signaling in AML.
Figure 15 demonstrates the effects of UC-764864 on UBE2N-dependent signaling in AML.
Figure 16 demonstrates the effects of UC-764864 on UBE2N-dependent signaling in AML. Heatmap of individual mutations or AML subtype in AML patient samples from TCGA clustered in Group 1 (UBE2N-dependent signature low) and Group 2 (UBE2N-dependent signature high).

### DETAILED DESCRIPTION OF THE INVENTION

Unless otherwise noted, terms are to be understood according to conventional usage by those of ordinary skill in the relevant art.

The following documents are incorporated by reference herein in their entirety and for all purposes: U.S. Patent Application No. 62/414,058, Overexpression of U2AF1 as a Genetic Predictor of Activated IRAK, filed October 28, 2016; U.S. Patent Application No. 62/429,289, Overexpression of U2AF1 as a Genetic Predictor of Activated IRAK, filed December 02, 2016; U.S. Patent Application No. 16/339,692, TREATMENT OF DISEASES ASSOCIATED WITH ACTIVATED IRAK, filed April 04, 2019; International Patent Application No. PCT/US2017/059091, TREATMENT OF DISEASES ASSOCIATED WITH ACTIVATED IRAK, filed October 30, 2017; U.S. Patent Application No. 61/826,211, Combination Therapy for MDS, filed May 22, 2013; U.S. Patent No. 9,168,257, Combination Therapy for MDS, issued October 27, 2015; U.S. Patent No. 9,504,706, Combination Therapy for MDS, issued November 29, 2016; U.S. Patent No. 9,855,273, Combination Therapy for MDS, issued January 02, 2018; International Patent Application No. PCT/US2014/039156, Combination Therapy for MDS, filed May 22, 2014; U.S. Patent Application No. 62/375,965 Compounds, Compositions, Methods for Treating Diseases, and Methods for Preparing Compounds, filed August 17, 2016; U.S. Patent Application No. 16/326,571, COMPOUNDS, COMPOSITIONS, METHODS FOR TREATING DISEASES, AND METHODS FOR PREPARING COMPOUNDS, filed February 19, 2019; U.S. Patent Application No. 16/804,518, COMPOUNDS, COMPOSITIONS, METHODS FOR TREATING DISEASES, AND METHODS FOR PREPARING COMPOUNDS, filed February 28, 2020; International Patent Application No. PCT/US2017/047088, Compounds, Compositions, Methods for Treating Diseases, and Methods for Preparing Compounds, filed August 16, 2017; U.S. Patent Application No. 62/248,050, Methods and Compositions for the Treatment of Head and Neck Cancer, filed October 29, 2015; U.S. Patent No. 10,487,329, Methods and Compositions for the Treatment of Head and Neck Cancer, issued November 26, 2019; International Patent Application No. PCT/US2016/058864, Methods and Compositions for the Treatment of Head and Neck Cancer, filed October 26, 2016; U.S. Patent Application No. 62/812,948, COMPOUNDS, COMPOSITIONS, METHODS FOR TREATING DISEASES, AND METHODS FOR PREPARING COMPOUNDS, filed March 01, 2019; U.S. Patent Application No. 62/812,954, filed March 01, 2019, METHODS, COMPOUNDS, AND COMPOSITIONS FOR THE TREATMENT OF HEAD AND NECK CANCER.

As used herein, the term "sample" encompasses a sample obtained from a subject or patient. The sample can be of any biological tissue or fluid. Such samples include, but are not limited to, sputum, saliva, buccal sample, oral sample, blood, serum, mucus, plasma, urine, blood cells (*e.g.,* white cells), circulating cells (*e.g.* stem cells or endothelial cells in the blood), tissue, core or fine needle biopsy samples, cell-containing body fluids, free floating nucleic acids, urine, stool, peritoneal fluid, and pleural fluid, tear fluid, or cells therefrom. Samples can also include sections of tissues such as frozen or fixed sections taken for histological purposes or microdissected cells or extracellular parts thereof. A sample to be analyzed can be tissue material from a tissue biopsy obtained by aspiration or punch, excision or by any other surgical method leading to biopsy or resected cellular material. Such a sample can comprise cells obtained from a subject or patient. In some embodiments, the sample is a body fluid that include, for example, blood fluids, serum, mucus, plasma, lymph, ascitic fluids, gynecological fluids, or urine but not limited to these fluids. In some embodiments, the sample can be a non-invasive sample, such as, for example, a saline swish, a buccal scrape, a buccal swab, and the like.

As used herein, "blood" can include, for example, plasma, serum, whole blood, blood lysates, and the like.

As used herein, the term "assessing" includes any form of measurement, and includes determining if an element is present or not. The terms "determining," "measuring," "evaluating," "assessing," "analyzing," and "assaying" can be used interchangeably and can include quantitative and/or qualitative determinations.

As used herein, the term "monitoring" with reference to a type of cancer refers to a method or process of determining the severity or degree of the type of cancer or stratifying the type of cancer based on risk and/or probability of mortality. In some embodiments, monitoring relates to a method or process of determining the therapeutic efficacy of a treatment being administered to a patient.

As used herein, "outcome" can refer to an outcome studied. In some embodiments, "outcome" can refer to survival / mortality over a given time horizon. For example, "outcome" can refer to survival / mortality over 1 month, 3 months, 6 months, 1 year, 5 years, or 10 years or longer. In some embodiments, an increased risk for a poor outcome indicates that a therapy has had a poor efficacy, and a reduced risk for a poor outcome indicates that a therapy has had a good efficacy.

As used herein, the term "high risk clinical trial" refers to one in which the test agent has "more than minimal risk" (as defined by the terminology used by institutional review boards, or IRBs). In some embodiments, a high risk clinical trial is a drug trial.

As used herein, the term "low risk clinical trial" refers to one in which the test agent has "minimal risk" (as defined by the terminology used by IRBs). In some embodiments, a low risk clinical trial is one that is not a drug trial. In some embodiments, a low risk clinical trial is one that that involves the use of a monitor or clinical practice process. In some embodiments, a low risk clinical trial is an observational clinical trial.

As used herein, the terms "modulated" or "modulation," or "regulated" or "regulation" and "differentially regulated" can refer to both up regulation (*i.e.,* activation or stimulation, *e.g.,* by agonizing or potentiating) and down regulation (*i.e.,* inhibition or suppression, *e.g.,* by antagonizing, decreasing or inhibiting), unless otherwise specified or clear from the context of a specific usage.

As used herein, the term "subject" refers to any member of the animal kingdom having cells (e.g., hematopoietic stem and/or progenitor cells (HSPCs)) responsive to UBE2N inhibitors. In some embodiments, a subject is a mammalian (e.g., human, etc.) patient. In some embodiments, a subject is a pediatric patient (e.g., a human pediatric patient). In some embodiments, a pediatric patient is a patient under 18 years of age, while an adult patient is 18 or older.

As used herein, the terms "treatment," "treating," "treat," and the like, refer to obtaining a desired pharmacologic and/or physiologic effect. The effect can be prophylactic in terms of completely or partially preventing a disease or symptom thereof and/or can be therapeutic in terms of a partial or complete cure for a disease and/or adverse effect attributable to the disease. "Treatment," as used herein, covers any treatment of a disease in a subject, particularly in a human, and includes: (a) preventing the disease from occurring in a subject which may be predisposed to the disease but has not yet been diagnosed as having it; (b) inhibiting the disease, *i.e.,* arresting its development; and (c) relieving the disease, *i.e.,* causing regression of the disease and/or relieving one or more disease symptoms. "Treatment" can also encompass delivery of an agent or administration of a therapy in order to provide for a pharmacologic effect, even in the absence of a disease or condition.

As used herein, the term "marker" or "biomarker" refers to a biological molecule, such as, for example, a nucleic acid, peptide, protein, hormone, and the like, whose presence or concentration can be detected and correlated with a known condition, such as a disease state. It can also be used to refer to a differentially expressed gene whose expression pattern can be utilized as part of a predictive, prognostic or diagnostic process in healthy conditions or a disease state, or which, alternatively, can be used in methods for identifying a useful treatment or prevention therapy.

As used herein, the term "expression levels" refers, for example, to a determined level of biomarker expression. The term "pattern of expression levels" refers to a determined level of biomarker expression compared either to a reference (*e.g.* a housekeeping gene or inversely regulated genes, or other reference biomarker) or to a computed average expression value (*e.g.* in DNA-chip analyses). A pattern is not limited to the comparison of two biomarkers but is more related to multiple comparisons of biomarkers to reference biomarkers or samples. A certain "pattern of expression levels" can also result and be determined by comparison and measurement of several biomarkers as disclosed herein and display the relative abundance of these transcripts to each other.

As used herein, a "reference pattern of expression levels" refers to any pattern of expression levels that can be used for the comparison to another pattern of expression levels. In some embodiments of the invention, a reference pattern of expression levels is, for example, an average pattern of expression levels observed in a group of healthy or diseased individuals, serving as a reference group.

"Antibody" or "antibody peptide(s)" refer to an intact antibody, or a binding fragment thereof that competes with the intact antibody for specific binding; this definition also encompasses monoclonal and polyclonal antibodies. Binding fragments are produced by recombinant DNA techniques, or by enzymatic or chemical cleavage of intact antibodies. Binding fragments include Fab, Fab', F(ab')₂, Fv, and single-chain antibodies. An antibody other than a "bispecific" or "bifunctional" antibody is understood to have each of its binding sites identical. An antibody, for example, substantially inhibits adhesion of a receptor to a counterreceptor when an excess of antibody reduces the quantity of receptor bound to counterreceptor by at least about 20%, 40%, 60% or 80%, and more usually greater than about 85% (as measured in an in vitro competitive binding assay).

As described herein, small molecule inhibitors of the ubiquitin conjugating enzyme UBE2N that modulate (e.g., suppress) UBE2N-dependent immune signalling in the context of, e.g., MDS, chronic inflammation, and AMI,-propagating cells *in vitro* and *in vivo* while simultaneously sparing healthy cells have been identified. This study establishes UBE2N as an immunomodulator and therapeutic target in e.g., AML, MDS, solid tumors, and chronic inflammation and fosters the development of clinically relevant strategies against immune-related disorders.

Extensive innate immune signaling pathways are broadly activated in leukemic hematopoietic stem and progenitor cells (HSPC) and contribute to the pathogenesis of myelodysplastic syndromes (MDS), and dysregulation of innate immune and inflammatory signaling pathways is implicated in various hematologic malignancies. Yet, these pathways have not been systematically examined in e.g., acute myeloid leukemia (AML). As described herein, AML HPSCs exhibit a high frequency of dysregulated innate immune-related and inflammatory pathways, referred to as oncogenic immune signaling states. Using newly identified small molecule inhibitors of the ubiquitin (Ub) conjugating enzyme UBE2N as chemical probes, this study revealed the therapeutic efficacy of interfering with UBE2N Ub-conjugating function by preventing ubiquitination of multiple innate immune- and inflammatory-related substrates in AML. Inhibition of UBE2N catalytic function in AML disrupted oncogenic immune signaling by promoting cell death of leukemic HSPCs while sparing healthy cells. Moreover, baseline oncogenic immune signaling states in discrete subsets of primary AML patients exhibited a selective dependency on UBE2N catalytic function. This study reveals that interfering with UBE2N function abrogates leukemic HSPCs and underscores the dependency of AML cells on UBE2N-dependent oncogenic immune signaling states. Thus, inhibition of UBE2N can be used as a therapeutic strategy for AML.

Recent studies have indicated that innate immune signaling pathways could represent features of leukemic HSPC that are amenable to therapeutic intervention, as they are co-opted in leukemic HSPC across various disease subtypes and independent of driver mutations [5, 6]. Under normal conditions, innate immune cells recognize pathogens and host cellular by-products by a family of pattern recognition receptors (PRRs), including Toll-like receptors (TLRs), NOD-like receptors (NLRs), RIG-I-like receptors (RLRs), AIM2-like receptors (ALRs), C- type lectins (CTLs), and OAS-like receptors (OLRs). Upon binding to ligand, innate immune receptors recruit intracellular adaptors, kinases, and effector molecules, which results in a context-dependent activation of transcription factors, such as NF-κB, STATs, AP1, and IRFs [7]. It has been demonstrated through mouse genetic models that chronic innate immune pathway activation in pre-leukemic HSPCs is required for the pathogenesis of myelodysplastic syndromes (MDS) in the absence of ligand activation and/or infection [8-10]. Moreover, genetic and pharmacologic inhibition of specific innate immune signaling mediators has shown promise of suppressing MDS- and AMI,-propagating cells, suggesting that innate immune signaling is requisite for pre-leukemic and leukemic HSPC function and amenable to therapeutic targeting [8, 11-13].

However, the therapeutic effectiveness of targeting these innate immune pathways in leukemic HSPCs has been lacking, which is attributed to the redundancy of signaling inputs that converge on inflammatory and immune-related pathways, referred to as oncogenic immune signaling states. Although innate immune signaling pathways are broadly activated in HSPCs and contribute to the pathogenesis of MDS, these pathways have not been systematically nor rigorously examined in, e.g., AML.

As described herein, dysregulated innate immune pathway activation is demonstrated to occur, for example, in leukemic HSPCs from distinct subtypes of AML and depends on the function of a convergent signaling node, UBE2N. UBE2N is an ubiquitin conjugating enzyme utilized by multiple immune-related pathways implicated in maintaining the oncogenic immune signaling states in leukemic HSPC. Collectively, these findings underscore the dependency of leukemic HSPCs on UBE2N-dependent oncogenic immune signaling states in AML.

Innate immune signaling pathways are co-opted in leukemic HSPC across various AML subtypes establishing oncogenic innate immune signaling dependencies. There are ongoing efforts to target oncogenic innate immune signaling states in AML by inhibiting specific immune receptors or downstream mediators relevant to only a single immune pathway.

As described herein, it was found that AML HSPC exhibit a high frequency of dysregulated innate immune-related genes and inflammatory pathways in ~50% of patients; thus, targeting one immune-related pathway will have limited therapeutic benefit. To circumvent these liabilities, the inventors searched for an integrated signaling node utilized by several innate immune pathways broadly dysregulated in AML.

The ubiquitin-conjugating enzyme UBE2N emerged as a convergent signaling node required for multiple innate immune pathways activated in AML HSPC and for leukemic cell function. Moreover, novel small molecule inhibitors were identified that selectively target UBE2N enzymatic activity, exhibit anti-leukemic activity by targeting the function of AML-propagating cells, and serve as chemical probes to identify UBE2N-dependent AMLs. Through these genetic and pharmacological studies, it was found that UBE2N is required for leukemic cell function in a discrete subset of AML patients with antecedent oncogenic immune signaling states. In particular, UBE2N is required for leukemic cell function in AML subtypes AML-M4 and AML-M5, and these subtypes can be treated by administrations of a UBE2N inhibitor.

UBE2N catalytic function is essential for transferring ubiquitin from a requisite E3 ubiquitin ligase to its substrates through formation of ubiquitin chains [16, 29]. Although UBE2N, together with select E3 ubiquitin ligases, synthesizes polyubiquitin chains on proteins implicated in innate immune signaling, DNA damage response, protein chaperone regulation, and cell motility, suppression of immune-related pathways was primarily observed in leukemic cells treated with the UBE2N inhibitors. Specifically, inhibition of UBE2N suppressed innate immune signaling pathways, including NF-xB, STAT, and TGFβ pathways. Importantly, these immune-related effector pathways are directly implicated in the pathogenesis of AML [30-33]. These observations further support the hypothesis that the most significant therapeutic benefit in AML will emerge by simultaneously targeting a convergent signaling node required by multiple immune-related pathways, such as by targeting UBE2N.

Although this demonstration of the inhibition of UBE2N in AML notably correlates with suppression of pathways of the innate immune response, how these pathways maintain leukemic stem cell function remains unclear. Chronic type 1 interferon signaling induces hematopoietic dysfunction by reducing HSC self-renewal [34, 35]. Similar effects on HSC are observed following stimulation of type 2 interferon IFNy [36-43]. Interestingly, sterile tonic IFNy and NF-κB signaling is required for normal HSC development at the embryonic stage or post-natal, respectively [37, 44], suggesting that these immune pathways have critical functions in the development and maintenance of HSC that are distinct from their role during acute inflammation.

Unlike the anti-leukemic effects observed by administration of IFNα/β [45], the present data indicate that low level persistent activation of innate immune signaling pathways are necessary for maintaining the viability and function of AML cells. Such a phenomenon has been observed for signaling associated with IFNy in solid tumors as sustained low-level IFNy has been reported to induce development of several solid tumors [46]. These observations are supported by findings that report IFNy maintains cancer stem cell dormancy and metastasis [47]. Future studies can determine the basis for the pro-leukemic cell state maintained by UBE2N-dependent immune signatures. Inhibition of UBE2N-dependent immune pathways suppressed oncogenic immune signaling and induced cytotoxic effects in leukemic cells *in vitro* and *in vivo* without affecting normal hematopoiesis or exhibiting toxicity in mice, indicating that there is a robust therapeutic window for UBE2N inhibitors with physical and chemical properties of UC-764864/5.

Importantly, these findings demonstrate that certain subtypes of AML are responsive to UBE2N inhibition, namely AMI,-M4 and AML-M5, and these subtypes can be treated by administrations of a UBE2N inhibitor. UBE2N inhibition therefore represents a useful treatment strategy for AML-M4 and AML-M5, which have been shown to be AML subtypes which are particularly resistant to other standard treatments, such as BCL2 inhibitors (e.g. venetoclax) (Pei S et al., Cancer Discovery, 2020 PMID 31974170). UBE2N inhibition can be used as an alternative therapy and can also be used to resensitize AML-M4 and AML-M5 to venetoclax, thereby enhancing the effectiveness of subsequent venetoclax administration.

According to further aspects, although this study demonstrates that UBE2N is an actionable target in malignant hematopoietic cells, the utility of UBE2N inhibitors can be extended to other cancers, chronic inflammatory disorders, and as immune checkpoint regulators. In addition to MDS and AML, UBE2N function is also implicated in other hematologic malignancies and solid tumors, including diffuse large B cell lymphoma [22], neuroblastoma [21], breast cancer [48-51], metastatic colorectal cancer [52], hepatocarcinoma [53], and ovarian cancer [54]. Conditional deletion of UBE2N in regulatory T cells (Tregs) revealed that UBE2N maintains the suppressive function of Tregs and prevents their conversion into effector-like T cells [55]. The removal of Treg cells can evoke and enhance anti-tumor immune response; therefore, UBE2N inhibitors may be effective at suppressing Treg function and consequently inducing anti-tumor immune responses in cancer. UBE2N is also implicated in a variety of chronic inflammatory disorders [56-58]. Collectively, the utility of UBE2N inhibitors can be extended beyond AML and utilized as anti-inflammatory agents, direct anti-cancer therapy, as well as enhancers of anti-tumor immune responses.

### Diseases and Disorders

Embodiments of the methods relate to administration of a compound or composition including a UBE2N inhibitor to treat any disease or disorder characterized by malignant hematopoietic cells, as well as other cancers, chronic inflammatory disorders, and as immune checkpoint regulators.

In some embodiments, treating a disease or disorder involving UBE2N, such as MDS, AML, other cancers, chronic inflammatory disorders, and as immune checkpoint regulators, and the like, can involve disease prevention, reducing the risk of the disease, ameliorating or relieving symptoms of the disease, eliciting a bodily response against the disease, inhibiting the development or progression of the disease, inhibiting or preventing the onset of symptoms of the disease, reducing the severity of the disease, causing a regression of the disease or a disease symptom, causing remission of the disease, preventing relapse of the disease, and the like. In some embodiments, treating includes prophylactic treatment. In some embodiments, treating does not include prophylactic treatment.

In some embodiments of the methods, the disease or disorder can be MDS and/or AML and/or a type of cancer. In some embodiments, the disease or disorder involves hematologic malignancies and/or solid tumors, such as, for example, diffuse large B cell lymphoma, neuroblastoma, breast cancer, metastatic colorectal cancer, hepatocarcinoma, ovarian cancer, and the like.

In some embodiments, the MDS can be selected from Fanconi Anemia, refractory anemia, refractory neutropenia, refractory thrombocytopenia, refractory anemia with ringed sideroblasts (RARS), refractory cytopenia with multilineage dysplasia (RCMD), refractory anemia with multilineage dysplasia and ringed sideroblasts (RCMD-RS), refractory anemia with excess blasts I and II (RAEB), myelodysplastic syndrome, unclassified (MDS-U), MDS associated with isolated del(5q)-syndrome, chronic myelomonocytic leukemia (CMML), juvenile myelomonocytic leukemia (JMML), refractory cytopenia of childhood, or a combination thereof. In some embodiments, the MDS is primary MDS. In some embodiments, the MDS is secondary MDS.

In some embodiments, the AML can be selected from AML with recurrent genetic abnormalities (such as, for example, AML with translocation between chromosomes 8 and 21, AML with translocation or inversion in chromosome 16, AML with translocation between chromosomes 9 and 11, APL (M3) with translocation between chromosomes 15 and 17, AML with translocation between chromosomes 6 and 9, AML with translocation or inversion in chromosome 3, and the like), AML (megakaryoblastic) with a translocation between chromosomes 1 and 22, AML with myelodysplasia-related changes, AML related to previous chemotherapy or radiation (such as, for example, alkylating agent-related AML, topoisomerase II inhibitor-related AML, and the like), AML not otherwise categorized (does not fall into above categories - similar to FAB classification; such as, for example, AML minimally differentiated (M0), AML with minimal maturation (M1), AML with maturation (M2), acute myelomonocytic leukemia (M4), acute monocytic leukemia (M5), acute erythroid leukemia (M6), acute megakaryoblastic leukemia (M7), acute basophilic leukemia, acute panmyelosis with fibrosis, and the like), myeloid sarcoma (also known as granulocytic sarcoma, chloroma or extramedullary myeloblastoma), undifferentiated and biphenotypic acute leukemias (also known as mixed phenotype acute leukemias), and the like.

In some embodiments, the type of cancer can be selected from diffuse large B cell lymphoma, neuroblastoma, breast cancer, metastatic colorectal cancer, hepatocarcinoma, ovarian cancer, cervical cancer, colorectal cancer, endometrial cancer, glioma, head and neck cancer, liver cancer, melanoma, pancreatic cancer, prostate cancer, renal cancer, stomach cancer, testicular cancer, thyroid cancer, urothelial cancer, and the like.

In some embodiments, the disease can be a chronic inflammatory disorder. In some embodiments, the chronic inflammatory disorder can be selected from ...

In some embodiments, the administration may decrease the incidence of one or more symptoms associated with MDS / AML / a type of cancer / chronic inflammatory disorder. In some embodiments, the administration may decrease marrow failure, immune dysfunction, transformation to overt leukemia, or combinations thereof in said individual, as compared to an individual not receiving said composition.

In some embodiments, the method may decrease a marker of viability of MDS cells or cancer cells. In one aspect, the method may decrease a marker of viability of MDS, AML, and/or cancer cells. The marker may be selected from survival over time, proliferation, growth, migration, formation of colonies, chromatic assembly, DNA binding, RNA metabolism, cell migration, cell adhesion, inflammation, or a combination thereof.

### UBE2N Inhibitors

The present invention encompasses methods of treating a disease or disorder by administering a compound or composition comprising an UBE2N inhibitor.

Compounds and compositions which can be useful as UBE2N inhibitors are known in the art and are in development. Methods of treating a disease or disorder by administration of an UBE2N inhibitor, according to the present invention can involve any compound or composition which is demonstrated to inhibit UBE2N. These include compounds which are currently commercially available, those which have been disclosed via publication, and those having yet to be contemplated. Embodiments of the invention also encompass methods of treating a disease or disorder by administration of an UBE2N inhibitor which can be administered in conjunction with, or separately in a treatment course along with, one or more additional treatments, such as cancer treatments, as set forth herein.

UBE2N inhibitors are known in the art. In some embodiments, UBE2N inhibitors include small molecules, and salts, cocrystals, hydrates, solvates, optical isomers, geometric isomers, salts of isomers, prodrugs, and derivatives thereof. In some embodiments, the UBE2N inhibitor can include, for example, one or more compounds such as NSC697923 (2-(4-methylphenyl)sulfonyl-5-nitrofuran), UC-764864 (1-(4-ethylphenyl)-3-[(6-methyl-1H-benzimidazol-2-yl) sulfanyl]prop-2-en-1-one), or UC-764865 (1-(4-methoxyphenyl)-3-[(6-methyl-1H-benzimidazol-2-yl) sulfanyl]prop-2-en-1-one), (1-(4-methylphenyl)-3-[(6-methyl-1H-benzimidazol-2-yl) sulfanyl]prop-2-en-1-one), and the like, as well as derivatives such as pharmaceutically-acceptable salts, cocrystals, hydrates, solvates, optical isomers, geometric isomers, salts of isomers, or prodrugs thereof, and combinations thereof. In some embodiments, the UBE2N inhibitor is UC-764864 (1-(4-ethylphenyl)-3-[(6-methyl-1H-benzimidazol-2-yl) sulfanyl]prop-2-en-1-one) or UC-764865 (1-(4-methoxyphenyl)-3-[(6-methyl-1H-benzimidazol-2-yl) sulfanyl]prop-2-en-1-one), or a salt, cocrystal, hydrate, solvate, optical isomer, geometric isomer, salt of isomer, prodrug, or derivative thereof. In some embodiments, the UBE2N inhibitor may comprise an RNAi sufficient to inhibit UBE2N expression.

Further UBE2N inhibitors could be identified and understood by those skilled in the art; it is contemplated that any such compound, or derivative, which is demonstrated or known in the art to have effectiveness as an UBE2N inhibitor can be used in accordance with the present invention. All references listed above are incorporated herein with respect to their teachings of specific UBE2N inhibiting compounds and genera.

In some embodiments, the UBE2N inhibitor can be administered in combination with an apoptotic modulator. The apoptotic modulator may comprise a BTK and/or a BCL2 inhibitor. BTK and BCL2 inhibitors may be, for example, those known in the art. In some embodiments, the method may comprise the step of administering to the individual an apoptotic modulator. In some embodiments, the apoptotic modulator may comprise a BCL2 inhibitor selected from ABT-263 (Navitoclax), ABT-737, ABT-199 (venetoclax), GDC-0199, GX15-070 (Obatoclax) (all available from Abbott Laboratories), HA14-1, S1, 2-methoxy antimycin A3, gossypol, AT-101, apogossypol, WEHI-539, A-1155463, BXI-61, BXI-72, TW37, MIM1, UMI-77, and the like, and combinations thereof. One skilled in the art would appreciate that there are many known BCL2 inhibitors which can be used in accordance with the present invention. In some embodiments, the BCL2 inhibitor comprises venetoclax, or a salt, isomer, derivative or analog thereof.

In some embodiments, the UBE2N inhibitor can be administered in combination with one or more cancer treatments, such as, for example, those described herein. In some embodiments, the UBE2N inhibitor can additionally or alternatively be administered in combination with one or more agent selected from an apoptotic agent, an immune modulating agent, an epigenetic modifying agent, and combinations thereof.

### Cancer Treatments

Treatment regimens for various types of cancers can involve one or more elements selected from chemotherapy, targeted therapy, alternative therapy, immunotherapy, and the like.

### Chemotherapy / Targeted Therapy / Alternative Therapy

Cancers are commonly treated with chemotherapy and/or targeted therapy and/or alternative therapy. Chemotherapies act by indiscriminately targeting rapidly dividing cells, including healthy cells as well as tumor cells, whereas targeted cancer therapies rather act by interfering with specific molecules, or molecular targets, which are involved in cancer growth and progression. Targeted therapy generally targets cancer cells exclusively, having minimal damage to normal cells. Chemotherapies and targeted therapies which are approved and/or in the clinical trial stage are known to those skilled in the art. Any such compound can be utilized in the practice of the present invention.

For example, approved chemotherapies include abitrexate (Methotrexate Injection), abraxane (Paclitaxel Injection), adcetris (Brentuximab Vedotin Injection), adriamycin (Doxorubicin), adrucil Injection (5-FU (fluorouracil)), afinitor (Everolimus), afinitor Disperz (Everolimus), alimta (PEMETREXED), alkeran Injection (Melphalan Injection), alkeran Tablets (Melphalan), aredia (Pamidronate), arimidex (Anastrozole), aromasin (Exemestane), arranon (Nelarabine), arzerra (Ofatumumab Injection), avastin (Bevacizumab), beleodaq (Belinostat Injection), bexxar (Tositumomab), BiCNU (Carmustine), blenoxane (Bleomycin), blincyto (Blinatumoma b Injection), bosulif (Bosutinib), busulfex Injection (Busulfan Injection), campath (Alemtuzumab), camptosar (Irinotecan), caprelsa (Vandetanib), casodex (Bicalutamide), CeeNU (Lomustine), CeeNU Dose Pack (Lomustine), cerubidine (Daunorubicin), clolar (Clofarabine Injection), cometriq (Cabozantinib), cosmegen (Dactinomycin), cotellic (Cobimetinib), cyramza (Ramucirumab Injection), cytosarU (Cytarabine), cytoxan (Cytoxan), cytoxan Injection (Cyclophosphamide Injection), dacogen (Decitabine), daunoXome (Daunorubicin Lipid Complex Injection), decadron (Dexamethasone), depoCyt (Cytarabine Lipid Complex Injection), dexamethasone Intensol (Dexamethasone), dexpak Taperpak (Dexamethasone), docefrez (Docetaxel), doxil (Doxorubicin Lipid Complex Injection), droxia (Hydroxyurea), DTIC (Decarbazine), eligard (Leuprolide), ellence (Ellence (epirubicin)), eloxatin (Eloxatin (oxaliplatin)), elspar (Asparaginase), emcyt (Estramustine), erbitux (Cetuximab), erivedge (Vismodegib), erwinaze (Asparaginase Erwinia chrysanthemi), ethyol (Amifostine), etopophos (Etoposide Injection), eulexin (Flutamide), fareston (Toremifene), farydak (Panobinostat), faslodex (Fulvestrant), femara (Letrozole), firmagon (Degarelix Injection), fludara (Fludarabine), folex (Methotrexate Injection), folotyn (Pralatrexate Injection), FUDR (FUDR (floxuridine)), gazyva (Obinutuzumab Injection), gemzar (Gemcitabine), gilotrif (Afatinib), gleevec (Imatinib Mesylate), Gliadel Wafer (Carmustine wafer), Halaven (Eribulin Injection), Herceptin (Trastuzumab), Hexalen (Altretamine), Hycamtin (Topotecan), Hycamtin (Topotecan), Hydrea (Hydroxyurea), Ibrance (Palbociclib), Iclusig (Ponatinib), Idamycin PFS (Idarubicin), Ifex (Ifosfamide), Imbruvica (Ibrutinib), Inlyta (Axitinib), Intron A alfab (Interferon alfa-2a), Iressa (Gefitinib), Istodax (Romidepsin Injection), Ixempra (Ixabepilone Injection), Jakafi (Ruxolitinib), Jevtana (Cabazitaxel Injection), Kadcyla (Ado-trastuzumab Emtansine), Keytruda (Pembrolizumab Injection), Kyprolis (Carfilzomib), Lanvima (Lenvatinib), Leukeran (Chlorambucil), Leukine (Sargramostim), Leustatin (Cladribine), Lonsurf (Trifluridine and Tipiracil), Lupron (Leuprolide), Lupron Depot (Leuprolide), Lupron DepotPED (Leuprolide), Lynparza (Olaparib), Lysodren (Mitotane), Marqibo Kit (Vincristine Lipid Complex Injection), Matulane (Procarbazine), Megace (Megestrol), Mekinist (Trametinib), Mesnex (Mesna), Mesnex (Mesna Injection), Metastron (Strontium-89 Chloride), Mexate (Methotrexate Injection), Mustargen (Mechlorethamine), Mutamycin (Mitomycin), Myleran (Busulfan), Mylotarg (Gemtuzumab Ozogamicin), Navelbine (Vinorelbine), Neosar Injection (Cyclophosphamide Injection), Neulasta (filgrastim), Neulasta (pegfilgrastim), Neupogen (filgrastim), Nexavar (Sorafenib), Nilandron (Nilandron (nilutamide)), Nipent (Pentostatin), Nolvadex (Tamoxifen), Novantrone (Mitoxantrone), Odomzo (Sonidegib), Oncaspar (Pegaspargase), Oncovin (Vincristine), Ontak (Denileukin Diftitox), onxol (Paclitaxel Injection), opdivo (Nivolumab Injection), panretin (Alitretinoin), paraplatin (Carboplatin), perjeta (Pertuzumab Injection), platinol (Cisplatin), platinol (Cisplatin Injection), platinolAQ (Cisplatin), platinolAQ (Cisplatin Injection), pomalyst (Pomalidomide), prednisone Intensol (Prednisone), proleukin (Aldesleukin), purinethol (Mercaptopurine), reclast (Zoledronic acid), revlimid (Lenalidomide), rheumatrex (Methotrexate), rituxan (Rituximab), roferonA alfaa (Interferon alfa-2a), rubex (Doxorubicin), sandostatin (Octreotide), sandostatin LAR Depot (Octreotide), soltamox (Tamoxifen), spry cel (Dasatinib), sterapred (Prednisone), sterapred DS (Prednisone), stivarga (Regorafenib), supprelin LA (Histrelin Implant), sutent (Sunitinib), sylatron (Peginterferon Alfa-2b Injection (Sylatron)), sylvant (Siltuximab Injection), synribo (Omacetaxine Injection), tabloid (Thioguanine), taflinar (Dabrafenib), tarceva (Erlotinib), targretin Capsules (Bexarotene), tasigna (Decarbazine), taxol (Paclitaxel Injection), taxotere (Docetaxel), temodar (Temozolomide), temodar (Temozolomide Injection), tepadina (Thiotepa), thalomid (Thalidomide), theraCys BCG (BCG), thioplex (Thiotepa), TICE BCG (BCG), toposar (Etoposide Injection), torisel (Temsirolimus), treanda (Bendamustine hydrochloride), trelstar (Triptorelin Injection), trexall (Methotrexate), trisenox (Arsenic trioxide), tykerb (lapatinib), unituxin (Dinutuximab Injection), valstar (Valrubicin Intravesical), vantas (Histrelin Implant), vectibix (Panitumumab), velban (Vinblastine), velcade (Bortezomib), vepesid (Etoposide), vepesid (Etoposide Injection), vesanoid (Tretinoin), vidaza (Azacitidine), vincasar PFS (Vincristine), vincrex (Vincristine), votrient (Pazopanib), vumon (Teniposide), wellcovorin IV (Leucovorin Injection), xalkori (Crizotinib), xeloda (Capecitabine), xtandi (Enzalutamide), yervoy (Ipilimumab Injection), yondelis (Trabectedin Injection), zaltrap (Ziv-aflibercept Injection), zanosar (Streptozocin), zelboraf (Vemurafenib), zevalin (Ibritumomab Tiuxetan), zoladex (Goserelin), zolinza (Vorinostat), zometa (Zoledronic acid), zortress (Everolimus), zydelig (Idelalisib), zykadia (Ceritinib), zytiga (Abiraterone), and the like, in addition to analogs and derivatives thereof. For example, approved targeted therapies include ado-trastuzumab emtansine (Kadcyla), afatinib (Gilotrif), aldesleukin (Proleukin), alectinib (Alecensa), alemtuzumab (Campath), axitinib (Inlyta), belimumab (Benlysta), belinostat (Beleodaq), bevacizumab (Avastin), bortezomib (Velcade), bosutinib (Bosulif), brentuximab vedotin (Adcetris), cabozantinib (Cabometyx [tablet], Cometriq [capsule]), canakinumab (Ilaris), carfilzomib (Kyprolis), ceritinib (Zykadia), cetuximab (Erbitux), cobimetinib (Cotellic), crizotinib (Xalkori), dabrafenib (Tafinlar), daratumumab (Darzalex), dasatinib (Sprycel), denosumab (Xgeva), dinutuximab (Unituxin), elotuzumab (Empliciti), erlotinib (Tarceva), everolimus (Afinitor), gefitinib (Iressa), ibritumomab tiuxetan (Zevalin), ibrutinib (Imbruvica), idelalisib (Zydelig), imatinib (Gleevec), ipilimumab (Yervoy), ixazomib (Ninlaro), lapatinib (Tykerb), lenvatinib (Lenvima), necitumumab (Portrazza), nilotinib (Tasigna), nivolumab (Opdivo), obinutuzumab (Gazyva), ofatumumab (Arzerra, HuMax-CD20), olaparib (Lynparza),osimertinib (Tagrisso), palbociclib (Ibrance), panitumumab (Vectibix), panobinostat (Farydak), pazopanib (Votrient), pembrolizumab (Keytruda), pertuzumab (Perjeta), ponatinib (Iclusig), ramucirumab (Cyramza), rapamycin, regorafenib (Stivarga), rituximab (Rituxan, Mabthera), romidepsin (Istodax), ruxolitinib (Jakafi), siltuximab (Sylvant), sipuleucel-T (Provenge), sirolimus, sonidegib (Odomzo), sorafenib (Nexavar), sunitinib, tamoxifen, temsirolimus (Torisel), tocilizumab (Actemra), tofacitinib (Xeljanz), tositumomab (Bexxar), trametinib (Mekinist), trastuzumab (Herceptin), vandetanib (Caprelsa), vemurafenib (Zelboraf), venetoclax (Venclexta), vismodegib (Erivedge), vorinostat (Zolinza), ziv-aflibercept (Zaltrap), and the like, in addition to analogs and derivatives thereof.

Those skilled in the art can determine appropriate chemotherapy and/or targeted therapy and/or alternative therapy options, including treatments that have been approved and those that in clinical trials or otherwise under development. Some targeted therapies are also immunotherapies. Any relevant chemotherapy, target therapy, and alternative therapy treatment strategies can be utilized, alone or in combination with one or more additional cancer therapy, in the practice of the present invention.

### Immunotherapy

In some embodiments, immunotherapies include cell-based immunotherapies, such as those involving cells which effect an immune response (such as, for example, lymphocytes, macrophages, natural killer (NK) cells, dendritic cells, cytotoxic T lymphocytes (CTL), antibodies and antibody derivatives (such as, for example, monoclonal antibodies, conjugated monoclonal antibodies, polyclonal antibodies, antibody fragments, radiolabeled antibodies, chemolabeled antibodies, etc.), immune checkpoint inhibitors, vaccines (such as, for example, cancer vaccines (e.g. tumor cell vaccines, antigen vaccines, dendritic cell vaccines, vector-based vaccines, etc.), e.g. oncophage, sipuleucel-T, and the like), immunomodulators (such as, for example, interleukins, cytokines, chemokines, etc.), topical immunotherapies (such as, for example, imiquimod, and the like), injection immunotherapies, adoptive cell transfer, oncolytic virus therapies (such as, for example, talimogene laherparepvec (T-VEC), and the like), immunosuppressive drugs, helminthic therapies, other non-specific immunotherapies, and the like. Immune checkpoint inhibitor immunotherapies are those that target one or more specific proteins or receptors, such as PD-1, PD-L1, CTLA-4, and the like. Immune checkpoint inhibitor immunotherapies include ipilimumab (Yervoy), nivolumab (Opdivo), pembrolizumab (Keytruda), and the like. Non-specific immunotherpaies include cytokines, interleukins, interferons, and the like. In some embodiments, an immunotherapy assigned or administered to a subject can include an interleukin, and/or interferon (IFN), and/or one or more suitable antibody-based reagent, such as denileukin diftitox and/or administration of an antibody-based reagent selected from the group consisting of ado-trastuzumab emtansine, alemtuzumab, atezolizumab, bevacizumab, blinatumomab, brentuximab vedotin, cetuximab, catumaxomab, gemtuzumab, ibritumomab tiuxetan, ilipimumab, natalizumab, nimotuzumab, nivolumab, ofatumumab, panitumumab, pembrolizumab, rituximab, tositumomab, trastuzumab, vivatuxin, and the like. In some embodiments, an immunotherapy assigned or administered to a subject can include an indoleamine 2,3-dioxygenase (IDO) inhibitor, adoptive T-cell therapy, virotherapy (T-VEC), and/or any other immunotherapy whose efficacy extensively depends on anti-tumor immunity.

Those skilled in the art can determine appropriate immunotherapy options, including treatments that have been approved and those that in clinical trials or otherwise under development. Any relevant immunotherapy treatment strategies, alone or in combination with one or more additional cancer therapy, can be utilized in the practice of the present invention.

### Other Cancer Treatments

In addition to chemotherapies, targeted therapies, alternative therapies, and immunotherapies, cancer can additionally be treated by other strategies. These include surgery, radiation therapy, hormone therapy, stem cell transplant, precision medicine, and the like; such treatments and the compounds and compositions utilized therein are known to those skilled in the art. Any such treatment strategies can be utilized in the practice of the present invention.

Alternative treatment strategies have also been used with various types of cancers. Such treatment can be used alone or in combination with any other treatment modality. These include exercise, massage, relaxation techniques, yoga, acupuncture, aromatherapy, hypnosis, music therapy, dietary changes, nutritional and dietary supplements, and the like; such treatments are known to those skilled in the art. Any such treatment strategies can be utilized, alone or in combination with one or more additional cancer therapy, in the practice of the present invention.

### Administration

Particular aspects of the invention relate to the use of cancer treatments, in the form of therapeutic compounds and/or compositions, directly administered to a subject. Such compounds and/or compositions and/or their physiologically acceptable salts or esters, can be used for the preparation of a medicament (pharmaceutical preparation). They can be converted into a suitable dosage form together with at least one solid, liquid and/or semiliquid excipient or assistant and, if desired, in combination with one or more further active ingredients.

Therapeutic compounds and/or compositions can be prepared and administered in a wide variety of ophthalmic, oral, parenteral, and topical dosage forms. The therapeutic compounds and/or compositions can be administered by eye drop. Also, therapeutic compounds and/or compositions can be administered by injection (e.g. intravenously, intramuscularly, intravitreally, intracutaneously, subcutaneously, intraduodenally, or intraperitoneally). As such, therapeutic compounds and/or compositions can also be administered by intravitreal injection. Also, therapeutic compounds and/or compositions can be administered by inhalation, for example, intranasally. Additionally, therapeutic compounds and/or compositions can be administered transdermally. It is also envisioned that multiple routes of administration (e.g., intramuscular, oral, ocular) can be used to administer therapeutic compounds and/or compositions.

### Formulations

Particular aspects of the invention furthermore include medicaments comprising at least one therapeutic compound or composition suitable for treatment of cancer, and/or its pharmaceutically usable derivatives, solvates and stereoisomers, including mixtures thereof in all ratios, and optionally excipients and/or assistants.

According to particular aspects, the therapeutic compounds and compositions can be administered by any conventional method available for use in conjunction with pharmaceutical drugs, either as individual therapeutic agents or in a combination of therapeutic agents. Such therapeutics can be administered by any pharmaceutically acceptable carrier, including, for example, any and all solvents, dispersion media, coatings, antibacterial and antifungal agents, isotonic and absorption delaying agents, and the like, compatible with pharmaceutical administration. The use of such media and agents for pharmaceutically active substances is known in the art. Except insofar as any conventional medium or agent is incompatible with the active compound, such media can be used in the compositions of the invention. Supplementary active compounds can also be incorporated into the compositions. A pharmaceutical composition in particular aspects of the invention is formulated to be compatible with its intended route of administration. Routes of administration include for example, but are not limited to, intravenous, intramuscular, and oral, and the like. Additional routes of administration include, for example, sublingual, buccal, parenteral (including, for example, subcutaneous, intramuscular, intraarterial, intradermal, intraperitoneal, intracisternal, intravesical, intrathecal, or intravenous), transdermal, oral, transmucosal, and rectal administration, and the like.

Solutions or suspensions used for appropriate routes of administration, including, for example, but not limited to parenteral, intradermal, or subcutaneous application, and the like, can include, for example, the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates, or phosphates and agents for the adjustment of tonicity such as sodium chloride or dextrose, and the like. The pH can be adjusted with acids or bases, such as, for example, hydrochloric acid or sodium hydroxide, and the like. The parenteral preparation can be enclosed in, for example, ampules, disposable syringes, or multiple dose vials made of glass or plastic, and the like.

Exemplary pharmaceutical compositions suitable for injectable use include, for example, sterile aqueous solutions (where water soluble) or dispersions and sterile powders for the extemporaneous preparation of sterile injectable solutions or dispersion, and the like. For intravenous administration, suitable carriers include, for example, physiological saline, bacteriostatic water, Cremophor ELTM (BASF, Parsippany, N.J.) or phosphate buffered saline (PBS), and the like. In all cases, the composition should be fluid to the extent that easy syringability exists. The carrier can be a solvent or dispersion medium containing, for example, water, ethanol, polyol (for example, glycerol, propylene glycol, and liquid polyethylene glycol, and the like), and suitable mixtures thereof, and the like. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, such as, for example, parabens, chlorobutanol, phenol, ascorbic acid, thimerosal, and the like. In many cases, it can be preferable to include isotonic agents, such as, for example, sugars, polyalcohols such as mannitol, sorbitol, and sodium chloride, and the like, in the composition. Prolonged absorption of the injectable compositions can be brought about by including in the composition an agent which delays absorption such as, for example, aluminum monostearate and gelatin, and the like.

Exemplary sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Exemplary oral compositions generally include an inert diluent or an edible carrier. They can be enclosed in gelatin capsules or compressed into tablets, for example. For oral administration, the agent can be contained in enteric forms to survive the stomach or further coated or mixed to be released in a particular region of the gastrointestinal (GI) tract by known methods. For the purpose of oral therapeutic administration, the active compound can be incorporated with excipients and used in the form of tablets, troches, or capsules, or the like. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches, and the like can contain any of the following exemplary ingredients, or compounds of a similar nature: a binder such as microcrystalline cellulose, gum tragacanth or gelatin; an excipient such as starch or lactose, a disintegrating agent such as alginic acid, Primogel^{®}, or corn starch; a lubricant such as magnesium stearate; a glidant such as colloidal silicon dioxide; a sweetening agent such as sucrose or saccharin; or a flavoring agent such as peppermint, methyl salicylate, or orange flavoring, or the like. Suitable excipients are organic or inorganic substances which are suitable for enteral (for example oral), parenteral or topical administration and do not react with the novel compounds, for example water, vegetable oils, benzyl alcohols, alkylene glycols, polyethylene glycols, glycerol triacetate, gelatin, carbohydrates, such as lactose or starch, magnesium stearate, talc or VASELINE^{®}. Suitable for oral administration are, in particular, tablets, pills, coated tablets, capsules, powders, granules, syrups, juices or drops, suitable for rectal administration are suppositories, suitable for parenteral administration are solutions, preferably oil-based or aqueous solutions, furthermore suspensions, emulsions or implants, and suitable for topical application are ointments, creams or powders or also as nasal sprays. The novel compounds may also be lyophilized and the resultant lyophilizates used, for example, to prepare injection preparations. The preparations indicated may be sterilized and/or comprise assistants, such as lubricants, preservatives, stabilizers and/or wetting agents, emulsifying agents, salts for modifying the osmotic pressure, buffer substances, colorants and flavors and/or a plurality of further active ingredients, for example one or more vitamins.

For administration by inhalation, the compositions can be delivered in the form of an aerosol spray from pressured container or dispenser, which contains a suitable propellant, *e.g.,* a gas such as carbon dioxide, or a nebulizer, or the like. Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives, and the like. Transmucosal administration can be accomplished through the use of nasal sprays or suppositories. For transdermal administration, the active compounds are formulated into ointments, salves, gels, or creams as generally known in the art.

The compositions can also be prepared in the form of suppositories (*e.g.,* with conventional suppository bases such as cocoa butter and other glycerides) or retention enemas for rectal delivery.

In particular embodiments, therapeutic compounds and/or compositions are prepared with carriers that will protect the compound against rapid elimination from the body, such as a controlled release formulation, including implants and microencapsulated delivery systems, and the like. Biodegradable, biocompatible polymers can be used, such as, for example, ethylene vinyl acetate, polyanhydrides, polyglycolic acid, collagen, polyorthoesters, and polylactic acid, and the like. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No. 4,522,811, which is incorporated herein by reference in its entirety.

In some embodiments, therapeutic compounds and/or compositions can be prepared in liquid pharmaceutical compositions for ocular administration. The composition for ocular administration can contain one or more agents selected from the group of buffering agents, solubilizing agents, coloring agents, viscosity enhancing agents, and preservation agents in order to produce pharmaceutically elegant and convenient preparations.

In some embodiments, the composition for ocular administration can contain preservatives for protection against microbiological contamination, including but not limited to benzalkodium chloride and/or EDTA. Other possible preservatives include but are not limited to benzyl alcohol, methyl parabens, propyl parabens, and chlorobutanol. Preferably, a preservative, or combination of preservatives, will be employed to impart microbiological protection in addition to protection against oxidation of components.

In some embodiments, therapeutic compounds and/or compositions can be administered orally as tablets, aqueous or oily suspensions, lozenges, troches, powders, granules, emulsions, capsules, syrups or elixirs. The composition for oral use can contain one or more agents selected from the group of sweetening agents, flavoring agents, coloring agents and preserving agents in order to produce pharmaceutically elegant and palatable preparations. Accordingly, there are also provided pharmaceutical compositions comprising a pharmaceutically acceptable carrier or excipient and one or more therapeutic compounds and/or compositions.

In some embodiments, tablets contain the acting ingredient in admixture with non-toxic pharmaceutically acceptable excipients that are suitable for the manufacture of tablets. These excipients can be, for example, (1) inert diluents, such as calcium carbonate, lactose, calcium phosphate, carboxymethylcellulose, or sodium phosphate; (2) granulating and disintegrating agents, such as corn starch or alginic acid; (3) binding agents, such as starch, gelatin or acacia; and (4) lubricating agents, such as magnesium stearate, stearic acid or talc. These tablets can be uncoated or coated by known techniques to delay disintegration and absorption in the gastrointestinal tract and thereby provide a sustained action over a longer period. For example, a time delay material such as glyceryl monostearate or glyceryl distearate can be employed.

For preparing pharmaceutical compositions from therapeutic compounds and/or compositions, pharmaceutically acceptable carriers can be either solid or liquid. Solid form preparations include powders, tablets, pills, capsules, cachets, suppositories, and dispersible granules. A solid carrier can be one or more substance that can also act as diluents, flavoring agents, binders, preservatives, tablet disintegrating agents, or an encapsulating material.

A compound disclosed herein, in the form of a free compound or a pharmaceutically-acceptable pro-drug, metabolite, analogue, derivative, solvate or salt, can be administered, for in vivo application, parenterally by injection or by gradual perfusion over time. Administration can be intravenously, intraperitoneally, intramuscularly, subcutaneously, intracavity, or transdermally. For *in vitro* studies the compounds can be added or dissolved in an appropriate biologically acceptable buffer and added to a cell or tissue.

In powders, the carrier is a finely divided solid in a mixture with the finely divided active component. In tablets, the active component is mixed with the carrier having the necessary binding properties in suitable proportions and compacted in the shape and size desired.

The powders and tablets preferably contain from 5% to 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

When parenteral application is needed or desired, particularly suitable admixtures for therapeutic compounds and/or compositions are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, polyoxyethylene-block polymers, and the like. Ampoules are convenient unit dosages. The therapeutic compounds and/or compositions can also be incorporated into liposomes or administered via transdermal pumps or patches. Pharmaceutical admixtures suitable for use in the pharmaceuticals compositions and methods disclosed herein include those described, for example, in PHARMACEUTICAL SCIENCES (17th Ed., Mack Pub. Co., Easton, PA) and WO 96/05309, the teachings of both of which are hereby incorporated by reference.

In some embodiments, preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's intravenous vehicles include fluid and nutrient replenishers, electrolyte replenishers (such as those based on Ringer's dextrose), and the like. Preservatives and other additives can also be present such as, for example, antimicrobials, anti-oxidants, chelating agents, growth factors and inert gases and the like.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations that are intended to be converted, shortly before use, to liquid form preparations for oral administration. Such liquid forms include solutions, suspensions, and emulsions. These preparations can contain, in addition to the active component, colorants, flavors, stabilizers, buffers, artificial and natural sweeteners, dispersants, thickeners, solubilizing agents, and the like.

Some compounds can have limited solubility in water and therefore can require a surfactant or other appropriate co-solvent in the composition. Such co-solvents include: Polysorbate 20, 60, and 80; Pluronic F-68, F-84, and P-103; cyclodextrin; and polyoxyl 35 castor oil. Such co-solvents are typically employed at a level between about 0.01 % and about 2% by weight.

Viscosity greater than that of simple aqueous solutions can be desirable to decrease variability in dispensing the formulations, to decrease physical separation of components of a suspension or emulsion of formulation, and/or otherwise to improve the formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, chondroitin sulfate and salts thereof, hyaluronic acid and salts thereof, and combinations of the foregoing. Such agents are typically employed at a level between about 0.01% and about 2% by weight.

The compositions disclosed herein can additionally include components to provide sustained release and/or comfort. Such components include high molecular weight, anionic mucomimetic polymers, gelling polysaccharides, and finely-divided drug carrier substrates. These components are discussed in greater detail in U.S. Pat. Nos. 4,911,920; 5,403,841; 5,212,162; and 4,861,760. The entire contents of these patents are incorporated herein by reference in their entirety for all purposes.

There are provided various pharmaceutical compositions useful for ameliorating certain diseases and disorders. The pharmaceutical compositions according to one embodiment are prepared by formulating a compound disclosed herein in the form of a free compound or a pharmaceutically-acceptable pro-drug, metabolite, analogue, derivative, solvate or salt, either alone or together with other pharmaceutical agents, suitable for administration to a subject using carriers, excipients and additives or auxiliaries. Frequently used carriers or auxiliaries include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. Intravenous vehicles include fluid and nutrient replenishers.

Preservatives include antimicrobial, anti-oxidants, chelating agents and inert gases. Other pharmaceutically acceptable carriers include aqueous solutions, non-toxic excipients, including salts, preservatives, buffers and the like, as described, for instance, in Remington's Pharmaceutical Sciences, 15th ed. Easton: Mack Publishing Co. , 1405-1412, 1461-1487 (1975) and The National Formulary XIV., 14th ed. Washington: American Pharmaceutical Association (1975), the contents of which are hereby incorporated by reference. The pH and exact concentration of the various components of the pharmaceutical composition are adjusted according to routine skills in the art. *See* e.g., Goodman and Gilman (eds.), 1990, THE PHARMACOLOGICAL BASIS FOR THERAPEUTICS (7th ed.).

The pharmaceutical compositions are preferably prepared and administered in dose units. Solid dose units are tablets, capsules and suppositories. For treatment of a subject, depending on activity of the compound, manner of administration, nature and severity of the disease or disorder, age and body weight of the subject, different daily doses can be used.

Under certain circumstances, however, higher or lower daily doses can be appropriate. The administration of the daily dose can be carried out both by single administration in the form of an individual dose unit or else several smaller dose units and also by multiple administrations of subdivided doses at specific intervals.

The method by which the compound disclosed herein can be administered for oral use would be, for example, in a hard gelatin capsule wherein the active ingredient is mixed with an inert solid diluent, or soft gelatin capsule, wherein the active ingredient is mixed with a co-solvent mixture, such as PEG 400 containing Tween-20. A compound disclosed herein can also be administered in the form of a sterile injectable aqueous or oleaginous solution or suspension. The compound can generally be administered intravenously or as an oral dose of 0.1 µg to 20 mg/kg given, for example, every 3 - 12 hours.

Formulations for oral use can be in the form of hard gelatin capsules wherein the active ingredient is mixed with an inert solid diluent, for example, calcium carbonate, calcium phosphate or kaolin. They can also be in the form of soft gelatin capsules wherein the active ingredient is mixed with water or an oil medium, such as peanut oil, liquid paraffin or olive oil.

Aqueous suspensions normally contain the active materials in admixture with excipients suitable for the manufacture of aqueous suspension. Such excipients can be (1) suspending agent such as sodium carboxymethyl cellulose, methyl cellulose, hydroxypropylmethylcellulose, sodium alginate, polyvinylpyrrolidone, gum tragacanth and gum acacia; (2) dispersing or wetting agents which can be (a) naturally occurring phosphatide such as lecithin; (b) a condensation product of an alkylene oxide with a fatty acid, for example, polyoxyethylene stearate ; (c) a condensation product of ethylene oxide with a long chain aliphatic alcohol, for example, heptadecaethylenoxycetanol; (d) a condensation product of ethylene oxide with a partial ester derived from a fatty acid and hexitol such as polyoxyethylene sorbitol monooleate, or (e) a condensation product of ethylene oxide with a partial ester derived from fatty acids and hexitol anhydrides, for example polyoxyethylene sorbitan monooleate.

The pharmaceutical compositions can be in the form of a sterile injectable aqueous or oleagenous suspension. This suspension can be formulated according to known methods using those suitable dispersing or wetting agents and suspending agents that have been mentioned above. The sterile injectable preparation can also a sterile injectable solution or suspension in a non-toxic parenterally-acceptable diluent or solvent, for example, as a solution in 1,3-butanediol. Among the acceptable vehicles and solvents that can be employed are water, Ringer's solution, and isotonic sodium chloride solution. In addition, sterile, fixed oils are conventionally employed as a solvent or suspending medium. For this purpose, any bland fixed oil can be employed including synthetic mono-or diglycerides. In addition, fatty acids such as oleic acid find use in the preparation of injectables.

A compound disclosed herein can also be administered in the form of ophthalmic compositions applied topically to the eye, preferably in the form of eye drops. A compound disclosed herein can also be administered in the form of intravitreal injection.

A compound disclosed herein can also be administered in the form of suppositories for rectal administration of the drug. These compositions can be prepared by mixing the drug with a suitable non-irritating excipient that is solid at ordinary temperature but liquid at the rectal temperature and will therefore melt in the rectum to release the drug. Such materials include cocoa butter and polyethylene glycols.

The therapeutic compounds and/or compositions as used in the methods disclosed herein can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

For topical use, creams, ointments, jellies, solutions or suspensions, etc., containing therapeutic compounds and/or compositions, are employed.

In addition, some treatment compounds can form solvates with water or common organic solvents. Such solvates are encompassed within the scope of the methods contemplated herein.

### Dosage

The pharmaceutical compositions contemplated herein can be administered locally or systemically in a therapeutically effective dose. Amounts effective for this use will, of course, depend on the severity of the disease or disorder and the weight and general state of the subject. Typically, dosages used *in vitro* can provide useful guidance in the amounts useful for in situ administration of the pharmaceutical composition, and animal models can be used to determine effective dosages for treatment of particular disorders.

Various considerations are described, e. g., in Langer, 1990, Science, 249: 1527; Goodman and Gilman's (eds.), 1990, *Id.,* each of which is herein incorporated by reference and for all purposes. Dosages for parenteral administration of active pharmaceutical agents can be converted into corresponding dosages for oral administration by multiplying parenteral dosages by appropriate conversion factors. As to general applications, the parenteral dosage in mg/mL times 1.8 = the corresponding oral dosage in milligrams ("mg"). As to oncology applications, the parenteral dosage in mg/mL times 1.6 = the corresponding oral dosage in mg. An average adult weighs about 70 kg. *See* e.g., Miller-Keane, 1992, ENCYCLOPEDIA & DICTIONARY OF MEDICINE, NURSING & ALLIED HEALTH, 5th Ed., (W. B. Saunders Co.), pp. 1708 and 1651.

It is especially advantageous to formulate oral or parenteral compositions in dosage unit form for ease of administration and uniformity of dosage. "Dosage unit form" as used herein refers to physically discrete units suited as unitary dosages for the subject to be treated; each unit containing a predetermined quantity of active compound calculated to produce the desired therapeutic effect in association with the required pharmaceutical carrier. The unit dosage form can be a packaged preparation, the package containing discrete quantities of preparation, such as packeted tablets, capsules, and powders in vials or ampoules. Also, the unit dosage form can be a capsule, tablet, cachet, or lozenge itself, or it can be the appropriate number of any of these in packaged form. The details for the dosage unit forms of the invention are dictated by and directly dependent on the unique characteristics of the active compound and the particular therapeutic effect to be achieved, and the limitations inherent in the art of compounding such an active compound for the treatment of individuals. Such details are known to those of skill in the art.

The dosage administered will, of course, vary depending upon known factors, such as the pharmacodynamic characteristics of the particular agent and its mode and route of administration; the age, health, sex, weight, and diet of the recipient; the nature and extent of the symptoms; the kind of concurrent treatment; the time and frequency of treatment; the excretion rate; and the effect desired.

Therapeutically effective amounts for use in humans can be determined from animal models. For example, a dose for humans can be formulated to achieve a concentration that has been found to be effective in animals. The dosage in humans can be adjusted by monitoring enzymatic inhibition and adjusting the dosage upwards or downwards, as described above.

Dosages can be varied depending upon the requirements of the patient and the compound being employed. The dose administered to a patient, in the context of the methods disclosed herein, should be sufficient to affect a beneficial therapeutic response in the patient over time. The size of the dose also will be determined by the existence, nature, and extent of any adverse side effects. Generally, treatment is initiated with smaller dosages, which are less than the optimum dose of the compound. Thereafter, the dosage is increased by small increments until the optimum effect under circumstances is reached. The composition can, if desired, also contain other compatible therapeutic agents.

Dosage amounts and intervals can be adjusted individually to provide levels of the administered compound effective for the particular clinical indication being treated. This will provide a therapeutic regimen that is commensurate with the severity of the individual's disease state.

Utilizing the teachings provided herein, an effective prophylactic or therapeutic treatment regimen can be planned that does not cause substantial toxicity and yet is entirely effective to treat the clinical symptoms demonstrated by the particular patient. This planning should involve the careful choice of active compound by considering factors such as compound potency, relative bioavailability, patient body weight, presence and severity of adverse side effects, preferred mode of administration, and the toxicity profile of the selected agent.

A daily dosage of active ingredient can be expected to be from about 0.001 to about 1000 milligrams (mg) per kilogram (kg) of body weight, with the preferred dose being 0.01 to about 30 mg/kg. The quantity of active component in a unit dose preparation can be varied or adjusted from about 0.1 mg to about 10000 mg, more typically 1.0 mg to 1000 mg, most typically 10 mg to 500 mg, according to the particular application and the potency of the active component. In some embodiments of a method disclosed herein, the dosage range is 0.001% to 10% w/v. In some embodiments, the dosage range is 0.1% to 5% w/v.

Dosage forms (compositions suitable for administration) contain, e.g., from about 1 mg to about 500 mg of active ingredient per unit. In these pharmaceutical compositions, the active ingredient will ordinarily be present in an amount of about 0.5-95% weight based on the total weight of the composition.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Having described the invention in detail, it will be apparent that modifications, variations, and equivalent embodiments are possible without departing from the scope of the invention defined in the appended claims. Furthermore, it should be appreciated that all examples in the present disclosure are provided as non-limiting examples.

### EXAMPLES

The following non-limiting examples are provided to further illustrate embodiments of the invention disclosed herein. It should be appreciated by those of skill in the art that the techniques disclosed in the examples that follow represent approaches that have been found to function well in the practice of the invention, and thus can be considered to constitute examples of modes for its practice. However, those of skill in the art should, in light of the present disclosure, appreciate that many changes can be made in the specific embodiments that are disclosed and still obtain a like or similar result without departing from the spirit and scope of the invention.

### EXAMPLE 1

### Materials, Methods, and General Experimental Procedures

### Materials

Small molecule UC-764864 and UC-764865 were initially obtained from the University of Cincinnati-Drug Discovery Center's compound library. UC-764864 and UC-764865 were purchased from Mcule (Palo Alto, CA) or synthesized at Wuxi AppTec (Shanghai, China). Chemical structure of the compounds was analyzed by nuclear magnetic resonance (NMR). All chemicals were purchased from Sigma-Aldrich (St. Louis, MO) if not otherwise specified. All LC-MS grade solvents were obtained from J.T. Baker (Fisher Scientific; Hampton, NH).

### In Silico Screening of Compounds

Compounds for evaluation as inhibitors of UBE2N were selected by an aggregate docking study of a Cysteine targeted subset of the University of Cincinnati/Cincinnati Children's Hospital Compound Library. This diverse library of over 350,000 compounds was filtered (Dassault Systemes Biovia Pipeline Pilot 8.5.0.200) for compounds bearing functionality that may covalently bind to Cys87 in the UBE2N active site (e.g. α,β-unsaturated carbonyls and other moderately reactive electrophiles), removal of pan-assay interference compounds PAINS [59] structures and focused toward compounds of MW range from 180-450, resulting in a cysteine targeting sublibrary of 8929 compounds. There are more than a dozen crystal structures of UBE2N, with overall consistent structure except in the active site loop (ca. 115-124). To account for this variability, the Cys-directed library above was submitted for virtual screen (Molsoft ICM-Pro 3.8-0 Win.) against 7 crystal structures which represented the dominant conformations of this loop (PDB IDs: 1J7D, 3HCT, 3VON, 3W3I, 4DHI, 4JP3, and 4ONM). Compounds selected for assay showed a combination of strong ligand binding score and a predicted binding pose wherein Cys-87 was proximal to the reactive center of the ligand, such that covalent addition would be plausible. From this combination evaluation, the top 160 compounds were advanced to testing. The programs used for this analysis are described herein.

### Patient Samples

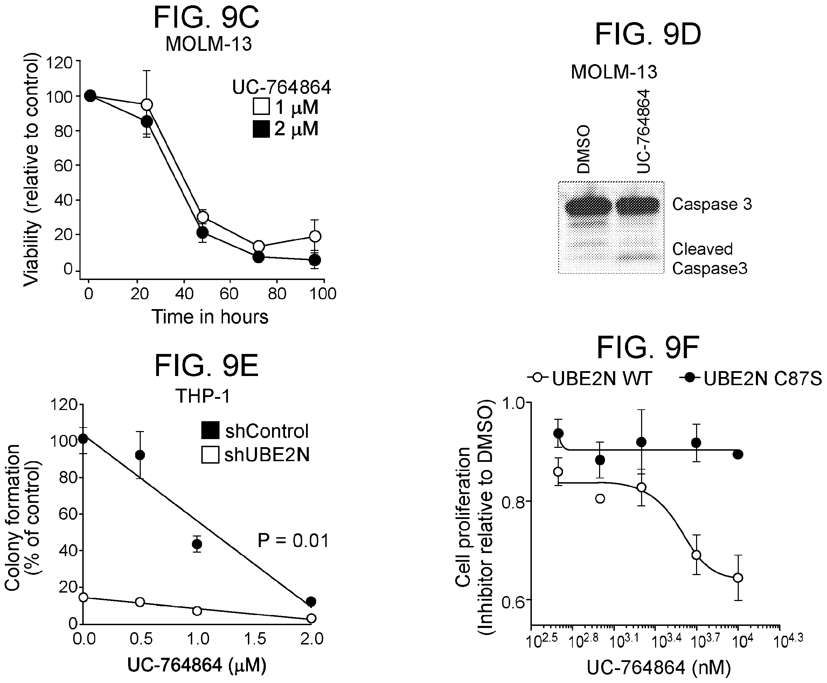

Bone marrow (BM) samples from 33 patients with AML or MDS at initial diagnosis were obtained with written informed consent and approved by the institutional review board of Cincinnati Children's Hospital Medical Center and University of Cincinnati, or from the Eastern Cooperative Oncology Group (ECOG). These samples had been obtained within the framework of routine diagnostic BM aspirations after written informed consent in accordance with the Declaration of Helsinki. For the *in vitro* cell proliferation studies in Figure 9E, de-identified, viably frozen, purified leukemic cells from peripheral blood and bone marrow of AML patients were obtained from patients at CCHMC consented under IRB approved Study ID # 2008-0021. Healthy BM specimens were obtained from ALLCells Inc. (Alameda, CA). In addition, human CD34+ umbilical cord blood (UCB) and adult BM-derived mononuclear cells (MNCs) were obtained from the Translational Research Development Support Laboratory of CCHMC under an approved Institutional Review Board protocol. Patient characteristics are shown in Table 1.

**Table 1. Patient sample characteristics.**

| **Specimen** | **Gender** | **Age at Diagnosis** | **Karvotype** |
|---|---|---|---|
| AML-17 (BM) | M | 56 | 47, XY,+4, add(6)(q24), add(14)(q24), del(21)(q22)[20] |
| AML-18 (BM) | M | 65 | 47, XY, del(5)(q31q35), +add(19)(p13.3), add(22)(p11.2)[1]/45, idem,-4, add(12)(p12), der(15;21)(q10;q10)[17]/46, XY[2] |
| AML-19 (BM) | F | 61 | 45,XX, -3,del(5)(q13q33), der(6)t(3;?;6)(p?;?;p23), der(16)t(3;16)(q23;q22)[19] |
| AML-20 (BM) | F | 49 | 44,XX, t(9;21)(q22;p13), der(13)t(13;18)(pI2;ql1.2), dic(18;?)(q11.2;?)dic(21;22)(q22;p11.2)[cp20] |
| AML-21 (BM) | M | 51 | 47,XY,+11[4]/47, idem,del(12)(p12)[6]/47, idem, del(9)(q13q22), del(12)(p12)[2]/46, XY[8] |
| AML-2017-103-4 (JM30) | M | 19 | FLT3 D835E - subclonal 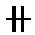 , D835V - subclonal 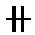 , D835Y - subclonal 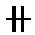 KIT D816V ASXL1 G645fs*58 CBL deletion exon 9 CHEK2 T367fs*15 PTPN11 D61V , inv(3) |
| AML-2017-94 (JM40) | F | 20 | CDKN2A/B p16INK4a loss and p14ARF loss exon 1 and CDKN2B loss |
| AML-2017-78 (JM01) | M | 25 | KRAS G12A - subclonal 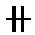 NRAS Q61H - subclonal 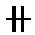 KMT2A (MLL) MLL-MLLT4 (AF6) fusion PTPN11 E76K - subclonal 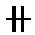 , S502L - subclonal 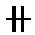 |
| AML-2017-63 (JM07) | F | 2 | t(7;12)(q36;p13) MNX1/ETV6 fusion PTPN11 p.D61V c.182A>T |
| AML-2017-42 (JM11) | F | 15 | MLL MLL-MLLT3 (AF9) fusion PC R583H - subclonal 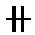 WT1 R462Q - subclonal 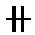 |
| AMI,-2017-14 (JM26) | F | 6 | FLT3 D835A - subclonal 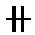 , D835E - subclonal 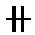 , D835Y -subclonal 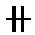 , I836del - subclonal 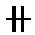 , V491L - subclonal 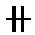 ,V579A - subclonal 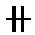 KRAS G13D MLL MLL-MVB12B fusion MLLT10 LL-MLLT10 (AF10) fusion |
| AML-2017-10-3 (JM18) | M | 2 | MLL inv(11)(p11.2q23) ; HPIM- β chain regulatory sequence on 7q fusion |
| AML-2016-35-2 (JM62) | F | 2 | RAM Immunophenotype |
| AML-2016-7-11 (JM65) | F | 17 | MLL MLL-MLLT3 (AF9) fusion |

**Table 1. Patient sample characteristics.**

| **Specimen** | **Gender** | **Age at Diagnosis** | **Karvotype** |
|---|---|---|---|
| AML-2016-1 (JM60) | F | 15 | NF1 Q1775* PTPN11 A461G- subclonal 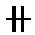 CDKN2A/B loss ETV6 loss MLLT10 PICALM-MLLT10 fusion PHF6 R274Q TP53 K164E - subclonal 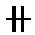 , Y126* |
| AML-2014-59-5 (JM20) | F | 21 | DNMT3AR882C FLT3 L576_Q577ins17 PTPN11 D61YNPM1 W288fs*10+ WT1 A382fs*11, A382fs*4 |
| AML-2013-7 (JM82) | M | 25 | 46,XY,t(11;19)(q23;p13.3)[8]/46,XY[12] MLL/ENL, APC/CBX3 |
| AML-2013-11( JM56) | F | 2 | 46,X,t(X; 11)(q24;q23), t(1; 10)(p22;p11.2), t(3; 14)(p25;q32), t(4;9)(q31;q21)[16]/46,XX[4] MLL/SEPT6 |
| AML-2013-28 (JM57) | F | 12 | 46, XX[20] FLT3-ITD |
| AML-2014-15 ( JM50) | M | 4 | 47, XY, t(2;5)(q31;q31),t(3;5)(p21;q31), ?der(14)t(14;17)(q24;q21), add(17)(q21),+21c[11]//46,XY[2] |
| AML-2014-31 (JM25) | M | 5 | not available t(X;7), del11q23? |
| AML-2015-37 (JM58) | M | 3 | 48,XY,+6,+19[cp20] NUP98/TAF3 |
| AML-2016-9 (JM66) | F | 1 | 50,XX, +der(6)t(6;11)(q27;q23)[19], t(6;11)(q27;q23), +8[19], +8[19], +19[19][cp20] MLL/AF6, FLT3-ITD/TKD, HOOK3/MYST3 |
| AML-2017-25 (JM17) | M | 7 | 46, XY, t(2;8)(p?24;p?12), add(9)(p21), der(11)add(11)(p15)ins(11;9)(q23;p21p21), del(17)(pI2pI2)[19]/46, |
| | | | sl,der(9)t(9;9)(q34;q22), der(9)add(9)(p22)t(9;9), t(13;14)(q32;q22), -15, +18, der(18)t(15;18)(q11.2;p11 MLL/AF9, WT1 |
| AML-2016-97 (JM68) | F | 2 | 46, XX,inv(7)(p13q36)[cp19]/46,XX[1] CBFA2T3/GLIS2 |
| AML-2016-99 (JM04) | F | 4 | 51, XX, t(1;22)(p13;q13), t(2;3)(p23;q26.2), +18, +19, +der919)t(1;19)(q23;p13.3) +20, +20[6]/51, sl, -t(2;3), +2, +3, der(6)t(3,6)(q23,p25)[3]/46XX[11] RBM15-MKL1 |
| AML-2016-102 (JM76) | M | 5 | 45, XY, i(5)(p10), -7,add(17)(p11.2)[5]/46, XY[2] del5q, TP53 |
| AML-2017-30 (JM51) | M | 16 | 46, XY, del(13)(q12q22), t(14;15)(p11.2;q12)[cp11]/46, XY[9] FLT3-ITD/TKD |

**Table 1. Patient sample characteristics.**

| **Specimen** | **Gender** | **Age at Diagnosis** | **Karyotype** |
|---|---|---|---|
| AML-2017-38 (JM02) | M | 2 | not available MLL/AF9, NRAS |
| AML-2017-114 (JM78) | M | 4 | 46,XY, der(10)?add(10)(p13)ins(10;11)(p12;q?23q?21), ?add(10)(p12), del(11)(q21q23)[14]/47, sl, +8[6] MLL/AF10, PTPN11 |
| AML-2018-10 (JM81) | M | 9 | 46, XY, del(9)(q12q34)[17]/46, XY[3] NUP98/NSD1, FLT3-ITD |
| UC-B1-MDS (PB) | not available | not available | not available |
| MDS04 | not available | not available | not available |
| HBM1 | M | 58 | normal |
| HBM2 | M | 50 | normal |

### Cell Lines

AML cell lines THP-1, KG-1a and HL-60, were purchased from the American Type Culture Collection (ATCC, Manassas, VA). MOLM-13 was purchased from AddexBio (San Diego, CA). OCI-AML2, OCI-AML3, NOMO-1 and SKM-1 were purchased from DSMZ. MDS-L and MDS92 were provided by Dr. Kaoru Tohyama (Kawasaki Medical School, Okayama, Japan) [60, 61]. MOLM-14 were provided by Dr. Neil Shah Lab (University of California, San Fran). MV4-11 cells were provided by Dr Grimes' lab (Cincinnati Children's Hospital Medical Center- CCHMC). Kasumi-1 was provided by Dr James Mulloy (CCHMC). THP-1, HL-60, MOLM-14, NOMO-1 and MOLM-13 were grown in RPMI medium supplemented with 10% Fetal Bovine Serum (FBS) and 1% penicillin/streptomycin. MDSL cells were grown in RPMI medium supplemented with 10% Fetal Bovine Serum (FBS, Atlanta Biologicals), 1% penicillin/streptomycin and IL-3 at 10 ng/ml. SKM-1 and Kasumi-1 cells were grown in RPMI medium supplemented with 20% Fetal Bovine Serum (FBS) and 1% penicillin/streptomycin. 293T cells were grown in DMEM medium supplemented with 10% FBS and 1% penicillin/streptomycin. THP-1-Blue NF-κB reporter cells (InvivoGen) were cultured in RPMI 1640, 2 mM L-glutamine, 25 mM HEPES, 10% heat-inactivated fetal bovine serum, 100 µg/ml NormocinTM, Pen-Strep (100 U/ml-100 µg/ml). KG-1a cells were grown in IMDM medium supplemented with 20% FBS and 1% penicillin/streptomycin. MDS92 cells were cultured in complete RPMI medium, supplemented with 10% fetal bovine serum, 1% penicillin-streptomycin and 50 ng/ml GM-CSF. MV4-11 cells were grown in IMDM medium supplemented with 10% FBS and 1% penicillin/streptomycin. All cell lines were authenticated and cultured at 37°C in 5% CO₂ atmosphere.

### Analysis of CRISPR Data

Pool-normalized sgRNA counts were downloaded for genome-wide human AML cell line screens and calculated CRISPR scores (by gene) as described in Wang *et al.* [15]. A CRISPR-score heatmap was generated using innate immune related genes from the canonical and non-canonical NFκB signaling pathways extracted from KEGG (Kyoto Encyclopedia of Genes and Genomes) [62-64].

### Quantitative Real-Time RT-PCR

mRNA expression of selected genes was corroborated by quantitative reverse transcriptase PCR (see primer sequences in Table 2) using cDNA with SYBR^{®} Green or Taqman^{®} gene expression assays (Applied Biosystems, Waltham, MA) on the StepOnePlus from Applied Biosystems. Abundance of each transcript was calculated using the Pfaffl Method [65]. Actin or GAPDH were used as housekeeping genes.

**Table 2. Primer Sequences.**

| **Gene name** | **RefSeq** | **Forward primer sequence** | **Reverse primer sequence** | **Source / Application** |
|---|---|---|---|---|
| *UBE2N* | NM_003348 | 5'- TGATGTAGCGGAGCAGTGGAAG -3' (SEQ ID NO:1) | 5'- GGAGGAAGTCTTGGCAGAACAG - 3' (SEQ ID NO:2) | Origene; qRT-PCR (sybr green) |
| *TRAF6* | NM_004620.3, NM_145803.2 | Taqman gene expression assay | | ThermoFisher #4331182-Hs00939742_g1; qRT-PCR |
| *GAPDH* | NM_001256799.2, NM_001289745.1, NM_001289746.1, NM_002046.5 | Taqman gene expression assay | | ThermoFisherI #4331182-Hs02758991_g1; qRT-PCR |
| *FOS* | NM_005252.3 | CCGGGGATAGCCTCTCTTACT (SEQ ID NO:3) | CCAGGTCCGTGCAGAAGTC (SEQ ID NO:4) | PrimerBank ID254750707c1; ; qRT-PCR |
| *JUN* | NM_002228.3 | TCCAAGTGCCGAAAAAGGAAG (SEQ ID NO:5) | CGAGTTCTGAGCTTTCAAGGT (SEQ ID NO:6) | PrimerBank ID44890066c1; qRT-PCR |
| *JUNB* | NM 002229.2 | ACAAACTCCTGAAACCGAGCC (SEQ ID NO:7) | CGAGCCCTGACCAGAAAAGTA (SEQ ID NO:8) | PrimerBank ID44921611c2; qRT-PCR |
| *IL6* | NM 000600.4 | 5'- GGTACATCCTCGACGGCATCT-3' (SEQ ID NO:9) | 5'-GT GCCTCTTTGCTGCTTTCAC-3' (SEQ ID NO:10) | Charlotte Keller et al J Physiol. (2003) ; qRT-PCR (sybr green) |
| *IL1B* | NM_000576.2 | 5’- AATCTGTACCTGTCCTGCGTGTT- 3’ (SEQ ID NO:11) | 5'- TGGGTAATTTTTGGGATCTACACTCT- 3' (SEQ ID NO:12) | doi: 10.1113/ jphysiol.2003.044883; qRT-PCR (sybr green) |
| *TNF* | NM 000594.3 | 5'-TCTTCTCGAACCCCGAGTGA- 3' (SEQ ID NO:13) | 5'-CCTCTGATGGCACCACCAG-3' (SEQ ID NO:14) | journal.pone.0002301.s001; qRT-PCR (sybr green) |
| *ACTIN* | NM 001101.3 | 5'-CTCTTCCAGCCTTCCTTCCT-3' (SEQ ID NO:15) | 5'-AGCACTGTGTTGGCGTACAG-3' (SEQ ID NO:16) | qRT-PCR (sybr green) |

### Lentiviral Vectors and Cell Transduction

For knockdown studies, UBE2N shRNA template oligonucleotides from the TRC shRNA library [66] cloned into the pLKO.1 TRC cloning vector (Addgene: #10878) and pLKO.1 TRC cloning vector (Table 3). The puromycin gene in pLKO.1 was replaced with GFP. pLKO.1 TRC control was used as non-silencing control (Addgene: #10879). For production of lentiviral particles, lentiviral shRNA expression constructs were transfected together with packaging vectors into 293T producer cells using TransIT transfection reagent (Mirus, MIR 2306), supernatants were harvested after 48 and 72 hours, and concentrated by ultracentrifugation. The THP-1, MOLM-13, HL-60 cell lines, CD34+ cord blood cells or patient derived AML cells were transduced with the short-hairpin-containing lentivirus and incubated for up to 15 days. After culture with fresh medium, transduced cells were selected with puromycin (2 or 2.5 µg/ml) or GFP-positive cells were sorted using a MoFlo XDP sorter (Beckman Coulter, Brea, CA) and used for experiments. UBE2N knockdown efficiency was determined by quantitative real time PCR (qRT-PCR) and western blot and quantified as described elsewhere [65, 67]. For UBE2N wild type or UBE2N C87S mutant overexpression, the corresponding cDNA were cloned in pCDH-EF1-MCS-IRES-GFP vector from Systems Biosciences (Palo Alto, CA) (CD530A-2). MOLM-13 cells were transduced and GFP positive cells were sorted 48 hours post transduction. Levels of overexpression of UBE2N were determined by western blot.

**Table 3. RNAi Sequences.**

| **Gene name/Symbol** | **RefSeq** | **label** | **Sequence** |
|---|---|---|---|
| UBE2N* | NM_003348 | shUBE2N-2 | 5'-CCGG-CTAGGCTATATGCCATGAATA-CTCGAG-TATTCATGGCATATAGCCTAG-TTTTTG-3' (SEQ ID NO: 17) |
| UBE2N* * | NM_003348 | shUBE2N-3 | 5'- CCGG-AGACAAGTTGGGAAGAATATG-CTCGAG-CATATTCTTCCCAACTTGTCT-TTTTTG-3' (SEQ ID NO: 18) |
| UBE2N* | NM_003348 | shUBE2N-1 | 5'CCGG-GCTGAGGCATTTGTGAGTCTT-CTCGAG-AAGACTCACAAATGCCTCAGC-TTTTT3' (SEQ ID NO: 19) |
| Non-silencing control* * * | | shControl | 5'CCGGCAACAAGATGAAGAGCACCAACTCGAGTTGGTGCTCTTCATCTTGTTGTTTTT3' (SEQ ID NO:20) |

| | | | |
|---|---|---|---|
| All vectors: pLKO.1 *Source: Cincinnati Children's Robotic Lenti library Core **Source: SIGMA SHCLND ***Source: SIGMA SHC202 | | | |

### Xenotransplantation Assays and In Vivo Studies

Lentivirally-infected MOLM-13 cells or HL-60 cells (1.9 x 10⁴ cells per recipient) expressing a non-silencing control shRNA or UBE2N specific shRNAs were tail vein transplanted into sublethally irradiated NOD-scid IL2R_{γ}^{-/-} (NSG) mice (250 rads whole body irradiation). Moribund mice were sacrificed and assessed for leukemic burden measurements. BM and spleen cells were analyzed for GFP expression by flow cytometry using a BD FACS Canto System (BD Biosciences, San Jose, CA). Bone marrow aspirates were taken 2 weeks after transplantation for smears or to assess engraftment by flow cytometry. For survival analysis after UBE2N inhibitors treatment, NSG mice (n = 10/group) were injected with 1 x 10⁴ MOLM-13 cells and treated with 4 doses of 2 mg/kg/d of UC-764865, or vehicle control (0.2% tween 20 in water) for 2 weeks (8 doses total). Time of death was recorded, and Kaplan Meier survival analysis was performed using GraphPad Prism version 7.00 for Mac (GraphPad Software, La Jolla California USA). Engraftment was assessed in bone marrow (BM) at one week after transplantation by flow cytometric determination of human CD33 (BDPharmingen 555450, Franklin Lakes, NJ), human CD45 and/or human CD15. Briefly, mice were euthanized with carbon dioxide following the AVMA Guidelines for the Euthanasia of Animals and BM cells were immediately extracted by breaking the femurs with a mortar and pestle. Red blood cells were lysed with RBC lysis buffer (555899 BD Biosciences) and washed twice with Phosphate-Buffered Saline (PBS) (Dulbecco's 45000-446 (PK)), 2% Fetal Bovine Serum (FBS). 1 x 10⁶ cells were stained with human CD33 (BDPharmighen 555450), murine CD45 (BDPharmighen 557659) by incubating in PBS, 0.2% FBS for 30 minutes on ice in the dark. After staining the cells were washed once with PBS and resuspended in incubation buffer with propidium iodine. Cells were analyzed using a BD FACS Canto System (BD Biosciences, San Jose, CA). Alternatively, 1 x 10⁴ MOLM-13 cells were xenotransplanted in NSG mice without conditioning (n=10/group). Treatment with UBE2N inhibitors was started one day after transplant for 7 days (5 days dosing of 2 mg/kg/d, resting 2 days, 2 days dosing of 2 mg/kg/d) by intraperitoneal injections (IP). All the mice were sacrificed at day 10 post transplantation and engraftment of MOLM-13 cells was analyzed in spleen and BM by staining for human CD15 (BDPharmighen 551376) as described above. For patient-derived xenografts (PDX), 1X106 to 6 x 10⁶ cells were transplanted via tail vain in NSGS mice conditioned with busulfan (final dose of 30mg/kg) 24 hours prior to transplantation. Treatment with UC-764865 was started 3 days after transplantation with 5 weekly doses of 25mg/kg UC-764865 or vehicle control (methyl-β-cyclodextrin, 50mg/ml) via I.P during a period of 2-6 weeks. BM aspirates were performed once a week and determination of engraftment was performed as described above.

### In Vivo Preclinical Pharmacokinetic Analysis and Toxicity Studies

PK studies of UC-764864 and UC-764865 were performed in C57BL/6 mice (18 - 22 g). 10 mg/kg and 20 mg/kg UC-764864 or UC-764865 was administered through intraperitoneal (i.p.) injection. After dosing, blood samples were collected at 15 min, 30 min, 1 h, 2 h, 4 h, 8 h, 12 h, and 24 h. Samples from three animals were collected at each time point. Approximately 500 µL of blood was collected via orbital vein from each mouse, processed, and analyzed by LC-MS. Standard curves were prepared in blood covering the concentration range of 50 - 3,0000 ng/mL. Using the data from the standard curves, calibration curves were generated for PK tests. For *in vivo* toxicity studies, 25 mg/kg UC-764864, UC-764865, 10 mg/kg NSC697923 or vehicle control (methocel 0.5% tween-20 0.2%,0.1% DMSO in water) were administered in B6.SJL-Ptprca Pepcb/BoyJ mice (n = 3/group) through i.p. injection. For 2 weeks the mice received 4 doses of each compound. Routine body weight and blood counts were performed. At the end of the study, tissues were harvested, placed in 10% buffered formalin, stained with hematoxylin and eosin (H&E) and examined by a pathologist.

### Linear Mass Spectrometry, Differential Scanning Fluorimetry (DSF) and Cellular Target Engagement Assay (CETSA) Analysis of Drug Binding

Recombinant UBE2N (2 .tg; Life sensors # UB218) in 3 .tl of buffer containing 20 mM Tris-HCl pH 7.4, 10 mM DTT, 10% glycerol, 150 mM NaCl and 0.02% DMSO as well as six samples of 1 .tg UBE2N mixed with 0.2 .tg of UC-764864 or UC-764865 were diluted to 50 ng/uL in 0.1% formic acid (FA) in preparation of MALDI-TOF analysis. Two .tl aliquots from the samples were used to make a 1:5 dilution with MALDI matrix (5 mg/mL sinapic acid in 60% ACN/0.1% formic acid) with 1 .tL spotted onto the target plate for analysis. For UBE2N C87S mutant, recombinant protein was purchased from Genescript. 2 micrograms of protein as well as 3 samples of UBE2N C87S were mixed with 0.4 micrograms of UC-764864 and UC-764865 in 10 microliters of buffer containing 20 mM Tris HCl pH 7.4, 1 mM DTT, 150 mM NaCl and ~0.01% of DMSO in each sample. All mixtures were brought up to 40 ul by adding 30 ul of 0.1% formic acid (FA). 5 ul aliquots from the samples were used to make a 1:5 dilution with MALDI matrix as described above. All spectra were acquired in Linear Positive Ion Mode on an ABSciex 4800 MALDI-TOF/TOF instrument, with *E. coli* thioredoxin (11,674.5; MH+ avg) and horse muscle apomyoglobin (15,952.6; MH+ avg) as mass calibration standards. For DSF, all proteins (UBE2N wild type and UBE2N C87S) were used at a final concentration of 5.tM. SYPRO Orange (Invitrogen S6651, Carlsbad, CA) was used at a final concentration of 10X for UBE2N wild type and UBE2N C87S. Experiments were carried out in 20 mM Tris-HCl pH 8, 150mM NaCl, 1 mM TCEP, and 10% DMSO. UC-764865 was mixed with the proteins at increasing concentrations, each sample was divided into three 25 .tl replicates in 48-well optical plates (Thermo Fisher Scientific 4375816, Waltham, MA) and the plate was sealed with optical PCR plate sheet (Thermo Fisher Scientific 4375928). All experiments were performed on a StepOne Real-Time PCR System (Applied Biosystems 4376357, Foster City, CA). The temperature was raised in 1°C increments from 25°C to 95°C and fluorescence was measured at 530 nm. Derivative Tm was determined using Protein Thermal Shift Software version 1.3 by Applied Biosystems and then were analyzed with Prism 6 (GraphPad Software Inc.).

CETSA was performed with MOLM-13 cells cultured in RPMI medium supplemented with 10% FBS. For an initial determination of the melting profile of UBE2N, fresh cell lysate prepared in non-denaturing buffer was dispensed into 96-well PCR plate in the above medium (approx. 8000 cells/well/50 µl), then was subjected to temperature gradient (37-60°C) for 20 min. Subsequently, centrifugation was performed at 14,000 rpm to sediment the unstable protein content. Supernatant was collected and SDS-PAGE gel was run, and immuno-detection was performed for UBE2N using corresponding primary antibody. Band intensity was quantified on LI-COR C-Digit Blot Scanner, and subsequently Tagg(50) and Tagg(75) values were calculated for UBE2N. In a subsequent run, fresh lysates of MOLM-13 cells were treated at various doses with 4-fold dilutions (10, 2.5, 0.62, 0.15, 0.04, 0,01 and 0.002 .tM) of compounds UC-764864 and UC-764865 together with DMSO control, for 1 hour. Samples were then subjected to heat challenge at Tagg(75) for 20 min, and unstable protein was removed by centrifugation step. Following an immuno-blotting step, bands of remaining stable UBE2N were quantified, normalized to loading control and plotted using GraphPad Prism software. EC50 values of engagement for both compounds with UBE2N were subsequently calculated.

### Analysis of Cell Morphology

Bone marrow aspirates or peripheral blood smears were spread onto a glass slide and stained with Wright-Giemsa stain using an automatic slide stainer (Hematek, Siemens, Lebanon, NJ). For MOLM-13, 200,000 cells were cytospun in coated glass slides for 5 minutes at 800 rpm with low acceleration. The slides were stained with Giemsa staining using an automatic stainer. Pictures were acquired with an Olympus LC30 camera and Motic BA310 microscope at the indicated magnification.

### Cell Proliferation Assays and Cell Viability

AML cell lines (5 x 103 cells/well) were seeded in 96-well plates and grown for 24 to 96 h in serum (10%)-containing medium in the presence or absence of inhibitors at the indicated concentrations. Proliferation was analyzed by means of an MTS assay using the CellTiter 96 Aqueous One Solution Cell Proliferation Assay (Promega, Madison, WI, USA), according to the manufacturer's instructions. Data are mean with standard deviation from three independent experiments in technical triplicates. For the screening of 160 compounds from the UC library, the cell metabolic activity was assessed at 24 hours of treatment of MOLM-13 cells. Top 15 candidates were re-screened at 2 µM, and the cell metabolic activity was determined at 24 hours of treatment. In certain experiments, cell viability was determined by staining the cells with trypan blue at a 1/2 dilution and counting total number of viable cells upon treatment with UC-764864 or DMSO control. The cells were counted daily for 4 days using an automated cell counter (Countess II FL from Life Technologies). The cell proliferation in primary AMLs in Figure 9E was performed as follows: 50,000 cells were plated in duplicate in a 96 well plate in IMDM containing 20% heat inactivated FBS plus 10 ng/ml of each SCF, IL3, IL6, TPO, and FLT3. UC-764864 was added at the indicated concentrations and cells were incubated with drug for 3 days, at which point cell viability was measured by MTS according to manufacturer's instructions.

### Thioester Bond Formation Assay

An *in vitro* Ubiquitin-E2 thioester (TE) bond formation assay was performed as described (Enzo Life Science, #BML-UW9920-0001, Farmingdale, NY). The reaction was prepared as follows: 10X E2 (UBE2N/Mms2, No.BML-UW9565; UbcH5a, No. BMI,-UW9050; or UbcH6, Prod. No. BML-UW8710) were incubated with UC-764864 or UC-764865 at 1 or 2 µM for 30 minutes at room temperature. After the incubation period, the following reagents were added to the reaction: distilled water, 10X ubiquitination buffer, inorganic pyrophosphatase solution (100 U/mL in 20 mM Tris-HCl, pH 7.5, Sigma, 83205, St. Louis, MO), 50 mM DTT, 0.1 M Mg-ATP, 20X E1 (recombinant human ubiquitin-activating enzyme) and 20X biotinylated ubiquitin to a final volume of 10 p.l. The mixture was incubated for 4 hours at 37°C. The reaction was stopped by adding 10 p.l 2X non-reducing gel loading buffer. 15 p.l of the samples were run on SDS-PAGE gels and transferred to PVDF membrane. The membrane was blocked with BSA/TBS-T blocking buffer for 1 hour at room temperature on a rocking platform, washed for 3 x 10 mins with TBS-T on a rocking platform and incubated with Streptavidin-HRP solution (Jackson ImmunoResearch, 016-030-084, West Grove, PA) for 1 hour at room temperature on a rocking platform. After washing the membrane for 6 x 10 mins with TBS-T on a rocking platform, total ubiquitin was detected with ECL detection reagent (PierceTM ECL Western Blotting Substrate, #32106, Appleton, WI) according to the manufacturer's instructions. Detect emitted signal with Biorad ChemiDocTM MP and analyzed with Image lab software 6.0.1 (Biorad, Hercules, CA) or Image J [67]. For detection of UBE2N, membranes were stripped with hydrogen peroxide for 30 minutes at 37°C and re-blotted with UBE2N antibodies.

### Ubiquitin-Enrichment Screen by Mass Spectrometry

Details of the ubiquitin-enrichment screen are described herein. Briefly, MOLM-13 cells were treated for 24 hr. with 2 µM of UC-764864. Ubiquitin-related peptide enrichment was performed by using ubiquitin remnant motif (K-ε-GG) antibody-conjugated beads (Cell Signaling Technology, Danvers, MA) following the manufacturer's instructions. Tryptic peptides were loaded onto the beads, and then ubiquitin-conjugated peptides were eluted from the bead. Nanoliquid chromatography coupled to electrospray tandem mass spectrometry (nanoLC-ESI-MS/MS) analyses were performed on a TripleTof 5600+ mass spectrometer (Sciex; Concord, Ontario, Canada) coupled with a nanoLC-ultra nanoflow system (Eksigent; Dublin, CA) in data dependent acquisition (DDA) or data independent acquisition (DIA) modes for Sequential Window Acquisition of all Theoretical mass spectra (SWATH-MS) analysis.

### Immunoblots

For immunoblots, total protein lysates were obtained from cells by lysing the samples in cold RIPA buffer (50mM Tris-HCl, 150 mM NaCl, 1 mM EDTA, 1% Triton X-100 and 0.1% SDS), in the presence of PMSF, sodium orthovanadate, protease and phosphatase inhibitors. After being re-suspended in RIPA, cells were lysed in the cold room with rocking for 15 min. Protein concentration was evaluated by a BCA assay (Pierce, Waltham, MA). SDS sample buffer was added to the lysates and the proteins were separated by SDS-polyacrylamide gel electrophoresis, transferred to PVDF or nitrocellulose membranes, and analyzed by immunoblotting. Western blot analysis was performed with the following antibodies: UBE2N (Abcam, ab25885, Cambridge, UK; Cell Signaling Technology, #6999 or #4919S, Danvers, MA), Vinculin (Cell Signaling, 13901T), p65 (Cell Signaling Technology 8242), p50 (Santa Cruz, sc-7178, Santa Cruz, CA), RelB (Santa Cruz, sc-226), STAT1 (Cell Signaling Technology, 9172S), LaminB (Cell Signaling Technology, 12586S) , Caspase 3 (Cell Signaling Technology, #9665), and Actin (Cell Signaling Technology, 4968). Membranes were imaged with Biorad ChemiDocTM MP and analyzed with Image lab software 6.0.1 (Biorad, Hercules, CA) or Image J [67].

### NF-κB Activation Assay

THP1-Blue NF-κB reporter cells (Invivogen) were diluted to 100,000 cells/mL and stimulated with 1 µg/mL IL1β, 100ng/ml Pam3CSK4 or 1 µ/m LPS. The cells were subsequently seeded into a 96 well plate at 20,000 cells per well (200 µl) and introduced to drugs in an increasing concentration. The plates were incubated at 37C, 5% CO₂ for 24 hours. The following day, QuantiBlue Reagent was warmed to 37C in a water bath and 180 µl was added to each well of a separate, clean 96 well plate. The incubated cells were spun down, and the supernatant was taken for the assay. To perform the assay, 20 µl of supernatant from each well was pipetted into the respective 180 µl QuantiBlue Reagent well, in triplicate. The reaction was mixed and incubated for 1 to 4 hours, when a color gradient could be seen. The absorbance was read at 630 nm for a final readout.

### FACS of Stem and Progenitor Cells

To purify the stem and progenitor compartments from total BM of AML or MDS patients, samples were processed as follows: Bone marrow mononuclear cells were obtained from fresh BM aspirates by Ficoll separation following Miltenyi Biotech protocol. Cells were resuspended in MACS buffer (Phosphate Buffer Saline (PBS) supplemented with 0.5% Bovine serum albumin (BSA) and 2mM EDTA, pH 7.2). CD34+ cells were immunomagnetically selected from mononuclear cells from AML/MDS patients and healthy donors utilizing Miltenyi MACS technology (130-046-702, Miltenyi Biotech, Bergisch Gladback, Germany) according to the manufacturer's protocol. Afterward, cells were stained for 30 minutes in the dark and on ice with Percp-Cy5.5 conjugated antibodies against lineage antigens (CD2 [RPA-2.10], CD3 [UCHT1], CD 4[S3.5], CD7 [6B7], CD8 [3B5], CD10 [CB-CALLA], CD11b [VIM12], CD14 [TueK4], CD19 [HIB19], CD20[2H7], CD24 [Biolegend, 311115, San Diego, CA], CD56 [MEM-188], Glycophorin A [CLB-ery-1(AME-1)]), and hematopoietic stem and progenitor markers (APC conjugated CD34[581/CD34(class III epitope), PE-CY7 conjugated CD38[HIT-2], FITC conjugated CD45RA [MEM-56], PE conjugated CD123 [6H6] and APC-Cy7 conjugated CD90 [5E10]) in order to distinguish LT-HSC (Lin-/CD34+/CD38-/CD90+/CD45RA-), MPP (Lin-/CD34+/CD38-/CD90-/CD45RA-) and Progenitors (Lin-/CD34+/CD38+). After staining, cells were washed with MACS buffer and subjected to 7-color sorting using a FACSAria II Special Order System (BD Biosciences, San Jose, CA). Cells were sorted directly into RLT plus buffer (Qiagen, La Jolla California) for RNA extraction.

### Gene Expression Analysis

For gene expression analysis from paired AML samples (n = 56) RNAseq bam files were aligned using STAR (v 2.4.0f1) [68] to human genome reference hg19 using Gencode v19 [69] transcriptome assembly for junction points. RNAseq counts were annotated by using subread (v 1.4.5-p1) featureCounts [70]. Counts were normalized to TPM (Transcripts Per Kilobase Million) [71]. Comparison analysis of transcript levels was calculated using DESeq2 [72] by comparing each diagnosis or relapse sample to the healthy controls in the same batch of sequencing. Heatmaps and word clouds of innate immune genes were generated in R. Dbgap: accession number for the RNA-seq data is phs001027.v2.p1.

Functional enrichment analysis was performed using the gene set enrichment analysis (GSEA) [73] method. The log fold change resulted from the DESeq2 analysis for diagnosis or relapse samples against normal controls was ranked from highest to lowest. This ranked list was used as input into the GSEAPreranked algorithm (GSEA v2-2.1.0) against immune-related gene ontology genesets (MSigDB v 5.2) to determine gene enrichment analysis of innate immune related GO terms in BM cells (PAML) and PB cells (SGUA) of AML patients at time of diagnosis (data not shown). Default permutation settings were used for significance determination. Genesets with an absolute NES score greater than 1.5 and an adjusted P value less than 0.01 were identified to be significantly enriched.

For gene expression analysis in sorted hematopoietic cells, total RNA was extracted from sorted long-term hematopoietic stem cells (LT-HSC), short-term hematopoietic stem cells (ST-HSC), and granulocytic monocytic progenitors (GMP) populations from AML patients with complex karyotype using a denaturing buffer containing guanidine isothiocyanate (ALLPrep Micro Kit, QIAGEN, Hilden, Germany). After checking the quality of RNA with an Agilent 2100 Bioanalyzer, total RNA was amplified using the Single Primer Isothermal Amplification (SPIANugen Ovation pico WTA) system according to the manufacturer's instructions. After labeling with the GeneChip WT terminal labeling kit (Affymetrix, Santa Clara, CA), labeled cRNA of each individual sample was hybridized to GeneChip Human Gene 1.0 ST microarrays (Affymetrix), stained, and scanned by GeneChip Scanner 3000 7G system (Affymetrix) according to standard protocols. The complete array data are deposited in the gene expression omnibus database (www <dot> ncbi <dot> nlm <dot> nih <dot> gov <slash> geo; accession number GSE115154) according to MIAME standards. Raw data from AML or healthy controls (GSE35010 and GSE35008) was normalized in Expression Console 1.2 software from Affymetrix using Robust Multichip Average. Normalized data were analyzed in MeV Version 4.7.3 software, for differential gene expression between groups using Welch t test with a significance level of P <0.05. Genes with an absolute value of the group mean difference equal or greater than 1.5 (log2 scale) and P values < 0.05 were called as differentially expressed between groups. After filtering out unannotated and duplicate genes, the remaining genes were clustered by hierarchical clustering, using Euclidean distance, complete linkage clustering, using MeV. Gene set and pathway enrichment analysis was performed using DAVID 6.8 [74, 75] and the Cytoscape [76] plugin Gene Enrichment Map [77] was used to generate graphic display of gene enrichment data. Heatmaps of gene expression of innate immune genes were generated in MeV (http <colon slash slash> mev <dot> tm4 <dot> org).

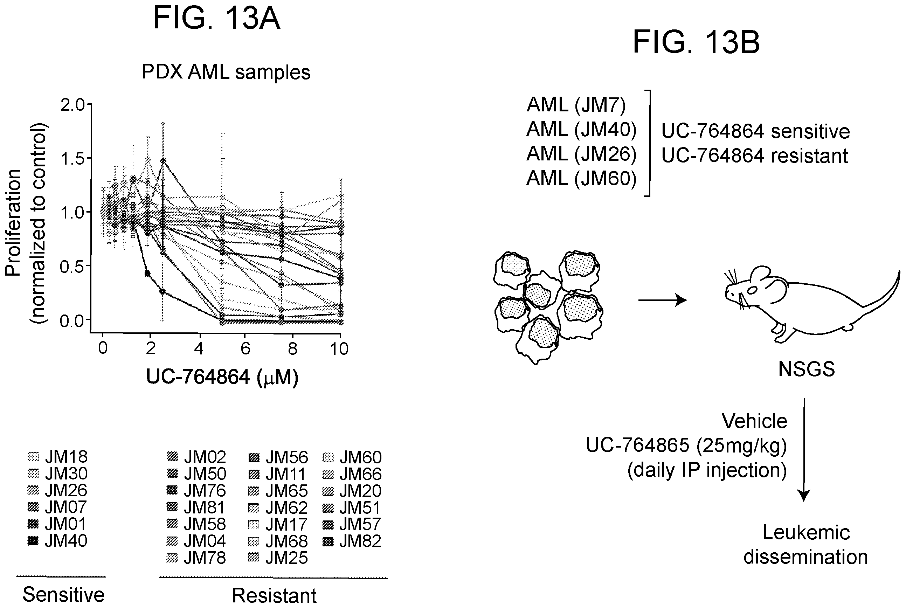

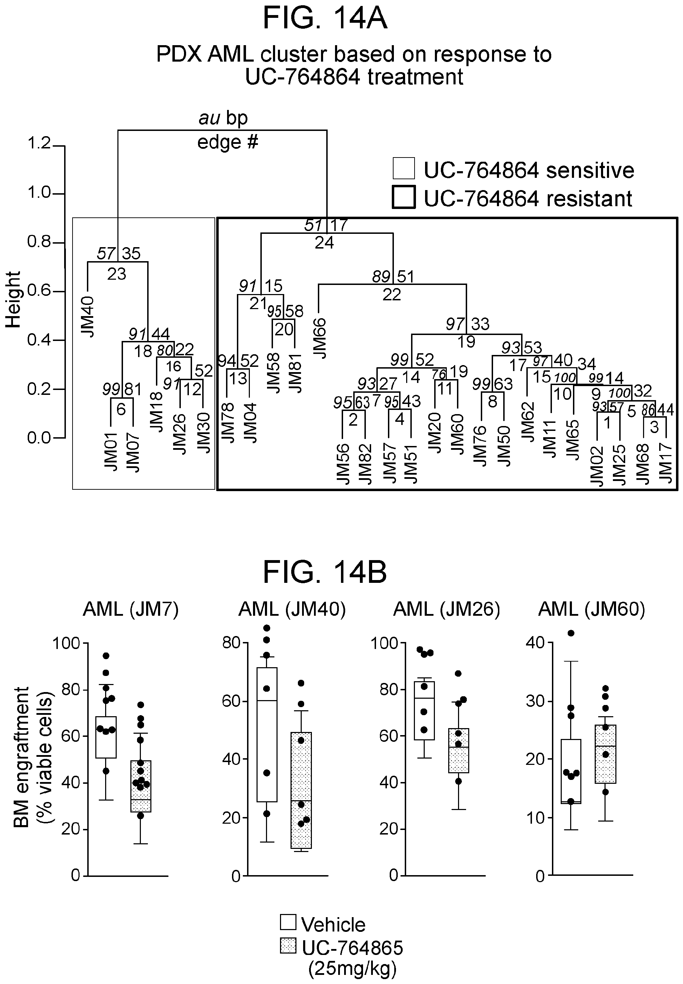

To classify the PDX AMLs from Figure 13A into sensitive or resistant to UC-764864, an unbiased analysis was performed, as shown in Figure 14A. First, the Euclidean distance among PDX samples was calculated, then average-linkage method was used (i.e. the distance between two clusters is defined as the average distance between each point in one cluster to every point in the other cluster) to build a final hierarchical clustering. For each cluster, p-values were calculated via 5000 bootstrap resampling. The plot in Figure 14A provides two types of p-values: AU (Approximately Unbiased-left side) p-value and BP (Bootstrap Probability-right side) value. AU p-value, which is computed by multiscale bootstrap resampling, is a better approximation to unbiased p-value than BP value computed by normal bootstrap resampling. The AU p-value > 90% corresponds to p-value < 10%.

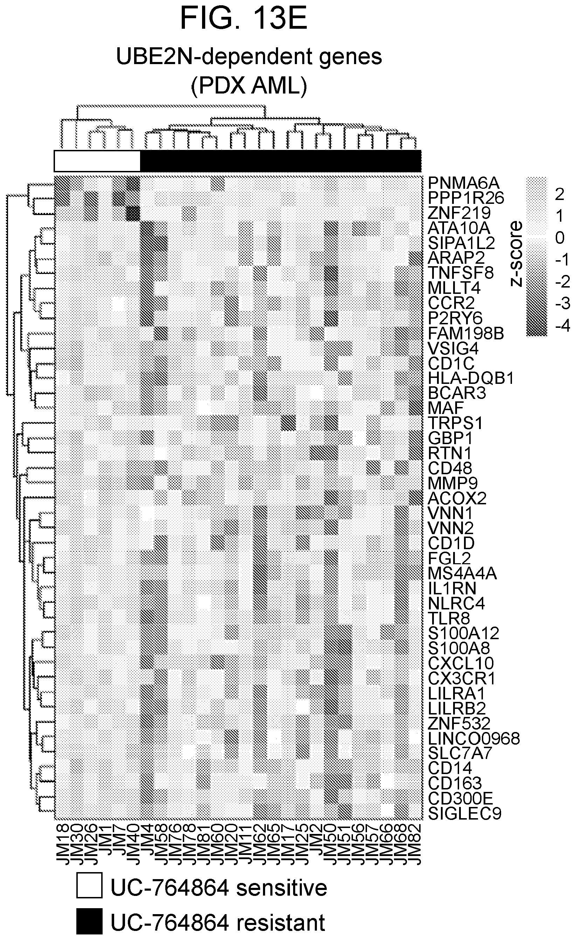

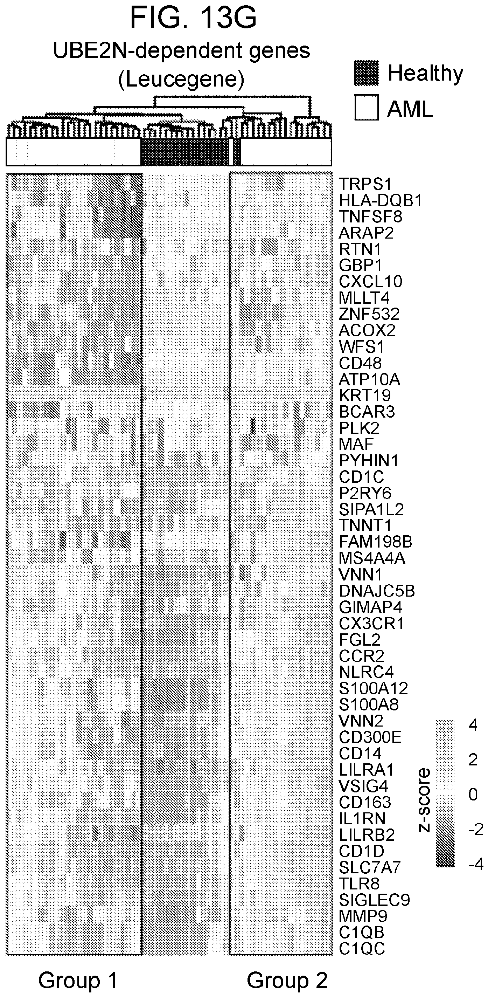

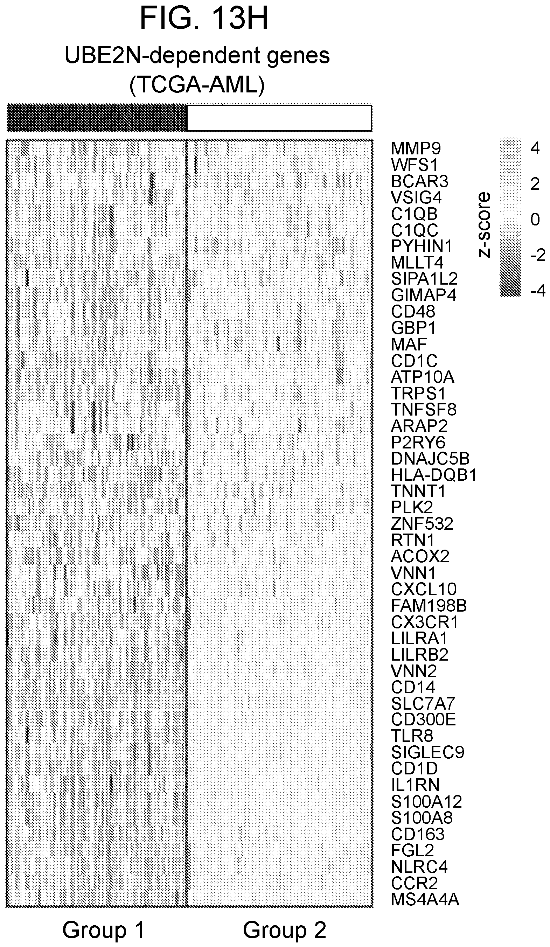

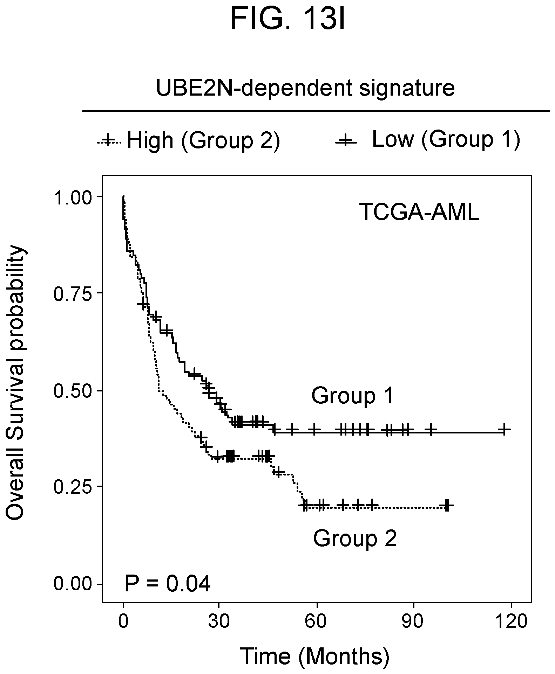

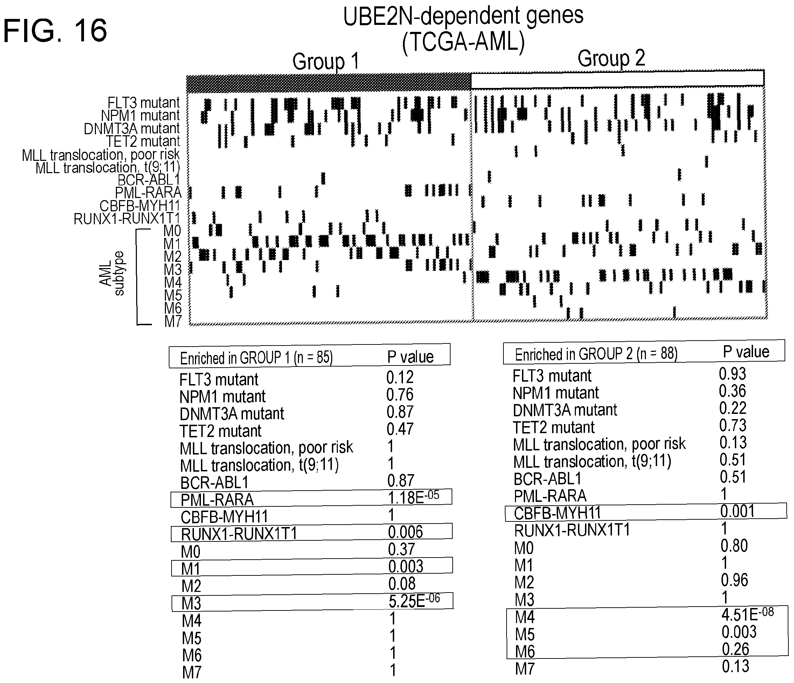

For the analysis in Figure 13E, a heatmap was generated to visualize the expression patterns of UBE2N-dependent genes across UC-764864 sensitive/insensitive AML patient samples. For the analysis in Figure 13G, RPKM-normalized gene expression data were downloaded from AML patients (GSE49642) and healthy controls (GSE48846), then a heatmap was generated using UBE2N-dependent immune genes and log2-RPKM values. For the analysis in Figure 13H, 173 TCGA-AML RNA-seq v2 data (RSEM) were downloaded from the Broad TCGA-GDAC site (https <colon slash slash> gdac <dot> broadinstitute <dot> org) and the RSEM-normalized gene expression values of UBE2N-dependent immune genes across samples were extracted. From this subset matrix, two major AML patient groups were identified using sample-wide hierarchical clustering analysis (with the complete linkage method in R v3.5.1). For the analysis in Figure 13I, based on two major AML groups from the previous hierarchical clustering analysis on 173 TCGA-AMI, dataset (a subset of UBE2N-dependent immune genes), we performed a survival analysis (survival package in R v3.5.1) to show a differential survival patterns between two groups. In Figure 16, hypergeometric tests were applied to determine which mutations or AML subtypes were statistically enriched in group 1 or 2.

### Flow Cytometric Determination of Cell Death and Clonogenic Assays

In order to determine viability after UBE2N knockdown or inhibition, 1 x 10⁶ AML cells were washed with PBS and mixed with pre-diluted APC-conjugated Annexin V (BD Pharmigen, Franklin Lakes, NJ) and Propidium iodide (PI) (Invitrogen ThermoFisher 00-6990-42, Waltham, MA). Cells were stained at room temperature for 15 minutes and suspended in 0.5 ml of Annexin V incubation buffer (eBiosciences 88-8007-74, San Diego, CA) for analysis using a BD FACS Canto System (BD Biosciences, San Jose, CA). For UBE2N knockdown experiments, PI-/GFP+ cells were sorted using a MoFlo XDP sorter (Beckman Coulter, Brea, CA) and plated in methylcellulose (StemCell Technologies H4236, Vancouver, CA) at 1000 cells/ml in 35 mm culture dishes. For UBE2N inhibition experiments, cell lines were plated in methycellulose (StemCell Technologies H4236, Vancouver, CA) at the indicated concentrations of inhibitor. Cells were incubated at 37°C and 5% CO₂. Colonies were scored after 7 days in culture. For UBE2N or UBE2N C87S overexpression experiments, MOLM-13 cells were transduced with lentivectors overexpressing UBE2N wild type or mutant in the presence of polybrene (8 µg/ml). GFP+/PI- cells were sorted at 48 hours and transduced with shUBE2N-2 expressing a puromycin resistant cassette. After one week with puromycin selection (2.5.tg/ml), 1000 cells were plated onto 1 ml of serum free human methylcellulose (Stem Cell Technologies H4236, Vancouver, CA) in the presence of 2.5 µg/ml of puromycin and antibiotics. Colonies were scored at 7 days. For primary cells (cord blood CD34+ cells, MDS primary samples and patient derived xenografts), the colony assays were performed as previously described [85] using methylcellulose from StemCell technologies H4434. Colonies were manually scored at 14-16 days after plating or counted with STEMVision (StemCell Technologies, Vancouver, CA).

### Immunofluorescence Assays

For determination of yH2AX foci, cells were collected by centrifugation after being treated under experimental conditions and resuspended in 1X PBS at a concentration of 500,000 cells/mL. 50,000 cells were spun down at 500rpm for 5 minutes with low acceleration. The slides were then submerged in fixative solution (1X PBS, 4% paraformaldehyde, 0.1% Triton X-100) for 10 minutes at room temperature. The slides were washed by submerging into 1X PBS for 2 minutes at room temperature for a total of 3 washes. They were then blocked with 200µl of 1X PBS, 3% BSA, 0.1% Tween20 for 30 minutes at room temperature and washed twice with 1X PBS. Primary antibody for γH2AX was diluted in PBS, 1% BSA, 0.1% Tween20 and incubated on the slides for 1 hour at room temperature. The slides were washed 3 times with 1X PBS and incubated with secondary antibody diluted in 1X PBS, 1% BSA, 0.1% Tween20 for 1 hour at room temperature, protected from light. After 3 washes in 1X PBS, the slides were submerged in fixative solution and incubated for 10 minutes at room temperature then washed 3 more times in 1X PBS. Finally, Prolong Gold was placed on the slides, coverslips were mounted, and the slides were stored at 4C until ready to image. Images were acquired using an Upright Zeiss Axio imaging microscope (Zeiss, Oberkochen, Germany) with a GFP filter and DAPI filter. Images were saved as. nd2 files and analyzed for foci within the nuclei using NIS-Elements Microscope Imaging Software (Nikon, Tokyo, Japan).

### Ubiquitin-Enrichment Screen by Mass Spectrometry

MOLM-13 cells were treated for 24 hours with 2 µM of UC-764864. The identification of ubiquitinated peptides was performed as follows: approximately 3 x 10⁸ cells were lysed in 10 mL urea-lysis buffer (20 mM HEPES pH 8.0, 9 M urea, 1 mM sodium orthovanadate, 2.5 mM sodium pyrophosphate, 1 mM β-glycerophosphate) and then sonicated at 15W x 3 bursts of 15 sec with cooling on ice between each burst. The lysate was collected by centrifuge at 20,000 xg, 15 min, 15°C. Protein estimation was performed by Pierce660 protein assay (ThermoFisher; Florence, KY). For the In-solution tryptic digestion, cell lysate proteins (10 mg) were reduced and alkylated by 4.5 mM dithiothreitol and 10 mM iodoacetamide, respectively, and then digested by 0.1 mg/mL TPCK-treated trypsin (Worthington; Lakewood, NJ) overnight at room temperature with gentle mixing. The reaction was stopped by adding trifluoroacetic acid (TFA) to 1% final concentration. Tryptic peptides were desalted and recovered by Sep-Pak C18 cartridge (Waters; Milford, MA), dried by a lyophilizer and resuspended in 1.4 mL IAP buffer (Cell Signaling Technology; Denvers, MA) for further enrichment procedure. Ubiquitin-related peptide enrichment was performed by using ubiquitin remnant motif (K-ε-GG) antibody-conjugated beads (Cell Signaling Technology, Danvers, MA) following the manufacturer's instruction. Briefly, tryptic peptides in IAP buffer (1.4 mL) were loaded onto the beads, gentle mixed, and then incubated at 4°C for 2 h. After washing the beads using 1 mL chilled HPLC-grade water three times, ubiquitin-related peptides were eluted from the bead by 0.15% TFA. The eluate was lyophilized and kept at -80°C until used. Nanoliquid chromatography coupled to electrospray tandem mass spectrometry (nanoLC-ESI-MS/MS) analyses were performed on a TripleTof 5600+ mass spectrometer (Sciex; Concord, Ontario, Canada) coupled with a nanoLC-ultra nanoflow system (Eksigent; Dublin, CA) in data dependent acquisition (DDA) or data independent acquisition (DIA) modes as described previously [86]. Briefly, samples were loaded via an Eksigent NanoLC-AS-2 autosampler onto a column trap (Eksigent Chrom XP C18-CL-3 µm 120 Å, 350 µm x 0.5 mm; Sciex) at 2 µL/min in 0.1% formic acid for 15 min which then separated by Acclaim PepMap100 C18 LC column (75 µm x 15 cm, C18 particle sizes of 3 µm, 120 Å) (Dionex; Thermo Fisher, Sunnyvale, CA) at a flow rate of 300 nL/min using a variable mobile phase gradient as followed; from 95% phase A (0.1% formic acid) to 40% phase B (99.9% acetonitrile in 0.1% formic acid) for 70 minutes, from 40% phase B to 85% phase B for 5 minutes, and then keeping 85% phase B for 5 minutes. Electrospray was performed by NANOSpray III Source (Sciex, Framingham, MA) using ion source gas 1 (GS1), GS2 and curtain gas at 13, 0 and 35 vendor specified arbitrary units. Interface heater temperature and ion spray voltage were kept at 150°C and at 2.6 kV, respectively. The DDA method was set to go through 1,929 cycles for 90 minutes in positive ion mode. Each cycle performed 1 time-of-flight (TOF) mass spectrometry scan type, 250 ms accumulation time, 350-1250 m/z window with a charge state of 2+ to 4+ and information dependent acquisition of the 50 most intense candidate ions. At least 150 counts of MS signal were required for triggering MS/MS scan. Each MS/MS scan was operated in high sensitivity mode, an accumulation time of 50 ms and a mass tolerance of 100 ppm. To reduce redundant peptide sequencing, former MS/MS-analyzed candidate ions were excluded after its first occurrence for 12 s. The DDA data was recorded using Analyst-TF (v.1.7) software. The DIA method was built using the SWATH-MS acquisition method editor using a predefined mass window width of 8 m/z with overlapping of 1 m/z for 57 transmission windows. A TOF-MS scan was set to go through 1,715 cycles, where each cycle performs one TOF-MS scan type (250 ms accumulation time, 350-750 precursor mass range, and a cycle time of ~3.15 s). MS spectra were collected from 100-1250 m/z with an accumulation time of 50 ms per SWATH window width. Nominal resolving power for MS1 and SWATH-MS2 scan were set at 30,000 and 15,000, respectively. The rolling collision energy was applied with the collision energy spread of 15. The DIA data was recorded by Analyst-TF (v.1.7) software. Each sample of the enriched ubiquitin-related peptides was re-suspended in 6 µl of 0.1% formic acid. For a spectral library generation, 1 µl of each sample (from a total of 12 samples) were pooled together to produce a 12 µl mixed peptide sample which was analyzed in duplicate by nanoLC-ESI-MS/MS in DDA mode. Two DDA files were subjected to a merged search by Protein Pilot v.5.0, revision 4769 (Sciex, Framingham, MA) using Paragon algorithm against SwissProt Homo Sapiens database (v.113016, 20,200 entries) with an automated false discovery rate. The search parameters included alkylation on cysteine by iodoacetamide, tryptic digestion, TripleTOF 5600 instrument, ubiquitin/SUMO enrichment, ID focus on biological modification, thorough ID search effort, and detected protein threshold [unused ProtScore (Conf)] > 0.05 (10%). The Protein Pilot search result was manually inspected for unique peptides with false discovery rate (FDR) <1% which were considered valid. The search file was loaded onto SWATH Acquisition MicroApp v.2.0.2133 in PeakView software v.2.2 (Sciex, Framingham, MA) to generate the spectral library. For SWATH-MS analysis, 5 µL of the enriched ubiquitin-related peptide sample was subjected to nanoLC-ESI-MS/MS in DIA mode. SWATH data extraction of 12 DIA files (obtained from 12 samples; 4 distinct treatment conditions, 3 biological replicates for each condition) was performed by SWATH Acquisition MicroApp (Sciex, Framingham, MA) and the generated spectral library using an extraction window of 5 min and the following parameters: 100 peptides/protein, 6 transitions/peptide, excluding shared peptides, peptide confidence>95%, FDR<1%, and XIC width of 0.05 Da. SWATH quantitative data was exported into an Excel file for further analysis. Expression data was normalized by the total area sum approach, while missing values were replaced by zero. The batch effect associated with the cell culture batches was removed by the removeBatchEffect function of the limma (v.3.34.9) R package [87]. Pathway analysis was performed using DAVID 6.8 [74,75] and the ClueGo plugin[88] in Cytoscape[76].

### In Silico Screening of Compounds

Database manipulations were performed using Pipeline Pilot (Ver 18.1.100.11, Dassault Systemes BioVia Corp, San Diego, CA), docking/virtual screening were performed in ICM-Pro (Ver 3.8-7/Win, MolSoft, LLC), and the graphics were prepared in Pymol Molecular Graphics System (Ver 1.8.6.0, Schrodinger, LLC). Cysteine targeting small molecule databases were constructed from graphical depictions of thiol reactive functions created in Chemdraw (Ver 16.0.0.82 (68), PerkinElmer Informatics) or mol files generated in Marvinsketch (Ver 5.3.8, ChemAxon Ltd.) by conversion and standardization into 2D sdf files in Pipeline Pilot (Ver 18.1.100.11, Dassault Systemes BioVia Corp, San Diego, CA). The CCHMC Compound library was then scanned for these functions by substructure search, also in Pipeline Pilot.

UBE2N crystal structure files were retrieved from the Protein Data Bank (www <dot> rcsb <dot> org; H.M. Berman, J. Westbrook, Z. Feng, G. Gilliland, T.N. Bhat, H. Weissig, I.N. Shindyalov, P.E. Bourne. (2000) The Protein Data Bank Nucleic Acids Research, 28: 235-242.) UBE2N structures were visualized in Pymol (Ver 1.8.6.0, Schrodinger LLC, New York, NY). Docking and Virtual Screening was performed in the ICM-Pro software suite (Ver 3.8-0, Molsoft LLC, San Diego, CA). UBE2N structures were read into ICM-Pro and stripped of crystallographic artifacts and waters. SD Files were read into ICM-Pro, converted into 3D structures (50 low energy Conformers), H-atoms and Gasteiger charges added, and then docked at Thoroughness of 10, keeping the top 3 scored conformations. For databases over 1000 compounds, a thoroughness of 3 was used, and promising compounds rerun at thoroughness of 10.

### EXAMPLE 2

### Dysregulation of UBE2N-dependent innate immune pathways is associated with AML

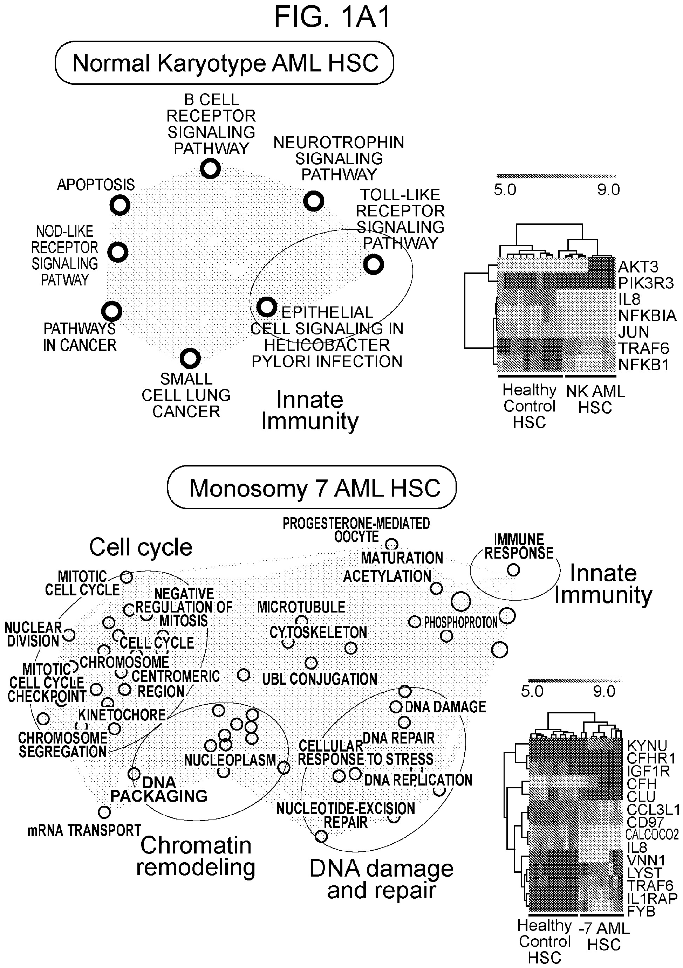

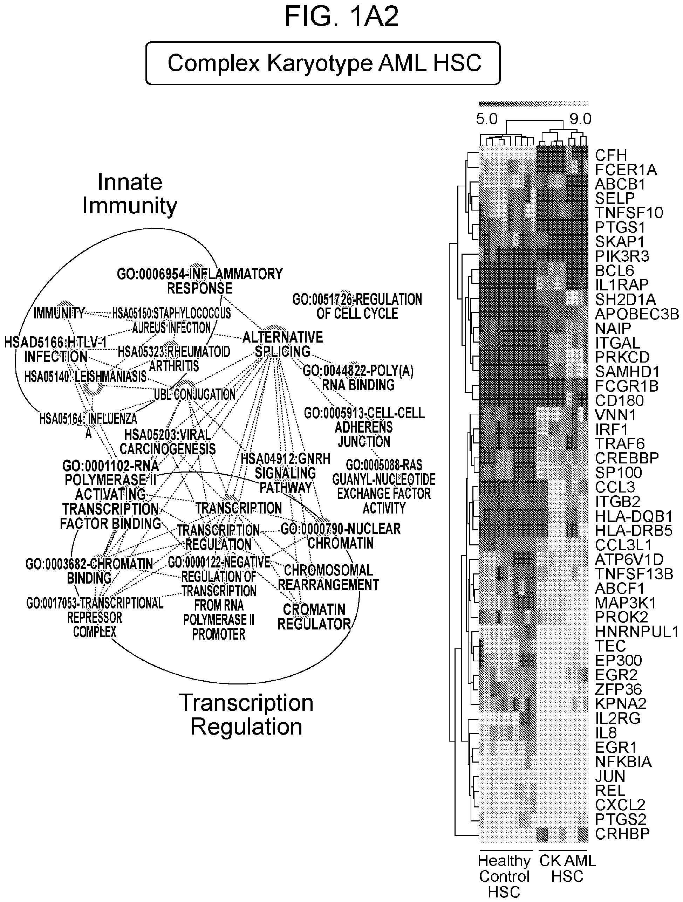

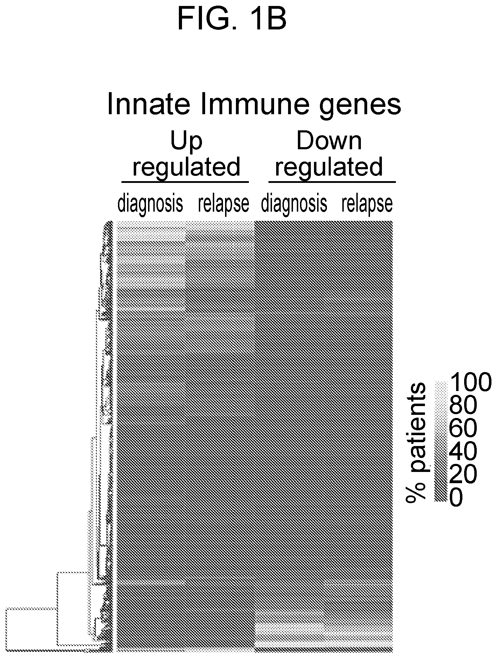

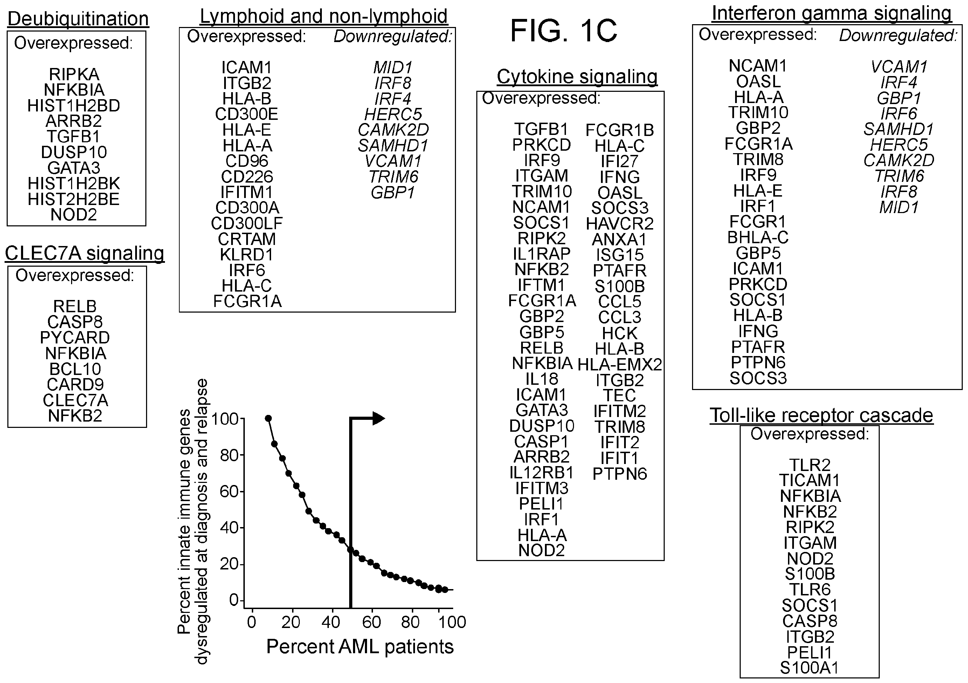

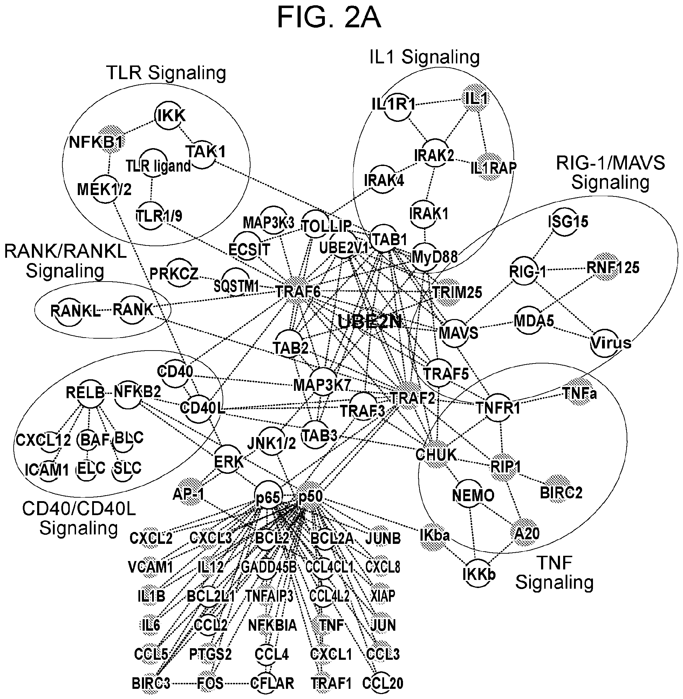

Examining gene expression profiles of bone marrow (BM) hematopoietic stem cells (HSC; Lin-CD34+CD38-) isolated from patients diagnosed with distinct subtypes of AML using publicly available data sets [14], it was observed that dysregulation of innate immune signaling genes is much more extensive than previously appreciated. Gene signatures associated with innate immune responses are significantly enriched in phenotypically defined AML HSC compared to healthy HSC (Figure 1A). Specifically, dysregulation of innate immune genes in AML HSC were associated with TNF receptor (TNFR), Interleukin 1 receptor (IL1R), B cell receptor (BCR), retinoic acid-inducible gene I (RIG-I)/mitochondrial antiviral-signaling protein (MAVS), Toll-like receptor family (TLR), CD40, and receptor activator of nuclear factor κB (RANK) pathways (Figure 2A). To determine whether dysregulation of the innate immune pathways observed at diagnosis remain durable after relapse from therapy, RNA-sequencing was performed on an independent cohort of patients with AML at diagnosis and relapse (n = 59). Fifty-six percent of the patients presented significant dysregulation of genes (enrichment score >1.5, q-value <0.01) in the blast population implicated in innate immune signaling in comparison with healthy controls (Supplemental Tables 1-3). Importantly, >30% of the innate immune genes were consistently dysregulated at diagnosis and relapse in these patients (Figure 1B, 1C). Gene enrichment analysis of innate immune related GO terms in BM cells (PAML) and PB cells (SGUA) of AML patients at time of diagnosis was determined (data not shown), along with expression of innate immune genes in BM cells (PAML) and PB cells (SGUA) of AML patients at time of diagnosis (data not shown), and expression of innate immune genes in BM cells (PAML) and PB cells (SGUA) of AML patients at time of relapse (data not shown), suggesting that the dysregulated innate immune genes are durable and inherent to the leukemic cell state. Collectively, these findings indicate that AML HSPC utilize signaling inputs via several innate immune sensors and that these dysregulated immune-related signaling networks (also referred to herein as, "oncogenic immune signaling state") can also be important in the development and maintenance of AML.

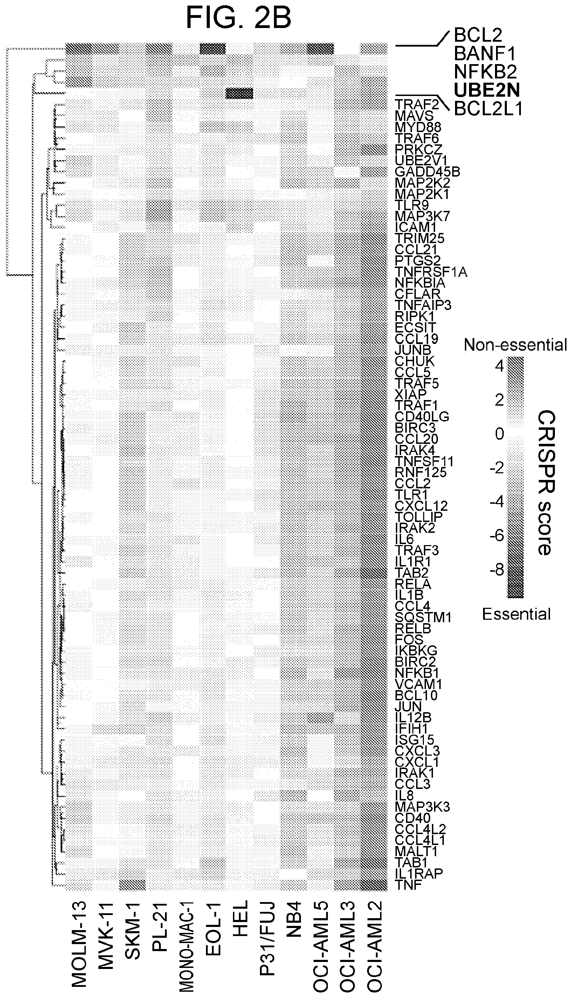

Given that dysregulation of innate immune pathways affects multiple downstream effectors in AML, the inventors sought to identify convergent immune-related signaling pathways and/or nodes that are required for leukemic cell function and amenable to therapeutic targeting. This study examined the requirement of innate immune signaling genes and associated complexes implicated in AML HSC (from Figure 2A) in a previously published genome-wide CRISPR-based screen to identify genes essential for the viability of a panel of human AML cell lines [15]. Whereas every immune-related gene examined was essential in at least two AML cell lines (dependency score < -0.5), UBE2N scored amongst the highest as it was essential in the majority of the AML cell lines (>7 of 12 AML cell lines) (Figure 2B). UBE2N was selected for further validation as it was ranked high on the AML essential gene list, is an established integrated signaling node required by several innate immune pathways dysregulated in AML and is a ubiquitin-conjugating enzyme (E2) that is amenable to therapeutic targeting.

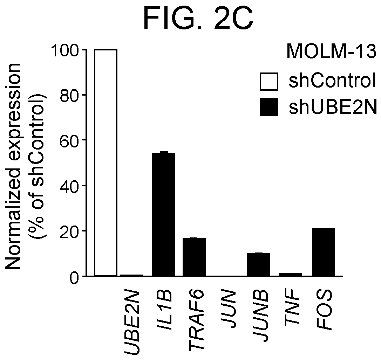

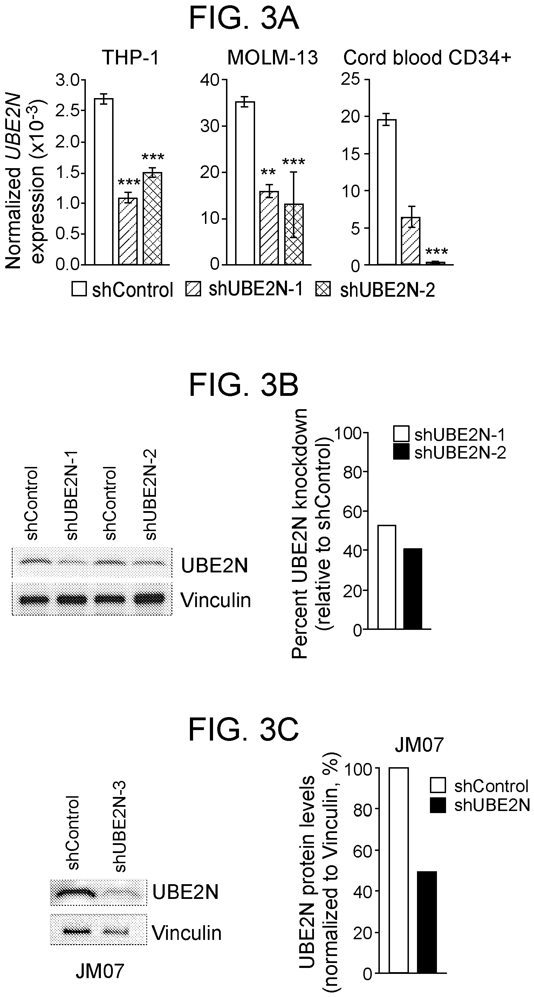

To determine the requirement of UBE2N for baseline oncogenic innate immune signaling in leukemic cells, UBE2N expression was knocked down in MOLM-13, an AML cell line that exhibited significant dependency on UBE2N (Figure 2B), by expressing lentiviral vectors encoding shRNAs targeting UBE2N (shUBE2N) (Figure 3A). As one indication that UBE2N is required for oncogenic innate immune pathways in leukemic HSC, knockdown of UBE2N in MOLM-13 cells corresponded with reduced expression of key downstream targets involved in TLR, IL1, TNF, RIG-I/MAVS, RANK, and CD40 signaling (Figure 2C).

Figure 1 depicts dysregulation of innate immune signaling in myeloid malignancies. Fig. 1A. Gene enrichment map of transcriptional profile of hematopoietic stem cells (HSC, or HSPC) isolated from acute myeloid leukemia (AML) patients with monosomy 7 (shown in Fig. 1A1), normal karyotype (shown in Fig. 1A1), or complex karyotype (shown in Fig. 1A2). Each node (empty circle) size corresponds to the number of differentially expressed genes in AML HSC versus healthy control HSC. The size of the node corresponds to the significance of the geneset enrichment. The edge size corresponds to the number of genes that overlap between the two connected genesets. The ellipses indicate nodes of the innate immunity process or other enriched sets, as shown. Genes within innate immune response categories are listed in the heatmap, wherein downregulation is shown darker and gene expression upregulation is shown lighter. Figure 1B. Heatmap showing the percentage of AML patients with up or down regulated innate immune genes at AML diagnosis and relapse (n = 56 patients). Figure 1C. Scatter plot showing the percentage of innate immune genes consistently dysregulated in AML patients (n = 56) at diagnosis and relapse. Innate immune genes consistently dysregulated in 50% of the patients are indicated. Downregulated and upregulated genes are indicated as well.

Figure 2 demonstrates that dysregulation of UBE2N-dependent innate immune pathways is associated with AML HSPC. Fig. 2A. Network of Kyoto Encyclopedia of Genes and Genomes (KEGG) pathway analysis of the genes dysregulated in AML phenotypically defined leukemia stem cells (LSC). Significantly dysregulated genes are depicted in shaded nodes. The ellipses represent the significantly dysregulated pathways associated with innate immune and inflammatory signaling. UBE2N is depicted as a central node shared by multiple pathways. Fig. 2B. Heatmap showing the innate immune gene essentiality in AML cell lines from publicly available CRISPR/Cas9 screening [15]. Essential genes are shown as darker, and non-essential genes are depicted as lighter. Fig. 2C. Normalized mRNA levels of the indicated innate immune and inflammatory genes (from Fig. 2A) upon knockdown with UBE2N shRNA-2 expressed as percentage of the non-silencing control as determined by qRT-PCR. Error bars represent the standard deviation from two independent experiments in technical triplicates.

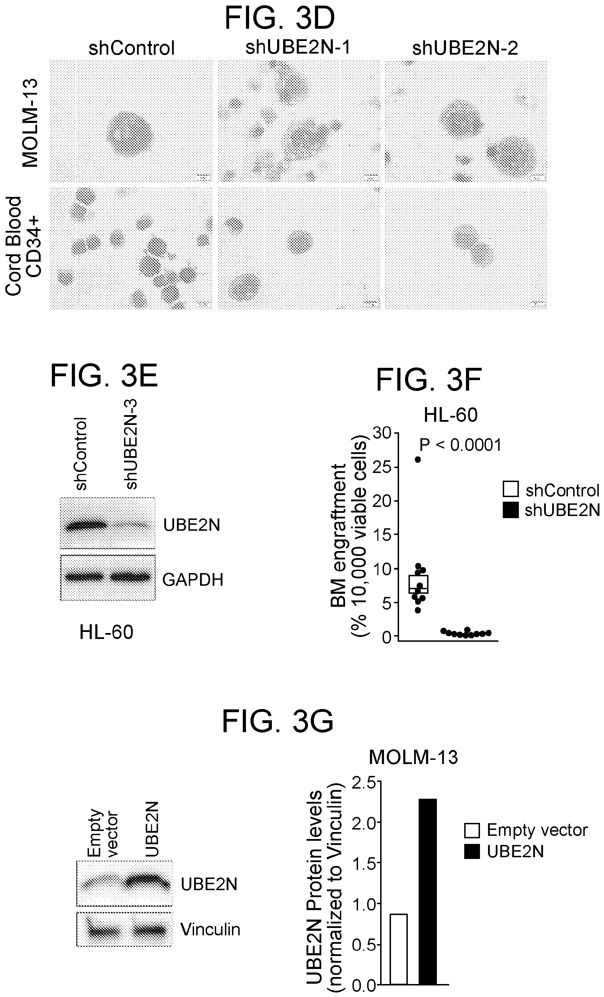

Figure 3 describes knockdown of UBE2N in leukemic and normal hematopoietic cells. Fig. 3A. Normalized mRNA levels of UBE2N upon knockdown with UBE2N shRNAs or non-silencing control as determined by qRT-PCR in the indicated cells. Error bars represent the standard deviation from two independent experiments in technical triplicates. Fig. 3B. Immunoblot showing the protein levels of UBE2N in MOLM-13 cells transduced with lentivirally expressed UBE2N shRNAs (shUBE2N-1 and shUBE2N-2) or non-silencing control shRNA (shControl). Vinculin was used as a loading control. The right panel shows the quantification of the immunoblot expressed as percentage of knockdown compared to non-silencing control. Fig. 3C. Knockdown levels of UBE2N in PDX AML cells (JM07) transduced with lentivirally expressed UBE2N shRNA (shUBE2N-3) or non-silencing control shRNA (shControl). Vinculin was used as a loading control. The right panel shows the quantification of the immunoblot normalized to Vinculin. Fig. 3D. MOLM-13 or CD34* cord blood cells were transduced with lentivirally expressed shRNAs against UBE2N (shUBE2N-1 and shUBE2N-2) or control shRNA (shControl). After 2 days, GFP7PI- cells were FACS sorted and cytospin preparations were stained with Wright-Giemsa stain. Colony formation assay of PDX AML (JM40, JM07) transduced with lentivirally expressed shRNA against UBE2N (shUBE2N-3) or non-silencing control (shControl). Error bars represent the standard deviation of technical duplicates or triplicates. Fig. 3E. Xenotransplantation of HL-60 cells in NSG mice. Immunoblot showing the protein levels of UBE2N in HL-60 cells transduced with lentivirally expressed UBE2N shRNA (shUBE2N-3) or non-silencing control shRNA (shControl). GAPDH was used as a loading control. 1 x 10⁵ of these transduced cells were transplanted per NSG mouse without preconditioning. Fig. 3F. Bone marrow (BM) engraftment of HL-60 cells expressed as percentage of 10,000 viable cells expressing human CD15. Box plots display the range of variation (first and third quartile) and the median; individual data points (one per mouse) are displayed as filled circles. Fig. 3G. Immunoblot showing the protein levels of UBE2N in MOLM-13 cells transduced with lentivirally expressed UBE2N cDNA or an empty vector and non-silencing control shRNA (shControl). Vinculin was used as a loading control. The right panel shows the quantification of the immunoblot normalized to Vinculin.

### EXAMPLE 3

### UBE2N expression is required for survival and function of leukemic cells

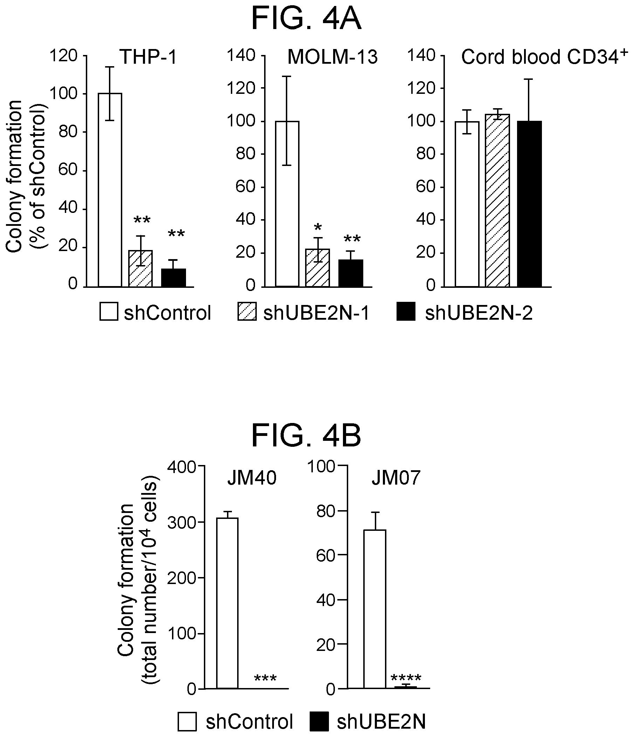

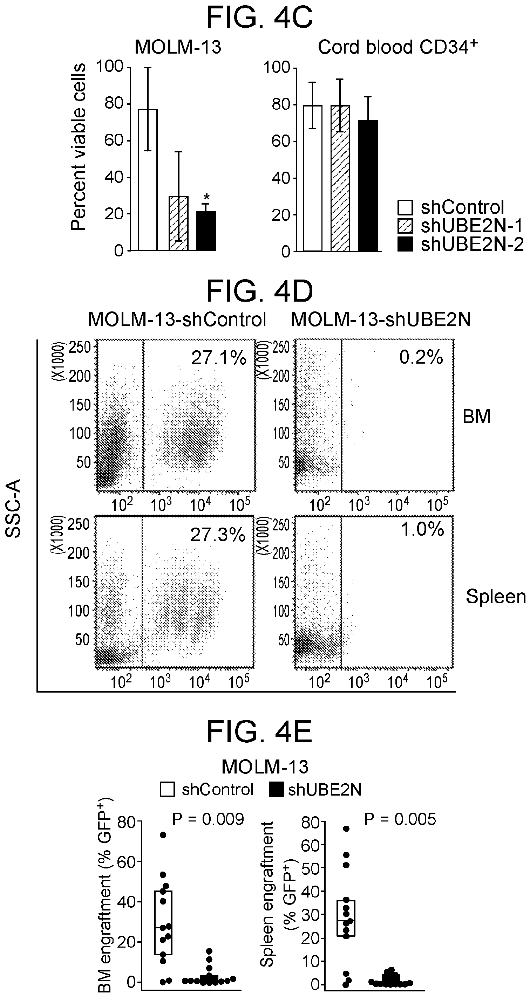

To determine the requirement of UBE2N for function of leukemic cells, UBE2N expression was knocked down in two AML cell lines, THP-1 and MOLM-13, by expressing lentiviral vectors encoding independent shRNAs targeting UBE2N (shUBE2N-1 and shUBE2N-2) (Figures 3A and 3B). Coinciding with loss of oncogenic innate immune signaling, expression of shUBE2N resulted in reduced clonogenic potential of THP-1 and MOLM-13 cell lines by >80% as compared to the non-targeting control shRNA (shControl) (Figure 4A). Moreover, expression of shUBE2N in two patient-derived AML samples (JM40 and JM07) resulted in a significant reduction of leukemic progenitor function (Figure 4B, Figure 3C). To establish the cellular basis of impaired leukemic cell function following knockdown of UBE2N, viability of MOLM-13 cells expressing shUBE2N or shControl was examined. Compared with the shControl-expressing MOLM-13 cells, knockdown of UBE2N in these cells resulted in significantly impaired viability (Figure 4C). Cytologic analysis of MOLM-13 shControl and MOLM-13 shUBE2N cells revealed morphologic changes consistent with monocytic differentiation upon UBE2N knockdown (Figure 3D). While downregulation of UBE2N suppressed the leukemic cell function of MOLM-13 cells and primary AML samples, expression of shUBE2N did not significantly affect the function, viability, nor morphology of healthy cord blood CD34+ cells (Figure 4A and 4C; Figure 3A and 3D).

To investigate the consequences of UBE2N knockdown on leukemic-propagating cells *in vivo,* MOLM-13 or HL-60 cells expressing a non-silencing shRNA control (shControl) or shRNA targeting UBE2N (shUBE2N) co-expressing GFP were FACS sorted for GFP and propidium iodide expression (GFP⁺/PI⁻) and equal numbers of viable cells were xenografted into sublethally irradiated NOD-scid IL2Rγ^{-/-} (NSG) mice. Four weeks post transplantation, organ analysis and histopathology revealed robust leukemic infiltration in BM and spleen, including splenomegaly in 11 of 13 mice transplanted with MOLM-13-shControl cells (Figure 4D-H). In contrast, only 3 out of 13 mice transplanted with MOLM-13-shUBE2N cells had detectable leukemic cells in the BM or spleen (Figure 4D-H). In agreement with the findings observed in mice xenografted with MOLM-13 cells, knockdown of UBE2N in HL-60 cells resulted in significantly diminished leukemic burden in NSG mice (Figure 3E and 3F).

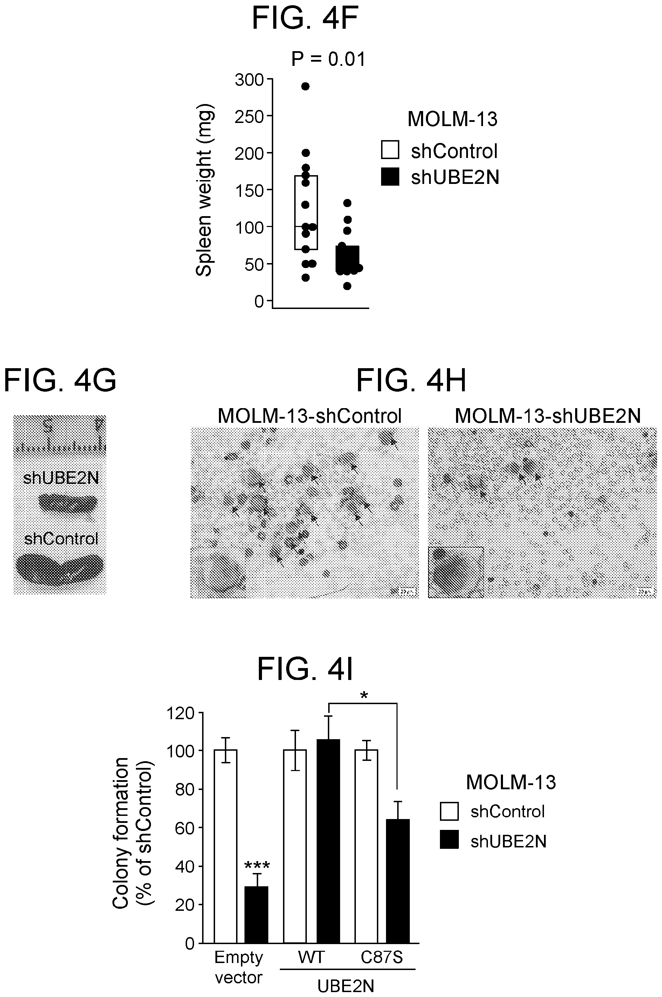

The active site of UBE2N contains a cysteine (Cys) at position 87 (Cys-87), which is essential for binding and transfer of ubiquitin to its substrates [16, 17]. To confirm that the catalytic ubiquitin-conjugating function of UBE2N is required for leukemic cell function, a mutant of UBE2N was generated in which Cys-87 was substituted with serine (C87S). UBE2N(C87S) can still synthesize polyubiquitin chains, albeit with reduced catalytic efficiency [18]. The clonogenic defect of UBE2N deficient MOLM-13 cells is completely rescued by overexpression of UBE2N (Figure 4I, Figure 3G), excluding the possibility of off-targets effects of shUBE2N. However, overexpression of UBE2N(C87S) in UBE2N-deficient cells was unable to fully rescue the leukemic colony formation, indicating that maximal UBE2N ubiquitin-conjugating function is required in leukemic cells (Figure 4I). Collectively, these data reveal that UBE2N and its ubiquitin conjugating function are required to selectively maintain the viability and function of leukemic cells and suggests that disruption of UBE2N activity represents a leukemia-targeting strategy.

Figure 4 demonstrates that UBE2N expression is required for leukemic cell function. Fig. 4A. Clonogenic potential of MOLM-13, THP-1 cells and cord blood CD34+ cells transduced with lentivirally expressed non-silencing control shRNA or shUBE2N. Error bars represent the standard error of the mean (SEM) of three independent experiments in technical duplicates. Fig. 4B. Colony formation assay of patient-derived (PDX) AMLs (JM40 and JM07) transduced with lentivirally expressed shRNA against UBE2N (shUBE2N-3) or non-silencing control (shControl). Error bars represent the standard deviation of technical duplicates or triplicates. Fig. 4C. Viability of MOLM-13 and cord blood CD34+ cells transduced with lentivirally expressed UBE2N shRNAs (shUBE2N-1 and shUBE2N-2) or non-silencing control shRNA (shControl) assessed by AnnexinV and propidium iodide (PI) staining (viable: Annexin V-/PI-). Error bars represent the standard deviation of three independent experiments. Fig. 4D. NSG mice (n = 13 per group) were transplanted with MOLM-13 cells expressing shUBE2N-2 or non-silencing control shRNA (shControl). Representative flow cytometric dot plots showing expression of GFP in bone marrow (BM) and spleen cells. Fig. 4E. BM and spleen engraftment expressed as percentage of GFP+ cells. Box plots display the range of variation (first and third quartile) and the median; individual data points (one per mouse) are displayed as filled circles. Fig. 4F. Spleen weights of the mice indicated in milligrams. Fig. 4G. Representative picture of murine spleens. Fig. 4H. BM aspirate smears stained with Wright-Giemsa stain (40X magnification) arrows indicate MOLM-13 cells. One representative MOLM-13 cell is magnified in the lower left corner. Fig. 4I. Clonogenic potential of MOLM-13 cells transduced with lentivirally expressed empty vector control, wild-type UBE2N or UBE2N(C87S) expressing vectors and non-silencing shRNA or shUBE2N. Error bars represent the standard error of the mean (SEM) of three independent experiments in technical duplicates. Significance was determined with a Student's T test (*, P < 0.05; **, P < 0.01; ***, P < 0.001).

### EXAMPLE 4

### Structure-based in silico and leukemia cell screen identified inhibitors of UBE2N

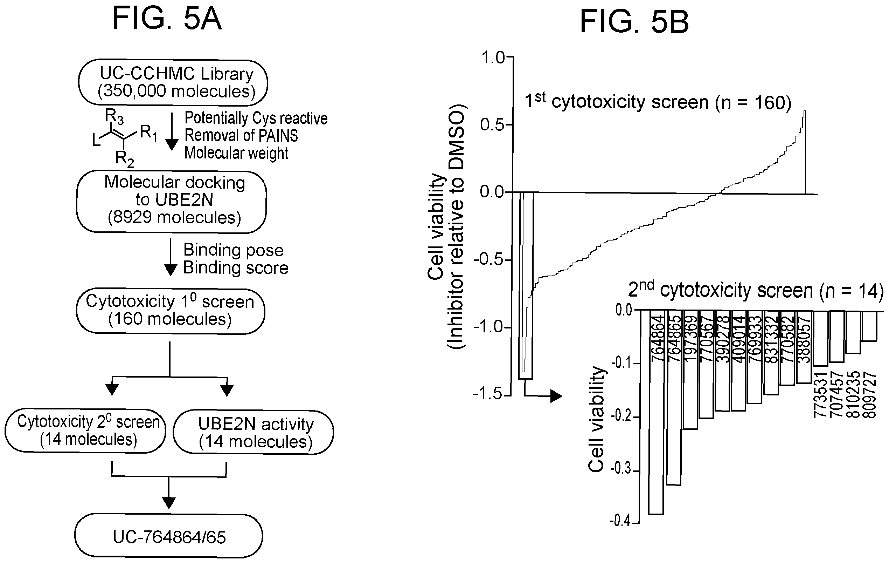

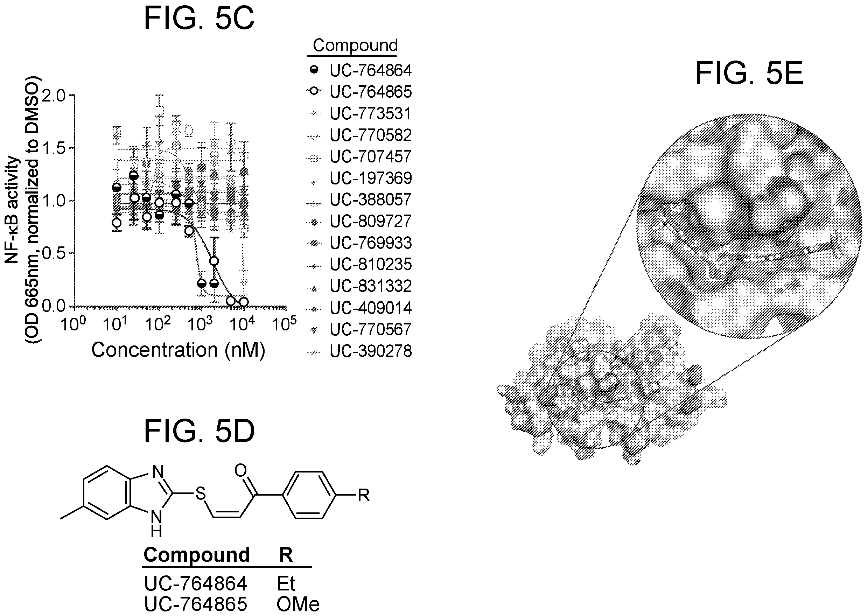

Next, the study sought to identify a selective inhibitor of UBE2N as a means to suppress AML cells but also to use as a chemical probe to interrogate oncogenic immune signaling states in AML. Ubiquitin conjugating enzyme function can be inhibited by interfering with thioester formation between ubiquitin and the active site cysteine [17, 19]. Such an approach has been demonstrated for UBE2N with NSC697923 and BAY11-7082, two structurally-related compounds containing electrophilic sulfanyl groups [16, 20-22]. The α,β-unsaturated nitro of NSC697923 and corresponding nitrile of BAY11-7082 covalently react with Cys-87 resulting in irreversible inhibition of UBE2N [16]. Although NSC697923 and BAY11-7082 are non-selective inhibitors of cysteine-containing ubiquitin enzymes [23-25] and/or exhibit undesirable toxicity[26], they provide proof-of-concept that inhibition of UBE2N catalytic function is feasible by targeting the active-site cysteine. To identify a cysteine-reactive small molecule inhibitor of UBE2N with potential clinical utility, in silico structure-based screens were performed using the α,β-unsaturated carbonyl from NSC697923 and BAY11-7082 as a chemical starting point followed by UBE2N-dependent activity and cytotoxic leukemia cell assays (Figure 5A). From an in-house library of over 350,000 compounds, a cysteine directed library of 8929 small molecules (molecular weight 180-450 g/mol) was constructed to focus on compounds predicted to dock within the active site of UBE2N and potentially covalently react with Cys-87. The cysteine-directed library was screened *in silico* against 7 crystal structures of UBE2N, which represent the dominant conformations of the active site loop. The compound selection prioritized small molecules that gave a favorable docking score based on the binding pose within the active site of UBE2N, and whose binding pose placed the reactive center proximal to Cys-87 of UBE2N. The top 160 compound derivatives were selected for cytotoxicity in MOLM-13 cells at an initial concentration of 250 µM (Figure 5A and 5B; full cytotoxicity screen of Tier 1 compound derivatives in MOLM-13 cells data set not shown). The top 14 cytotoxic derivatives were then rescreened in MOLM-13 cells at a final concentration of 2 µM (2nd cytotoxicity screen, Figure 5B). In parallel, UBE2N activity was evaluated in a κB-site containing human leukemia (THP-1) reporter cell line, which measures UBE2N-dependent activation of NF-κB (Figure 5C). Among the 14 small molecules, two chemically related compounds, UC-764864 (1-(4-ethylphenyl)-3-[(6-methyl-1H-benzimidazol-2-yl) sulfanyl]prop-2-en-1-one) and UC-764865 (1-(4-methoxyphenyl)-3-[(6-methyl-1H-benzimidazol-2-yl) sulfanyl]prop-2-en-1-one) emerged as the top candidates from both screens as having cytotoxic effects and inhibition of UBE2N-dependent signaling in leukemic cells (Figures 5B-D).

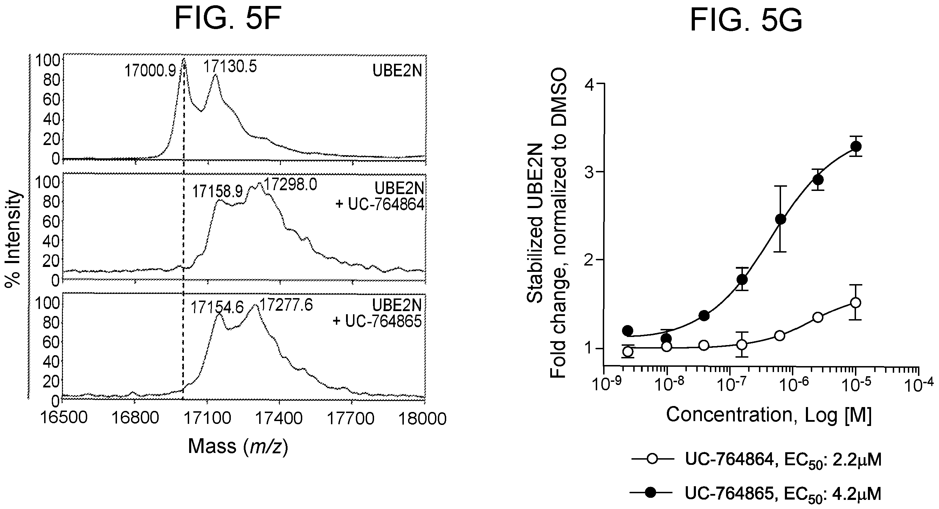

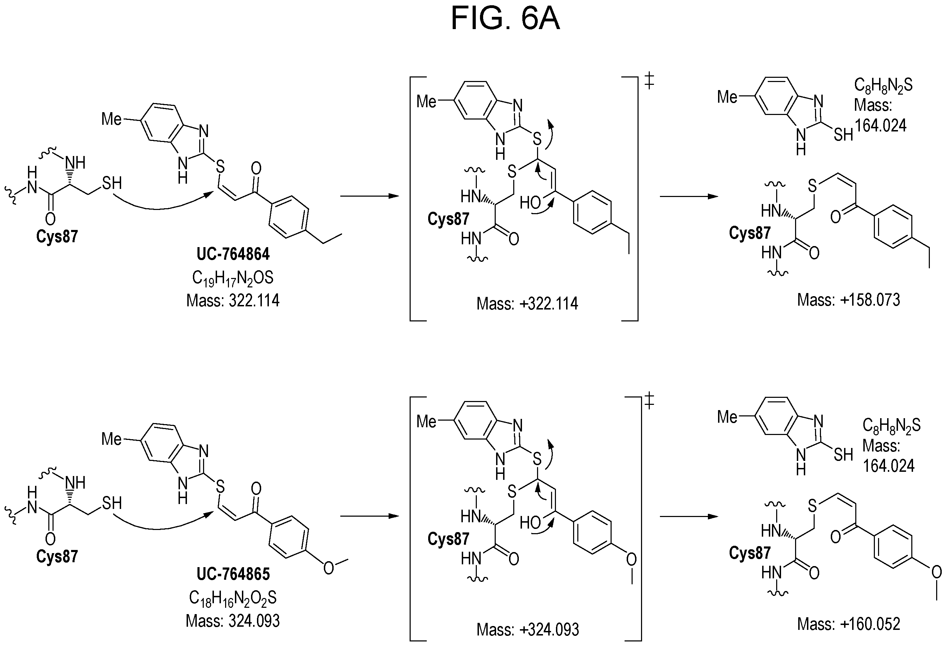

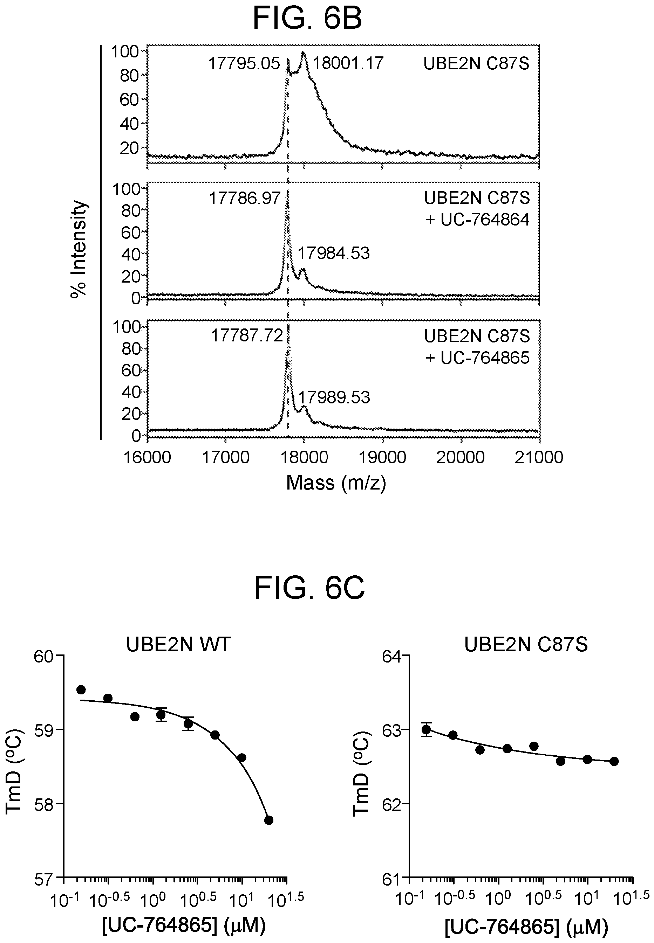

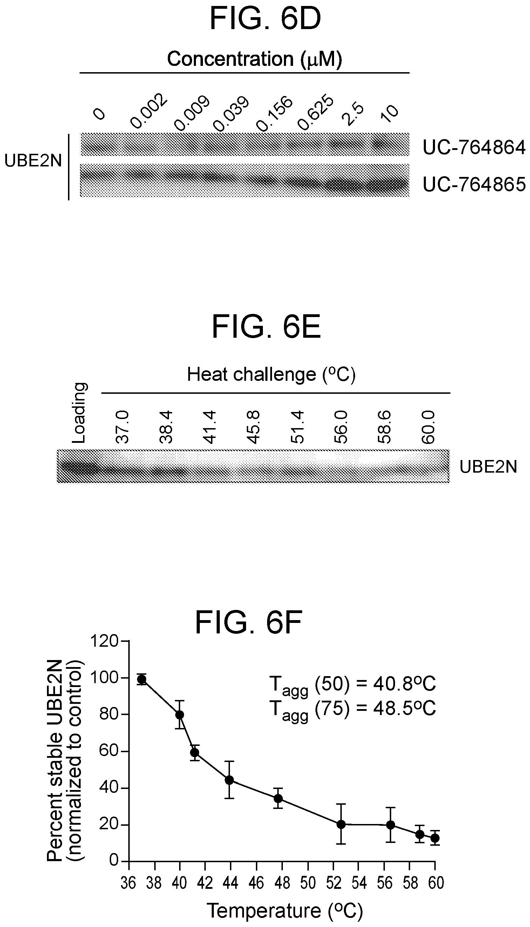

Computational modeling of UC-764864/65 within the cleft of the UBE2N active site suggests that UC-764864/65 are positioned with the reactive center proximal to Cys-87 (Figure 5E), leading to the formation of a covalent bond through a Michael addition. Cys-87 alkylation is incompatible with thioester conjugation between ubiquitin (Ub) and UBE2N and subsequent transfer of Ub to substrates (Figure 6A). Consistent with this model, matrix-assisted laser desorption ionization/time-of-flight mass spectrometry (MALDI-TOF-MS) and differential scanning fluorimetry (DSF) analyses show that UC-764864/65 bind to recombinant UBE2N protein. MALDI-TOF-MS revealed that the mass shift of wild-type UBE2N protein was -160 Da when incubated with UC-764864/65 (Figure 5F), which is the predicted molecular weight of the chemical adduct of UC-764864/65 resulting from a Michael addition (Figure 6A). Since serine is less reactive than cysteine toward Michael additions of this type, it is anticipated that UC-764864/65 would not bind to UBE2N(C87S) under similar conditions [27]. As expected, MALDI-TOF-MS analyses confirmed that UC-764864/65 does not bind to recombinant UBE2N(C87S) as indicated by the absence of a mass shift (Figure 6B). In addition, DSF of UC-764865 caused a decrease in melting temperature (Tm) of -2C degrees with recombinant UBE2N protein while it did not change the Tm of UBE2N(C87S), indicating that the compound interacted with wild-type UBE2N but not UBE2N(C87S) (Figure 6C). Lastly, to evaluate the interaction of UC-764864/65 with UBE2N *in situ,* a cellular thermal shift assay (CETSA) was performed in MOLM-13 cells. A dose-dependent stabilization of UBE2N was observed with UC-764864 (EC50 = 2.2 µM) and UC-764865 (EC50 = 0.42 µM) (Figure 5G, Figure 6D-F). Collectively, these results are consistent with direct binding of UC-764864/65 to the catalytic Cys-87 and inhibition of UBE2N ubiquitin conjugating function.

Figure 5 depicts identification of UBE2N inhibitors. Fig. 5A. Overview of *in silico* screening of small molecule inhibitors of UBE2N. Structure shows α,β-unsaturated carbonyl as chemical starting point for the screen. Fig. 5B. *In vitro* cell proliferation of MOLM-13 cells treated in triplicate with each of the 160 small molecules prioritized from the *in-silico* screen using MTS assay (24 hr). The final concentration compounds for the primary screen was 250 pM and 2 pM for the secondary screen. Fig. 5C. UBE2N-dependent activation of NF-κB was determined in THP1-NF-κB reporter cells following treatment with the top 14 small molecule inhibitors. Fig. 5D. Structure of UC-764864 and UC-764865. Fig. 5E. Molecular docking of UC-764864 and UC-764865 in the active site of human UBE2N (crystal structure 1J7D). The surface representation of UBE2N was enlarged to show the interaction of UC-764864/65 with Cys-87 in the active site. Fig. 5F. Linear mass spectrometry spectra of wild-type UBE2N without (upper panel) or with UC-764864 (lower panels) showing a mass shift of UBE2N with the addition of UC764864. Note that there are two peaks for the UBE2N protein (upper panel). The higher MW peak is consistent with the [M+H]⁺ ion of the intact protein, and the lower molecular weight peak is consistent with the loss of the N-terminal a methionine. The broader m/z shift of UBE2N is consistent with alkylation. Fig. 5G. CETSA curves for MOLM-13 cells treated with UC-764864/65. The plate was heated to 48.5 °C in a thermal cycler. Error bars represent the standard deviation of 3 replicates.

Figure 6 depicts interaction of UC-764864/65 with UBE2N. Fig. 6A. Predicted expectations for the mass shift after binding of UC-764864/65 to Cysteine (Cys) 87 in the active site of UBE2N. Cys-87 of UBE2N is predicted to react with UC-764864/65 by a Michael addition. Fig. 6B. Linear mass spectrometry spectra of mutant UBE2N C87S without (upper panel) or with UC-764864/65 (lower panels) showing absence of mass shift of UBE2N C87S with the addition of UC764864/65. Fig. 6C. Interaction of UC-764865 with UBE2N wild type and C87S mutant measured by differential scanning fluorimetry (DSF). An experiment testing a wide range of UC-764865 concentrations suggests that UC-764865 interacts with UBE2N wild type (left plot) but not with the C87S mutant (right plot). TmD: melting temperature derivative. Fig. 6D. Dose cellular thermal shift assay (CETSA) blots for UC-764864/65. Fig. 6E and Fig. 6F depict melting profile for UBE2N. SF3E. Representative immunoblot showing thermostable UBE2N following indicated heat shocks. SF3F. Densitometric analysis of immunoblot in SF3E enables quantification of Ts, for endogenous UBE2N.

### EXAMPLE 5

### UBE2N inhibitor (UC- 764864/65) abrogates UBE2N catalytic activity and UBE2N-mediated ubiquitin signaling

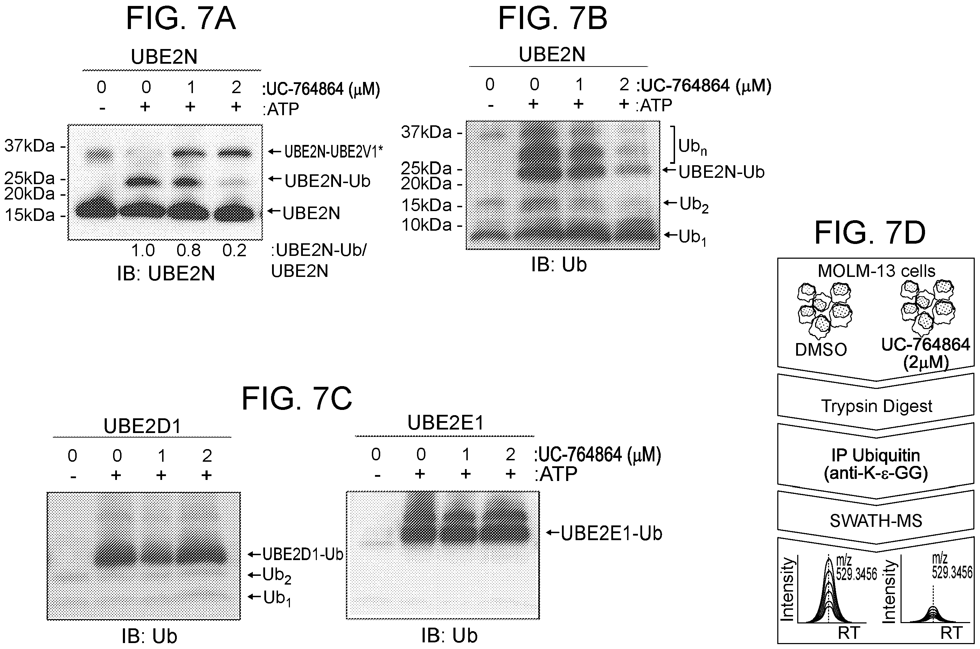

The active site cysteine residue (C87) within the E2 catalytic domain of UBE2N is required for the formation of a thioester bond with Ub, a step necessary for the ATP-dependent synthesis of polyubiquitin chains. To test the ability of UC-764864 to directly inhibit UBE2N-mediated Ub conjugation, a cell-free *in vitro* biochemical assay was utilized that measures ATP-dependent Ub-activating enzyme E1-mediated transfer of ubiquitin to UBE2N, resulting in a thioester bond between Ub and the active site cysteine of UBE2N (UBE2N-Ub). As expected, UC-764864 and UC-764865 exhibit comparable inhibitory properties against UBE2N. A UBE2N-Ub thioester conjugate was readily detectable in the reaction consisting of E1, UBE2N, UBE2V1 (cofactor), Ub, and ATP (Figure 7A, lanes 1-2). However, in the presence of UC-764864 the UBE2N-Ub thioester conjugate formation was inhibited by 20% at 1 µM and 80% at 2 µM (Figure 7A, lanes 3-4). Inhibition of UBE2N-Ub thioester conjugate formation by UC-764864 coincided with reduced Ub chain elongation, an indication that UBE2N function is diminished (Figure 7B). Using the same biochemical assays, it was found that UC-764864 did not interfere with the activity of closely related E2 Ub-conjugating enzymes, UBE2D1 and UBE2E1 (Figure 7C), suggesting that UC-764864 is selective against UBE2N.

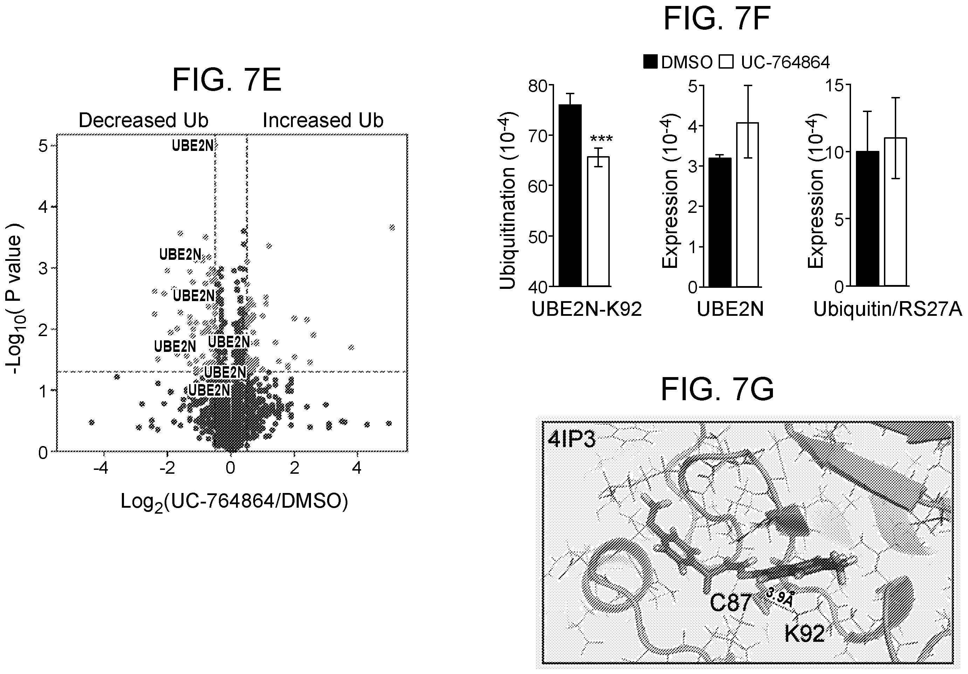

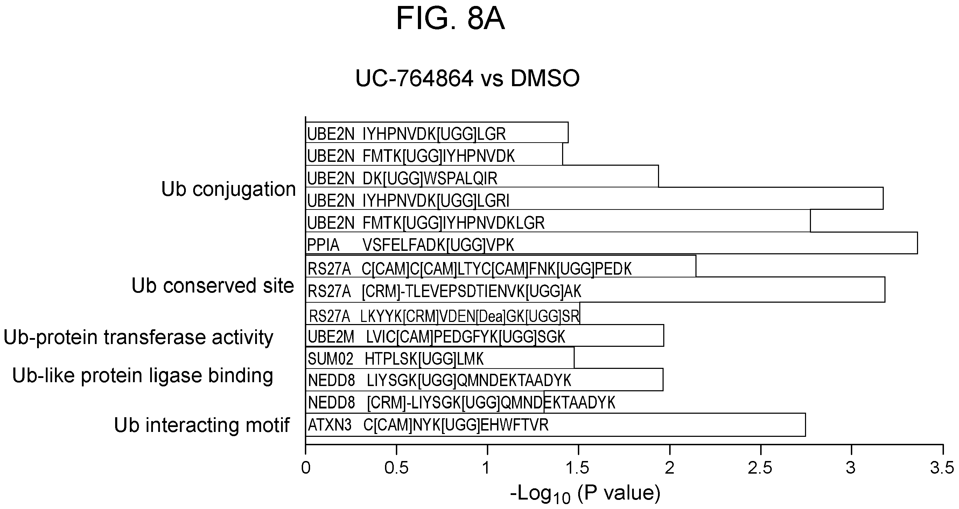

To evaluate the protein targets and signaling pathways affected by UC-764864, global quantitative ubiquitin capture proteomics was performed in MOLM-13 cells treated with UC-764864 and then the ubiquitination status of all enzymes related to Ub conjugation and ligation was examined. Ubiquitinated peptides immunoprecipitated from MOLM-13 cells treated with 2 µM of UC-764864 for 24 hs or vehicle (DMSO) were analyzed by mass spectrometry (Figure 7D). The proteomic analysis identified 121 peptides corresponding to 73 proteins that were differentially ubiquitinated following treatment with UC-764864 as compared to DMSO (Fold change >|0.5|; P value = <0.05) (Figure 7E; differential ubiquitination enrichment screen data not shown). In parallel with the results of the thioester formation assay, among the top differentially ubiquitinated proteins, UC-764864 treatment resulted in significantly reduced ubiquitination of UBE2N at Lys-92 (K92, P < 0.01), without affecting the total levels of ubiquitin (RS27A) or unmodified UBE2N (Figure 7F). Lys-92 is adjacent to Cys-87 and is a known site of autoubiquitination, thus indicating that UBE2N is a target of UC-764864 in cells (Figure 7G) [28]. Notably, UC-764864 did not significantly alter the ubiquitination levels of any other Ub-conjugating enzymes, suggesting that this class of small molecules is selective for UBE2N (Figure 8A, Supplemental Table 5).

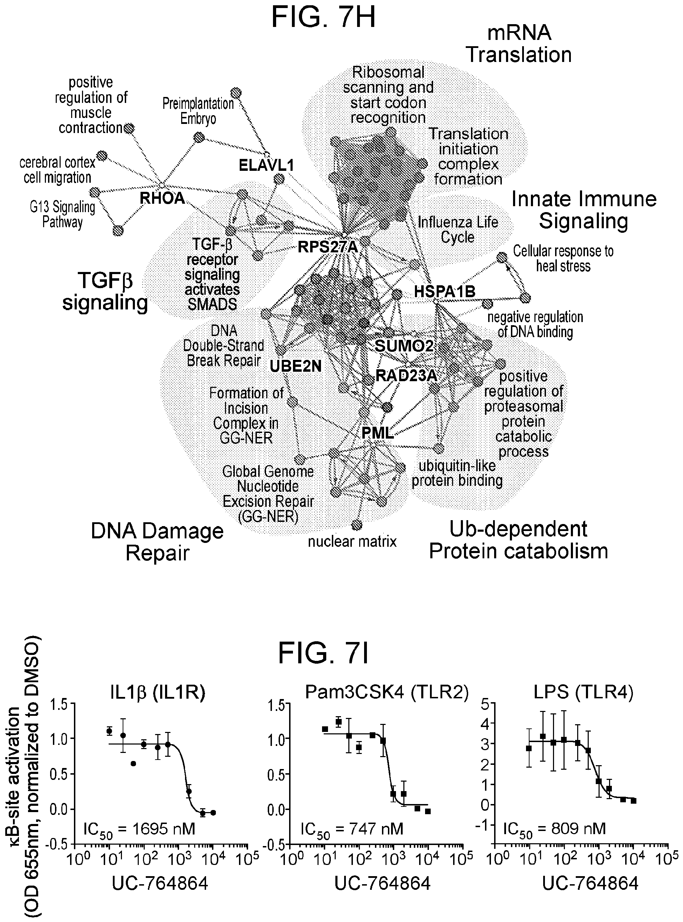

Figure 7 demonstrates that UC-764864 suppresses UBE2N enzymatic activity and innate immune signaling. Figs. 7A-7C. Thioester bond formation assay was performed with recombinant E1, UBE2N/UBE2V1 (Fig. 7A and Fig. 7B), UBE2D1 or UBE2E1 (Fig. 7C) and Ub along with ATP and the indicated concentration of UC-764864. UBE2N or biotinylated Ub enzyme conjugates were detected using UBE2N antibodies (Fig. 7A) or Streptavidin-HRP detection system (Fig. 7B and Fig. 7C), respectively as described in Example 1. Quantification of Ub-UBE2N relative to UBE2N is indicated below. *, denotes the predicted bands. Fig. 7D. Scheme of comparative analysis of ubiquitin-related proteome in MOLM-13 cells treated with UC-764864 or DMSO. Cells were treated for 24 hr and protein lysate were digested with trypsin, immunoprecipitated for ubiquitin glycine-glycine remnant with the enrichment peptides subjected to label-free quantitative mass spectrometry using the SWATH workflow all as described in the supplemental methods. A ratio of peptide intensity for each treated sample to DMSO control was calculated. Peptides with P < 0.05 and ratio ≥|0.5| were advanced for pathway enrichment analysis. Fig. 7E. Volcano plot showing the differentially ubiquitinated proteins after treatment of MOLM-13 cells with UC-764864 (2 µM) with a threshold of a 0.5-fold change and P < 0.05. Fig. 7F. Ubiquitination levels of UBE2N at lysine 92 (K92) (UBE2N-K92) and total levels of UBE2N and Ubiquitin (RS27A) in MOLM-13 cells treated with UC-764864 (2 µM), or DMSO. Error bars represent the standard deviation of biological triplicates. ***, P < 0.001. Fig. 7G. Docking of UC-764864 into the active site of UBE2N (PDBID: 1J7D). Distance between Lysine 92 (K92) and Cysteine 87 (C87) is indicated. Fig. 7H. ClueGO annotation of differentially ubiquitinated proteins (from panel B) in MOLM-13 cells upon treatment with UC-764864. Functionally grouped GO/pathway term networks were computed with ClueGO referenced by the Reactome, KEGG and GO term database. The circular size depicts the statistical significance based on percentage of genes per term. Edge thickness represents the degree of connectivity between terms. Proteins differentially ubiquitinated are shown in black. Fig. 7I. Responses of THP1-NF-xB reporter cells to UC-764864 after stimulation with indicated immune/inflammatory ligands. Dose-response profiles indicate strong responses to UC-764864 for all three innate immune ligands.

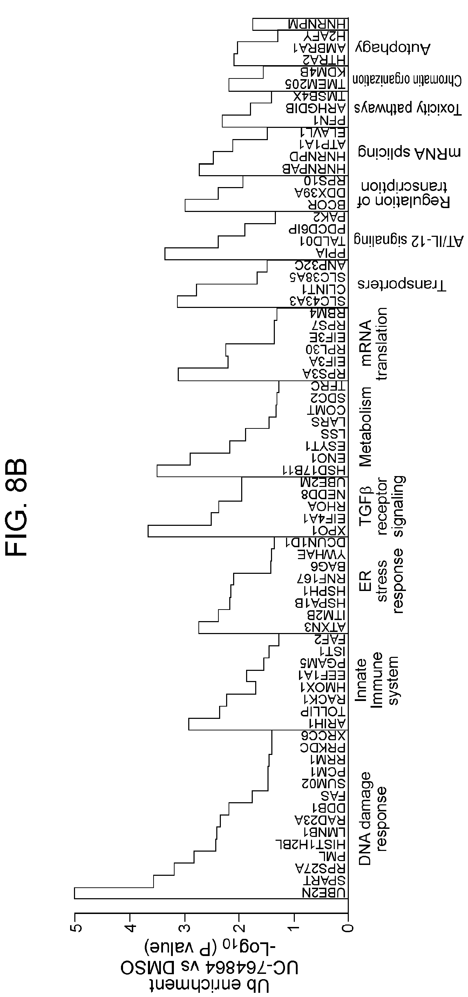

Figure 8 depicts proteomic analysis and evaluation of ubiquitin posttranslational modifications induced by UC-764864. Fig. 8A. Ubiquitin related proteins that are significantly differentially ubiquitinated in MOLM-13 cells upon treatment with UC-764864 relative to DMSO as determined by the screen described in Figure 7D. Fig. 8B. Bar graph of all differentially ubiquitinated proteins upon treatment of MOLM-13 cells with UC-764864 or DMSO. Proteins and P values are indicated. Data were normalized as detailed in Example 1. The pathways or cellular processes enriched in the differential ubiquitination screen in MOLM-13 cells are indicated.

Of the differentially ubiquitinated peptides, 66 peptides exhibited a decrease in ubiquitination after UC-764864 treatment, and 55 peptides exhibited an increase in ubiquitination after UC-764864 treatment. Gene Ontology (GO) enrichment and pathway analysis revealed ubiquitin modifications on proteins involved in cellular processes previously associated with UBE2N, such as innate immune, DNA damage response and TGFβ signaling (Figure 7H, Figure 8B). To determine whether the disruption of ubiquitin signaling caused by UC-744864 correlates with impaired oncogenic immune signaling in leukemic HSPC via UBE2N, AML cells (THP-1) expressing an NF-κB reporter gene were stimulated with innate immune-related ligands for the IL-1 receptor (IL1R), Toll like 2 receptor (TLR2), or Toll like receptor 4 (TLR4) and then treated with increasing concentrations of UC-764864. UC-764864 inhibited UBE2N-mediated NF-κB activity downstream of all the tested innate immune receptors at equal potency and in a concentration dependent manner (Figure 7I). Collectively, these findings indicate that UC-764864 is effective and selective at inhibiting UBE2N and that the ubiquitin modifications caused by UC-764864 disrupt the UBE2N-dependent ubiquitin equilibrium that enables the proper regulation of immune signaling states in AML.

### EXAMPLE 6

### Inhibition of UBE2N catalytic function suppresses AML while sparing healthy hematopoietic cells

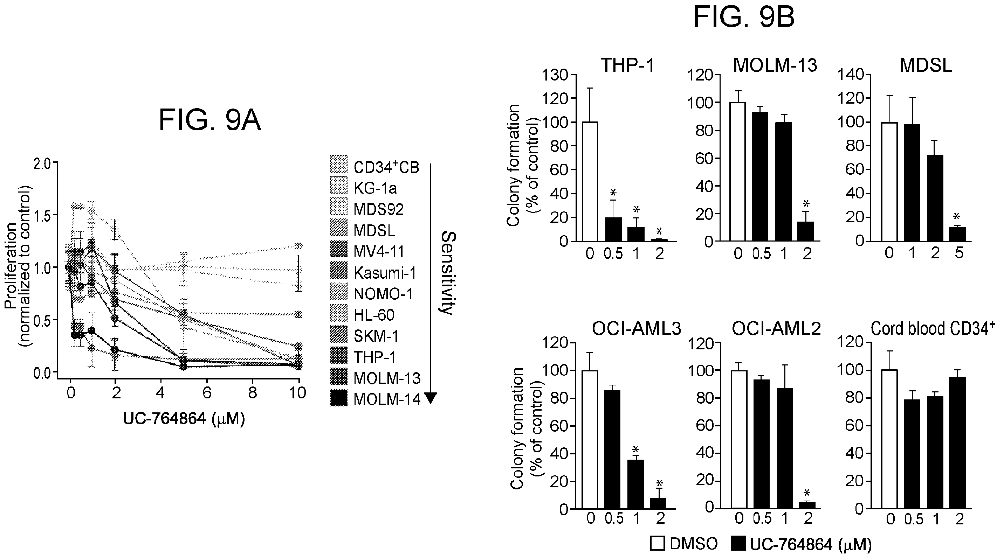

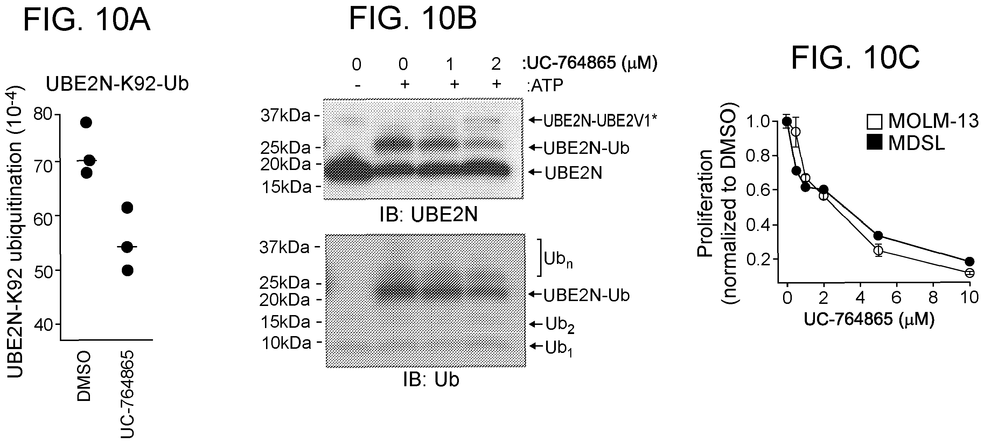

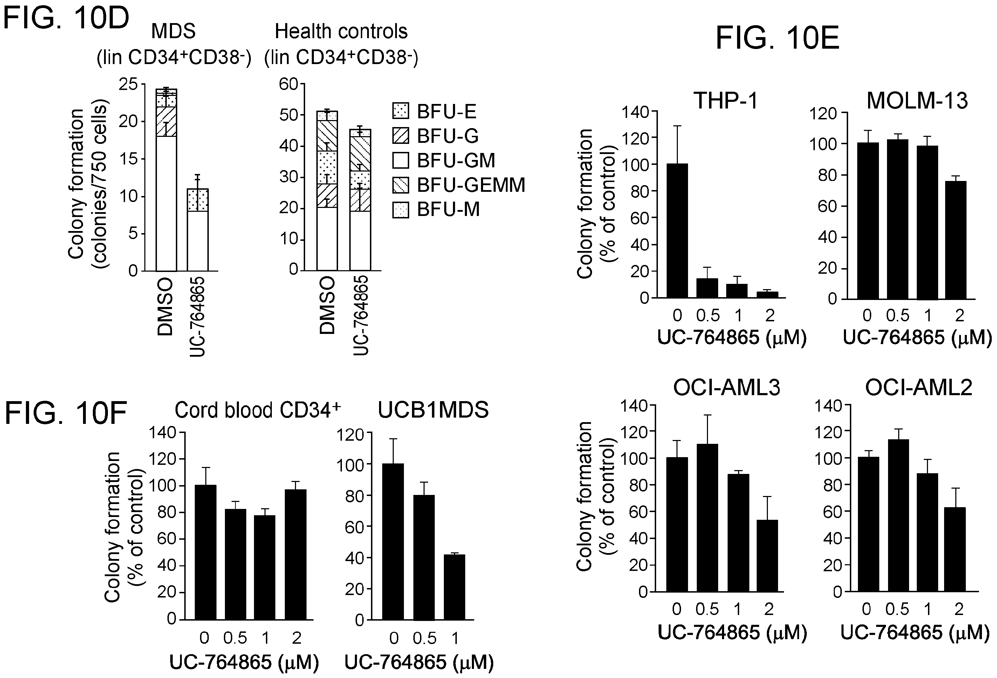

Next, the inventors tested whether inhibition of UBE2N catalytic function can suppress AML leukemic cells *in vitro* by evaluating the activity of UC-764864 across a panel of AML cell lines and healthy CD34 positive cells. Approximately 40% (4 of the 11) of the AML cell lines tested were highly sensitive to UC-764864 as determined by MTS assay, with IC50 values ranging from 0.02 to 2.5 µM (highly sensitive cell lines: MOLM-14, MOLM-13, THP-1, SKM-1) or 4.3 to 6.7 µM (cell lines with intermediate sensitivity: HL-60, NOMO-1, Kasumi-1, MV4-11, MDSL) (Figure 9A, Table 4). AML cells treated with UC-764864 showed a reduction of 20-95% in colony formation at 2 µM as compared to vehicle treated cells (Figure 9B). Consistent with dose-dependent decrease in AML cell proliferation, the colony formation assay showed differential sensitivity of the cell lines to UC-764864 treatment. In these assays, THP-1 (MLL-AF9), MOLM-13 (FLT3-ITD), OCI-AML3 (NPM1 and DNMT3A-R882C), and OCI-AML2 (DNTM3A-R635W) were most sensitive to UC-764864, while MDSL (5q-) exhibited an intermediate sensitivity to UC-764864 (Figure 9A and 9B). In contrast, UC-764864 did not affect the clonogenic potential nor the proliferation capacity of cord blood CD34+ cells (Figure 9A and 9B). Inhibition of leukemic progenitor function of AML cells (MOLM-13) coincided with an increase in apoptosis as indicated by trypan blue uptake (Figure 9C) and cleavage of Caspase 3 (Figure 9D). As expected, similar effects of UC-764865 on UBE2N function and AML cell viability were detected (Figure 10).

To determine whether the inhibitory effects of UC-764864 on AML cell function relies on UBE2N expression and function, first THP-1 cells lacking UBE2N expression (THP-1-shUBE2N) were generated. UC-764864 significantly repressed the leukemic progenitor function of THP-1-shControl cells. However, following knockdown of UBE2N, UC-764864 did not significantly further suppress leukemic colony formation of THP-1 cells (Figure 9E). Moreover, AML cells were generated expressing wild-type UBE2N or the UBE2N mutant (C87S) that retains partial enzymatic activity but is resistant to UC-764864 (Figures 4I, Figure 6B and 6C). UC-764864 significantly suppressed the viability of MOLM-13 cells expressing wild-type UBE2N but did not affect the viability of MOLM-13 cells expressing UBE2N(C87S) (Figure 9F). Overall, these results show that inhibition of the enzymatic activity of UBE2N is feasible and results in selective suppression of AML cell viability and leukemic progenitor function without significantly affecting the function of healthy hematopoietic cells.

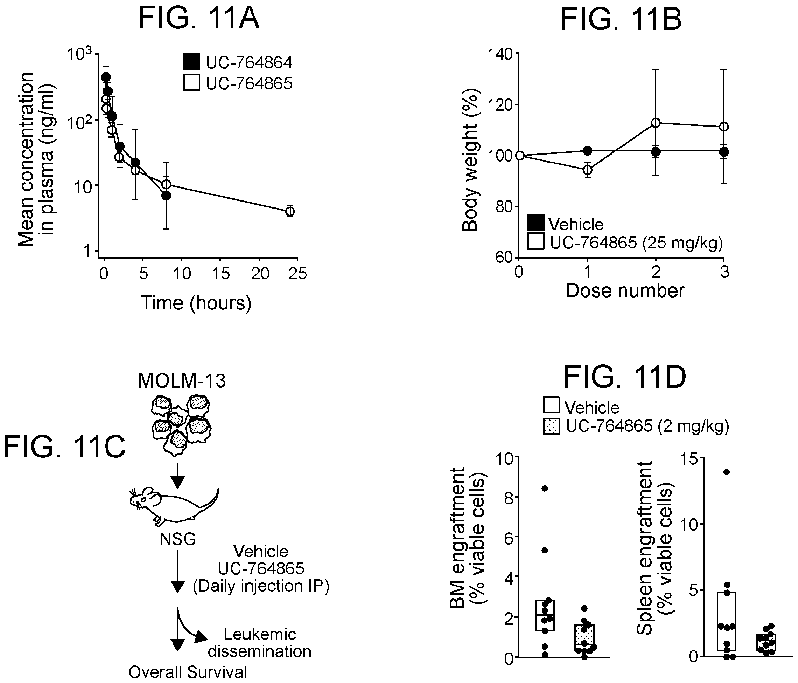

To determine the effects of inhibiting UBE2N *in vivo,* the inventors first performed an *in vitro* and *in vivo* assessment of the drug-like properties of UC-764864 and UC-764865. UC-764864 was readily metabolized in human and mouse liver microsomes and correspondingly exhibited poor pharmacokinetic (PK) properties in mice (Figure 11A, Table 5). In contrast, the structurally-related analog UC-764865 had modestly improved solubility, stability, and PK properties (Figure 11A, Tables 5 and 6). UC-764865 was detected in plasma 24 hours after a 20 mg/kg dose in C57Bl/6 mice with a maximum exposure of 346.06 ± 46.29 hr*ng/ml, making it sufficiently suitable for exploratory in vivo studies (Figure 11A, Supplemental Table 7).

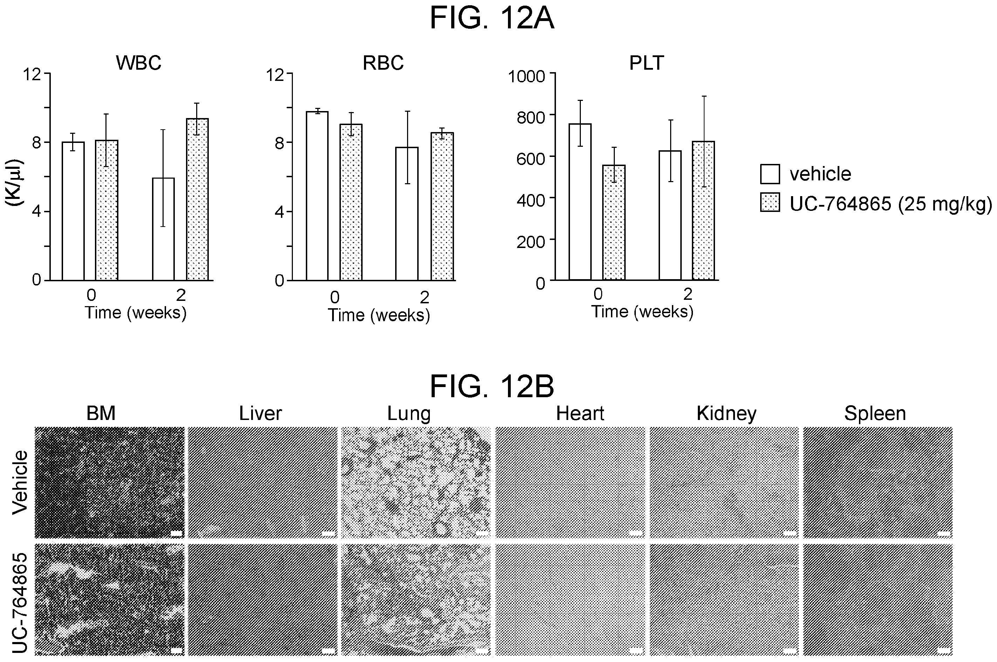

Since UBE2N has 100% protein sequence identity between mouse and human, potential toxicity was evaluated after repeated administration of UC-764865 in mice. C57Bl/6 mice received 4 doses of 25 mg/kg of UC-764865 for 2 weeks (2 doses per week). Mice tolerated UC-764865 and there were no abnormalities noted during this study. Administration of UC-764865 in mice did not significantly affect body weight, hematologic blood parameters, or survival of the mice (Figure 11B, Figure 12A). In addition, histological evaluation of several tissues showed no evidence of overall tissue toxicity (Figure 12B).

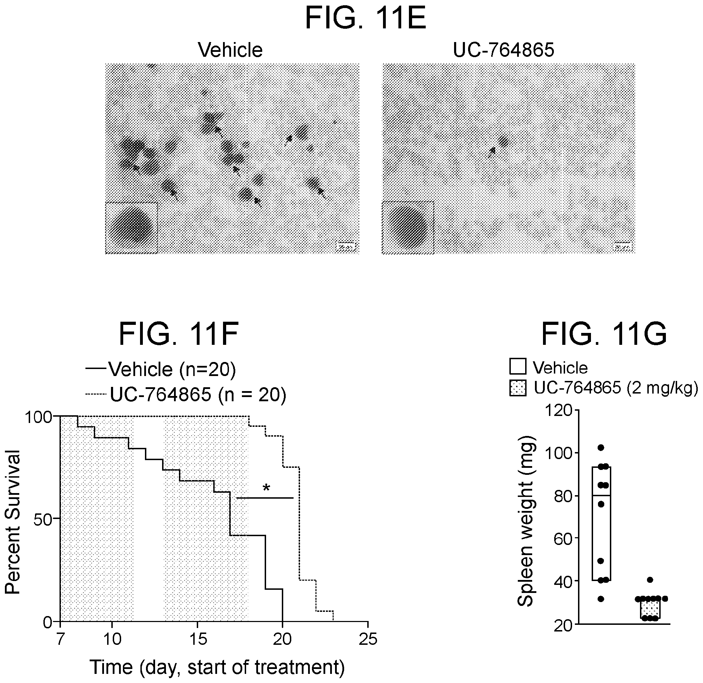

Given that UC-764865 is tolerated by mice, the study then evaluated whether inhibition of UBE2N with UC-764865 is effective at suppressing leukemic cell function *in vivo* using an aggressive xenograft cell line model of human AML. MOLM-13 cells were injected intravenous (i.v.) into NSG mice, and then following engraftment (on day 7), UC-764865 was administered daily at 2 mg/kg by intraperitoneal (i.p.) injection for 4 days per week for 2 weeks (4 doses/week) (Figure 11C). To determine the effects of UC-764865 on leukemic burden during the course of treatment, a subset of mice was sacrificed 10 days post transplantation after receiving 7 doses of UC-764865 or vehicle and measured leukemic cell dissemination into BM and spleen. The leukemic burden (percent of human CD15+ cells) was reduced 40-70% following treatment with UC-764865 in the BM (P = 0.03) and spleen (P = 0.3), respectively, as compared to vehicle-treated mice (Figure 11D and 11E). For the remaining animals, UC-764865 administration significantly delayed onset of leukemia from 8 days to 18 days and extended the overall median survival from 17 days to 21 days (P <0.0001) (Figure 11F). At time of death, splenomegaly was significantly reduced by 80% in mice receiving UC-764865 (18 ± 6 mg; P = 0.0001) as compared to vehicle treated mice (63 ± 29 mg) (Figure 11G). Upon discontinuation of UC-764865 treatment, all remaining mice succumbed to leukemia as evident by leukemic cell dissemination in the BM. These observations suggest that suppression of UBE2N with UC-764865 is sufficient to delay disease onset and that prolonged administration of UC-764865 is likely required to fully suppress leukemic cells *in vivo.*

Figure 9 demonstrates that inhibition of UBE2N catalytic function suppresses AML *in vitro.* Fig. 9A. Proliferation of AML cell lines upon treatment with increasing concentrations of UC-764864 for 24 hours. Error bars represent the SEM of 3 independent experiments in technical triplicates. Fig. 9B. Colony formation assay of AML (THP-1, MOLM-13, OCI-AML2, OCI-AML3), MDS (MDSL), and cord blood CD34+ cells upon treatment with DMSO or UC-764864 at the indicated concentrations. Data are presented normalized to DMSO. Error bars represent the SEM of 3 independent experiments in technical duplicates. Fig. 9C. Viability of MOLM-13 cells treated with UC-764864 at the indicated concentrations or DMSO was assessed by trypan blue staining followed by cell counts for 96 hours. Data are normalized to DMSO. Error bars are the standard deviation of 3 independent experiments. Fig. 9D. Immunoblot of total and cleaved caspase 3 in MOLM-13 cells treated with UC-764864 (2 µM) for 24 hours. Fig. 9E. Colony formation assay of THP-1 cells transduced with lentivirally expressed non-silencing control shRNA (shControl) or UBE2N shRNA (shUBE2N-3) and treated with increasing concentrations of UC-764864. Fig. 9F. Cell proliferation by MTS assay of MOLM-13 cells transduced with lentivirally expressed UBE2N wild type or UBE2N C87S mutant overexpressing vectors after 48 hr treatment with increasing concentrations of UC-764864 treated with increasing concentrations of UC-764864 was measured after 72 hr.

Figure 10 depicts characterization of UC-764865. Fig. 10A. Ubiquitination levels of UBE2N at lysine 92 (K92) in MOLM-13 cells treated with 2µM UC-764865 or DMSO. Fig. 10B. Immunoblot of thioester bond formation assay (with ATP/without ATP controls and reactions with UC-764865 at 1 or 2 pM) for UBE2N/MMS2. UBE2N or biotinylated-ubiquitin-enzyme conjugates were detected using UBE2N antibodies or Streptavidin-HRP detection system, respectively as described in "Methods" section. Quantification of Ub-UBE2N is indicated. (*, denote predicted bands). Fig. 10C. Proliferation of MOLM-13 and MDSL cells upon treatment with increasing concentrations of UC-764865 for 24 hr. Error bars represent the SEM of 3 independent experiments in technical triplicates. Fig. 10D. Colony formation assay in primary Lin-C D34+C D38- sorted M DS patient samples (n = 2) and healthy BM (n = 2). Cells were plated in methylcellulose containing UC-764865 (2 pM) and then colonies were manually scored at 14 days. Fig. 10E. Colony formation assay using AML (THP-1, MOL M-13, OCI-AML2, and OCI-AML3) cells treated with UC-764865 at the indicated concentrations. Data is presented as normalized to DMSO. Error bars represent the SEM of 3 independent experiments in technical duplicates. Significance was determined with a Student's T test (", P < 0.05). Fig. 10F. Colony formation assay using a primary MDS patient sample (n =1) and cord blood CD344 (n = 3). Cells were plated in methylcellulose containing UC-764865 at the indicated concentrations and then colonies were scored at 14 days. Error bars represent the standard deviation of technical duplicates (MDS sample) or SEM of three independent experiments in technical duplicates (Cord blood C D34+ cells).

Figure 11 demonstrates that inhibition of UBE2N catalytic function suppresses AML *in vivo.* Fig. 11A. Plasma levels of UC-764864 and UC-764865 in mice measured at the indicated time points during 24 hrs. Fig. 11B. Body weight of BoyJ mice (n = 3/group) treated with four doses of 25 mg/kg of UC-764865 over two weeks. Body weight of the mice was determined before the start of the treatment and after each dose (0 indicates after the first dose). Data is normalized to body weight prior to dosing. Fig. 11C. Schematic of experimental design for xenotransplants of MOLM-13 cells in NSGmice. Fig. 11D. MOLM-13 cells (1 x 104) were xenotransplanted into NSG mice (n = 10/group). Starting the day after transplantation, the mice were treated with 2 mg/kg of UC-764865 or vehicle control for 7 days. At day 10 after transplantation all the mice were euthanized and engraftment in BM and spleen were determined. Box plots display the range of variation (first and third quartile) and the median; individual data points (one per mouse) are displayed as filled circles. Fig. 11E. H&E stains of peripheral blood (PB) smears from mice transplanted with MOLM-13 treated with UC-764865 or vehicle control. MOLM-13 cells are indicated with black arrows (40X magnification). One MOLM-13 cell is shown in lower left corner. Fig. 11F. Kaplan Meier survival analysis of NSG mice (n = 10/group from 2 independent experiments) xenotransplanted with MOLM-13 cells (1 x 104 cells per mouse). Mice were treated with UC-764865 at 2 mg/kg or vehicle control as indicated (shaded grey). * P < 0.05. Fig. 11G. Spleen weight of mice treated with UC-764865 or vehicle (n = 10/group).

Figure 12 depicts *in vivo* pharmacokinetic properties and toxicity of UC-764865. Fig. 12A. Peripheral blood counts of BoyJ mice (n = 3/group) treated with four doses of 25 mg/kg of UC-764865 over two weeks. White blood cell counts (WBC), Red blood cell counts (RBC) and platelet (PLT) levels are indicated. Error bars are the standard deviation of data from 3 mice. Blood counts were determined before dosing (0) and at the end of the treatment (2). Fig. 12B. H&E staining of murine tissues after treatment with UC-764865 or vehicle control.

**Table 4. AML cell lines IC₅₀.**

| **Cell line** | **IC₅₀ (mM)** | **Assay** |
|---|---|---|
| KG-1a | >10 | MTS |
| MDS92 | >10 | MTS |
| MDSL | 5.6 | MTS |
| MV4-11 | 5 | MTS |
| Kasumi-1 | 6.6 | MTS |
| NOMO-1 | 6.7 | MTS |
| HL-60 | 4.3 | MTS |
| MOLM-14 | 0.02 | MTS |
| MOLM-13 | 1.9 | MTS |
| THP-1 | 2.5 | MTS |
| SKM-1 | 0.4 | MTS |
| Cord Blood CD34+ | >10 | MTS |

**Table 5. Differential ubiquitination enrichment screen.**

| **Plasma Stability** | | | | | | | |
|---|---|---|---|---|---|---|---|
| **Compound** | **Species** | **Concentration (mM)** | **Anti-coagulant** | T **1/2 (min)** | **% Remaning at T60** | | |
| UC-764864 | C57/Bl6 | 1 | K2EDTA | 31.8 | 21.4 | | |
| UC-764865 | C57/Bl6 | 1 | K2EDTA | 53.6 | 39.6 | | |

| **Microsomal Stability** | | | | | | | |
|---|---|---|---|---|---|---|---|
| **Compound** | **Species** | **Concentration (mM)** | **Matrix concentration (mg/ml)** | **T 1/2 (min)** | **% Remaning at T60** | **CLint (ml/min/kg)** | **CLint (L/hr/kg)** |
| UC-764864 | C57/Bl6 | 1 | 0.5 | 4.99 | 0.02 | 1093 | 65.6 |
| UC-764865 | C57/Bl6 | 1 | 0.5 | 4.97 | 0.2 | 1097 | 65.8 |

| **Aqueous Solubility** | | | | | | | |
|---|---|---|---|---|---|---|---|
| **Compound** | **Species** | **Highest soluble concentration (mM)** | | | | | |
| UC-764864 | C57/Bl6 | 1 | | | | | |
| UC-764865 | C57/Bl6 | 5 | | | | | |

**Table 6. In vivo PK.**

| **UC-764864** | | | | | | |
|---|---|---|---|---|---|---|
| **Dose** | **T1/2 (hr)** | **Tmax (hr)** | **Cmax (ng/ml)** | **AUClast (hr*ng/mL)** | **AUCinf (hr*ng/mL)** | |
| 20 mg/kg | 2.07 | 0.25 | 446.3 | 435.8 | 460.7 | Mean |
| | 0.53 | 0.00 | 169.4 | 150.2 | 181.3 | SD |

| **UC-764865** | | | | | | |
|---|---|---|---|---|---|---|
| **Dose** | **T1/2 (hr)** | **Tmax (hr)** | **Cmax (ng/ml)** | **AUClast (hr*ng/mL)** | **AUCinf (hr*ng/mL)** | |
| 20 mg/kg | 8.80 | 0.33 | 216.00 | 346.06 | 412.52 | Mean |
| | 3.28 | 0.14 | 103.06 | 46.29 | 42.15 | SD |

### EXAMPLE 7

### Baseline oncogenic immune signaling states in AML confer sensitivity to inhibition of UBE2N catalytic activity

Next, the inventors sought to identify molecular features that correlate with AML cell sensitivity to inhibition of UBE2N catalytic activity by using UC-764864 as a chemical probe. For this, the response of 26 patient-derived AML samples (PDX AMLs) was correlated to inhibition with escalating doses of UC-764864 with baseline gene expression signatures by performing RNA sequencing. The PDX AMLs showed a range of sensitivity to UBE2N inhibition (Figure 13A); therefore, a hierarchical clustering method was first applied to unbiasedly categorize the PDX AMLs according to their sensitivity to UC-764864 (Figure 14A). The analysis classified the PDX AMLs into two significantly distinct subgroups: six AMLs (JM1, JM7, JM18, JM26, JM30, and JM40) that were sensitive to UC-764864 ("UBE2N-dependent"), whereas 20 AMLs were resistant to UC-764864 ("UBE2N-resistant") (Figure 13A, Figure 14A).

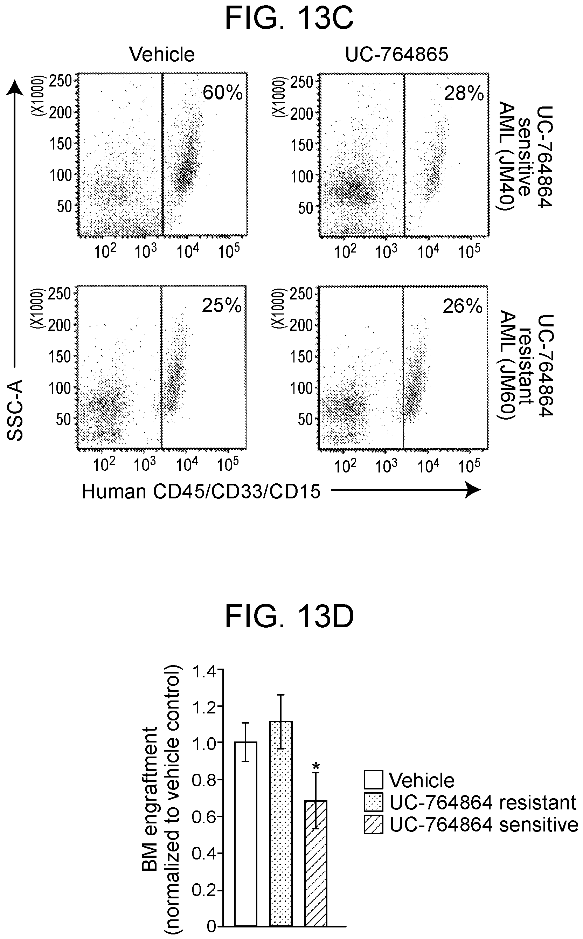

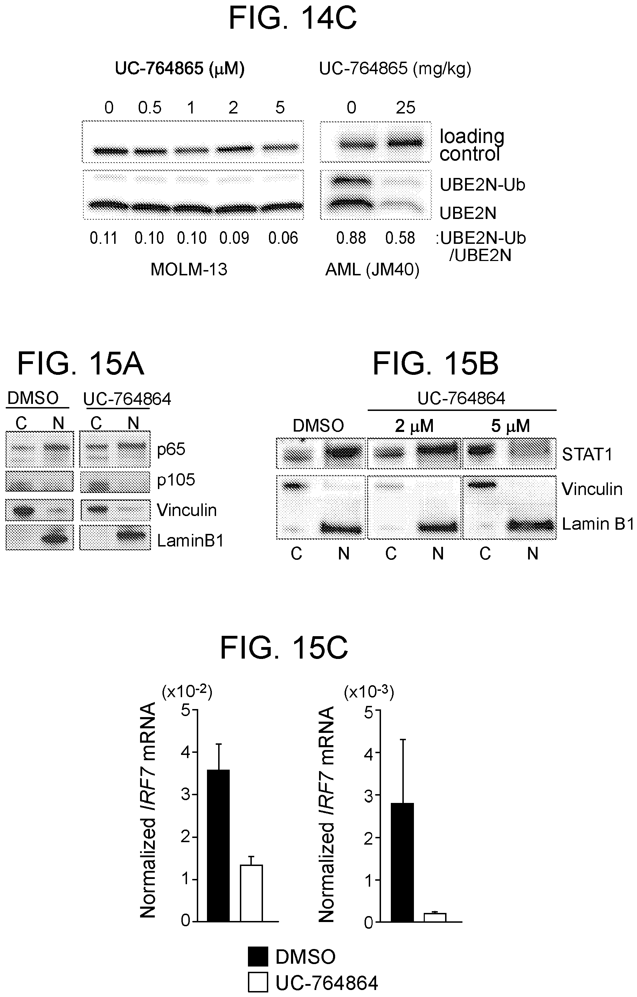

To determine whether UBE2N-dependent AMLs remain sensitive to inhibition of UBE2N catalytic function *in vivo,* the inventors examined three of the UBE2N-sensitive PDX AMLs (JM7, JM40, JM26) and one UBE2N-resistant PDX AML (JM60) in xenograft models. Patient-derived AML cells were xenografted in NSGS mice and 5 days after transplantation the mice were treated with UC-764865 or vehicle control IP daily (Figure 13B). A significant overall reduction in leukemic burden was observed in the BM following treatment with UC-764865 in mice xenografted with UBE2N-sensitive AML cells (P = 0.008) (Figure 13C and 13D, Figure 14B). UC-764865 resulted in an approximate 30-60% reduction in leukemic burden of the UBE2N-sensitive AML cells in the BM of xenografted mice (Figure 13B). In contrast, the leukemic burden of mice xenografted with an UBE2N-resistant sample (AML-JM60) did not decrease after treatment with UC-764865 (Figure 13C and 13D, Figure 14B). The reduction in leukemic cells in vivo following treatment with UC-764865 correlated with suppression of UBE2N function (Figure 14C). Total and ubiquitinated UBE2N were reduced in BM cells of mice xenografted with AML-JM40 cells treated with UC-764865 in comparison with vehicle treated mice (Figure 14C, right panel), which is consistent with reduced UBE2N charged with Ub ("UBE2N-Ub") following *in vitro* treatment (Figure 14C, left panel).

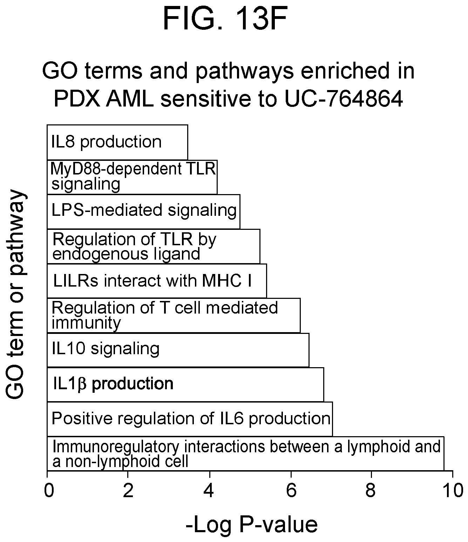

Comparison of baseline gene expression profiles between UBE2N-dependent and UBE2N-resistant PDX AMLs identified overexpression of genes related to innate immune signaling as the main predictor of response to UC-764864 (Figure 13E and 13F; Table 7 and Table 8). Specifically, UBE2N-dependent PDX AMLs exhibited an enrichment of genes at baseline belonging to the TLR signaling pathway, complement and coagulation cascades and production of proinflammatory cytokines (Figure 13F; Table 8), indicating that these AMLs rely on oncogenic immune signaling states. As evidence that UBE2N catalytic activity directly regulates the expression of UBE2N-dependent genes in AML, inhibition of baseline NF-κB, STAT1, IRF7 and IRF1 activity was observed in UBE2N-dependent AML cells upon treatment with UC-764864 (Figure 15A-C). These findings indicate that UBE2N regulates oncogenic immune signaling states in AML cells, and that baseline oncogenic immune signaling states in AML confer sensitivity to inhibition of UBE2N catalytic activity.

To establish whether the UBE2N-dependent oncogenic immune signaling states are evident in an independent cohort of AML patient samples, an unsupervised hierarchical clustering analysis of the RNA sequencing data from the Cancer Genome Atlas (TCGA) and Leucegene databases for differential expression of the UBE2N-dependent genes was performed. AML patients of the Leucegene database revealed 2 groups of AML patients characterized by the distinct expression of UBE2N-dependent genes (Figure 13G). Group 1 consists of reduced expression of UBE2N-dependent genes, while Group 2 consists of AML patients with increased expression of these genes at diagnosis. Interestingly, the UBE2N-dependent gene signature segregated healthy controls from both AML Groups 1 and 2 (Figure 13G). Independent gene expression analysis of the UBE2N-dependent gene signature in the TCGA also divided the AML patients in two groups based on low (Group 1) and high (Group 2) expression of UBE2N-dependent genes (Figure 13H). AML patients expressing higher levels of UBE2N-dependent genes (Group 2) associated with shorter overall survival as compared to patients expressing lower levels of UBE2N-dependent genes (Group 1) (Figure 13I), and were significantly enriched for myelomonocytic (AML-M4 (M4); Hypergeometric test, P = 4 x 10⁻⁸) and monocytic (AML-MS (M5); Hypergeometric test, P = 0.003) AML subtypes (Figure 16A).

Collectively, these findings indicate that interfering with UBE2N catalytic function abrogates leukemic function and underscores the dependency of AML cells on UBE2N-dependent oncogenic immune signaling states. These findings further demonstrate that certain subtypes of AML are responsive to UBE2N inhibition, namely AML-M4 and AML-M5, and these subtypes can be treated by administrations of a UBE2N inhibitor. UBE2N inhibition therefore represents a useful treatment strategy for AML-M4 and AML-M5, which have been shown to be AML subtypes which are particularly resistant to other treatments, such as BCL2 inhibitors (e.g. venetoclax). UBE2N inhibition can be used as an alternative therapy and can also be used to resensitize AML-M4 and AML-M5 to venetoclax, thereby enhancing the effectiveness of subsequent venetoclax administration.

Figure 13 demonstrates that baseline oncogenic immune signaling states in AML confer sensitivity to inhibition of UBE2N catalytic function. Fig. 13A. AML patient derived cells were incubated for 24 h with increasing concentrations of UC-764864. Cell viability was measured by MTS assay. Data is expressed normalized to the DMSO control. Error bars are the standard deviation of technical triplicates. Fig. 13B. UC-764864 sensitive (JM7, JM40, JM26) and resistant (JM60) PDX AML samples were xenografted in sublethally-irradiated NSGS mice and treated with vehicle control or 25 mg/kg UC-764865 by intraperitoneal (IP) injection daily for 4-6 weeks. Fig. 13C. Representative FACS plot of human engraftment in BM of NSGS mice xenotransplanted with a sensitive and resistant PDX AML and treated with 25 mg/kg of UC-764865 or vehicle control at 6 weeks after transplant. Fig. 13D. Summary of BM engraftment in UC-764864 sensitive and resistant PDX AMLs determined by flow cytometry. Data is normalized to vehicle control (n=3 sensitive PDX samples xenotransplanted into 10 mice each, n=1 resistant PDX sample xenotransplanted into 10 mice). Error bars are the Standard error of the mean. Fig. 13E. Heatmap showing the expression levels of the top 50 genes that are differentially expressed between UC-764864 sensitive and resistant PDX AML cells (based on Figure 13A and Figure 14A). Fig. 13F. Pathways and GO terms enriched in PDX AML sensitive to UC-764864. Fig. 13G. Heatmap showing the expression levels of UBE2N-dependent genes in AML patients from Leucegene (GSE49642). Group 1 indicates patients with lower gene expression and Group 2 patients with higher gene expression of UBE2N-dependent genes. Healthy controls (GSE48846) are indicated in red and AML patient samples in black. Overexpressed genes and downregulated genes are indicated. Fig. 13H. Heatmap showing the expression levels of UBE2N-dependent innate immune genes in AML patients from TCGA. Group 1 indicates patients with lower gene expression and Group 2 patients with high gene expression of UBE2N-dependent immune genes. Overexpressed genes and downregulated genes are indicated. Fig. 13I. Kaplan Meier analysis of 173 TCGA AML patient samples stratified according to low (blue) or high (red) expression of UBE2N-dependent immune genes.

Figure 14 demonstrates the effects of UC-764864 on UBE2N-dependent signaling in AML. Fig. 14A. Dendrogram of unsupervised hierarchical clustering of patient derived AML (PDX AML) based on in vitro sensitivity to UC-764864. The numbers on the left of each pair are called AU (Approximately Unbiased) P value and clusters with AU larger than 90% are highlighted by shaded rectangles. This AU P value > 90% corresponds to P value < 10%. Fig. 14B. BM cells were aspirated from vehicle and UC-764865 treated mice after 2-6 weeks of treatment and BM engraftment was determined by flow cytometry (human CD15, CD33 and/or human CD45). Data are expressed as a percentage of the total number of viable cells. Fig. 14C. Immunoblot for UBE2N and ubiquitinated UBE2N (Ub-UBE2N) in MOLM-13 cells treated with increasing concentrations of UC-764865 *in vitro* (left panel) or JM40 cells (right panel) collected from BM of xenografted NSGS mice that were treated with 25 mg/kg of UC-764865 or vehicle control. BM cells were collected at time of death from animals that presented human engraftment >60%. Loading controls are either GAPDH (MOLM-13) or vinculin (JM40). The UBE2N and Ub-UBE2N bands were quantified and normalized to the corresponding loading control. The ratio of Ub-UBE2N to total UBE2N is indicated.

Figure 15 demonstrates the effects of UC-764864 on UBE2N-dependent signaling in AML. Fig. 15A. Immunoblot showing the nuclear (N) and cytoplasmic (C) expression of the indicated NF-KB transcription factors upon treatment of MOLM-13 cells with UC-764864 (2 iiM) or DMSO for 48 hr. Vinculin and LaminB1 were used as loading controls for cytoplasmic (C) and nuclear fractions (N), respectively. Fig. 15B. Immunoblot showing the nuclear (N) and cytoplasmic (C) expression of STAT1 upon treatment of MOLM-13 cells with increasing concentrations of UC-764864 or DMSO for 24 hr. Fig. 15C. Gene expression levels of IRFs in THP-1 cells treated with DMSO or 2µM UC-764864. Cells were incubated in the presence of lipopolisacharide (LPS) for 3 hours in the presence or absence of UC-764864. Gene expression was determined by qRT-PCR and normalized to GAPDH. Error bars are the SD of technical triplicates.

Figure 16 demonstrates the effects of UC-764864 on UBE2N-dependent signaling in AML. Heatmap of individual mutations or AML subtype in AML patient samples from TCGA clustered in Group 1 (UBE2N-dependent signature low) and Group 2 (UBE2N-dependent signature high). Positive events are shown. Mutations or AML subtype are listed on the left.

**Table 7. Differential gene expression between UC-764864 sensitive and resistant AML PDX.**

| **Gene ID** | **log Fold Change [UC-764864 Resistant AML-UC-764864 Sensitive AML]** | **AveExpr** | **t** | **P. Value** | **adj.P.Val** | **B** |
|---|---|---|---|---|---|---|
| S100A12 | -4.52 | 0.9021 | -3.999 | 0.000454 | 0.000454 | -0.4728 |
| S100A8 | -4.391 | 4.795 | -2.972 | 0.006219 | 0.006219 | -2.268 |
| GIMAP4 | -4.266 | -0.8328 | -4.697 | 7.12E-05 | 7.12E-05 | -0.2057 |
| C1QB | -3.93 | -1.693 | -4.371 | 0.00017 | 0.00017 | -1.133 |
| CD1C | -3.725 | -0.3159 | -3.316 | 0.002648 | 0.002648 | -2.063 |
| VSIG4 | -3.559 | 0.6764 | -3.899 | 0.000591 | 0.000591 | -0.8291 |
| CD300E | -3.556 | 1.241 | -3.154 | 0.003973 | 0.003973 | -1.988 |
| TLR8 | -3.462 | 1.329 | -3.723 | 0.000935 | 0.000935 | -0.9253 |
| HLA-DOB1 | -3.443 | 3.446 | -3.038 | 0.005281 | 0.005281 | -2.106 |
| CD163 | -3.415 | 1.437 | -3.43 | 0.001985 | 0.001985 | -1.47 |
| CCR2 | -3.382 | 3.023 | -3.013 | 0.005615 | 0.005615 | -2.155 |
| TNFSF8 | -3.323 | 1.76 | -4.347 | 0.000181 | 0.000181 | 0.3627 |
| ARAP2 | -3.323 | 3 | -3.53 | 0.001535 | 0.001535 | -1.092 |
| P2RY6 | -3.231 | 1.807 | -3.83 | 0.000707 | 0.000707 | -0.6331 |
| CD14 | -3.175 | 1.245 | -3.646 | 0.001139 | 0.001139 | -1.139 |
| FAM198B | -3.117 | 2.644 | -2.867 | 0.008014 | 0.008014 | -2.443 |
| GBP1 | -3.116 | 0.129 | -2.937 | 0.006762 | 0.006762 | -2.565 |
| KRT19 | -3.113 | -0.04997 | -3.802 | 0.000761 | 0.000761 | -1.286 |
| LILRA1 | -3.085 | 2.477 | -3.97 | 0.000489 | 0.000489 | -0.2261 |
| MS4A4A | -3.072 | 2.395 | -3.205 | 0.003502 | 0.003502 | -1.791 |
| SIGLEC9 | -3.059 | 2.208 | -4.988 | 3.27E-05 | 3.27E-05 | 1.805 |
| TRPS1 | -3.056 | 3.969 | -3.393 | 0.002177 | 0.002177 | -1.363 |
| MMP9 | -3.044 | 1.881 | -3.727 | 0.000925 | 0.000925 | -0.8467 |
| MLLT4 | -2.992 | 4.213 | -4.213 | 0.000258 | 0.000258 | 0.4695 |
| MAF | -2.981 | 0.4357 | -3.788 | 0.000788 | 0.000788 | -1.148 |
| FGL2 | -2.974 | 5.398 | -3.17 | 0.003818 | 0.003818 | -1.873 |
| MEIKIN | -2.959 | -1.917 | -3.456 | 0.001858 | 0.001858 | -2.444 |
| ZNF532 | -2.949 | 3.503 | -3.84 | 0.000688 | 0.000688 | -0.4008 |
| C1QC | -2.944 | -1.467 | -3.359 | 0.002376 | 0.002376 | -2.398 |
| CD48 | -2.879 | 3.019 | -3.033 | 0.005355 | 0.005355 | -2.116 |
| IL1RN | -2.84 | 2.803 | -3.311 | 0.002679 | 0.002679 | -1.56 |
| RTN1 | -2.821 | 0.3468 | -2.879 | 0.007782 | 0.007782 | -2.633 |
| CX3CR1 | -2.787 | 3.747 | -3.288 | 0.002839 | 0.002839 | -1.588 |
| CD1D | -2.769 | 3.41 | -3.358 | 0.002382 | 0.002382 | -1.444 |
| LILRB2 | -2.75 | 2.612 | -3.508 | 0.001625 | 0.001625 | -1.18 |
| SIPA1L2 | -2.748 | 1.913 | -3.096 | 0.004586 | 0.004586 | -2.055 |
| VNN1 | -2.746 | 3.296 | -3.419 | 0.002039 | 0.002039 | -1.318 |
| WFS1 | -2.731 | -0.4466 | -3.134 | 0.004168 | 0.004168 | -2.411 |
| BCAR3 | -2.676 | 1.372 | -5.471 | 9.06E-06 | 9.06E-06 | 2.137 |
| VNN2 | -2.665 | 1.697 | -3.217 | 0.003398 | 0.003398 | -1.859 |
| SLC7A7 | -2.655 | 3.081 | -5.422 | 1.03E-05 | 1.03E-05 | 2.968 |
| ATP10A | -2.617 | 3.39 | -2.86 | 0.008145 | 0.008145 | -2.462 |
| NLRC4 | -2.59 | 2.502 | -4.699 | 7.08E-05 | 7.08E-05 | 1.26 |
| CXCL10 | -2.572 | 0.000616 | -3.232 | 0.003267 | 0.003267 | -2.177 |
| LINC00968 | -2.558 | -1.248 | -3.348 | 0.002443 | 0.002443 | -2.368 |
| DNAJC5B | -2.532 | -0.8336 | -2.921 | 0.007033 | 0.007033 | -2.795 |
| ACOX2 | -2.52 | 0.07631 | -2.832 | 0.008719 | 0.008719 | -2.757 |
| PYHIN1 | -2.505 | -0.7658 | -3.424 | 0.002013 | 0.002013 | -2.119 |
| PLK2 | -2.481 | 2.192 | -3.45 | 0.001885 | 0.001885 | -1.361 |
| TNNT1 | -2.475 | 2.522 | -2.838 | 0.008586 | 0.008586 | -2.505 |
| TSPEAR-AS1 | -2.457 | -1.039 | -3.44 | 0.001936 | 0.001936 | -2.218 |
| LPAR1 | -2.404 | 1.621 | -3.37 | 0.002311 | 0.002311 | -1.613 |
| HLA-DMB | -2.378 | 4.489 | -2.844 | 0.008456 | 0.008456 | -2.532 |
| SECTM1 | -2.376 | -0.7786 | -3.234 | 0.003254 | 0.003254 | -2.392 |
| GPR84 | -2.367 | 1.385 | -3.028 | 0.005422 | 0.005422 | -2.262 |
| CCR1 | -2.35 | 4.05 | -2.847 | 0.00841 | 0.00841 | -2.509 |
| EMP1 | -2.338 | 2.301 | -2.889 | 0.007602 | 0.007602 | -2.419 |
| C1orf115 | -2.33 | -0.337 | -3.106 | 0.004478 | 0.004478 | -2.452 |
| LILRA2 | -2.304 | 4.561 | -2.896 | 0.007477 | 0.007477 | -2.43 |
| SIGLEC7 | -2.301 | 1.02 | -3.065 | 0.004942 | 0.004942 | -2.252 |
| IFITM3 | -2.274 | 4.031 | -3.344 | 0.00247 | 0.00247 | -1.47 |
| LINC01272 | -2.245 | 0.8181 | -3.031 | 0.005378 | 0.005378 | -2.341 |
| GPAT3 | -2.232 | 3.491 | -2.998 | 0.005829 | 0.005829 | -2.186 |
| EGLN1 | -2.226 | 4.289 | -3.052 | 0.00511 | 0.00511 | -2.095 |
| AHNAK2 | -2.158 | 2.187 | -2.837 | 0.008614 | 0.008614 | -2.524 |
| LRP1 | -2.156 | 4.258 | -3.087 | 0.004682 | 0.004682 | -2.019 |
| CTSE | -2.151 | -0.2873 | -3.212 | 0.003441 | 0.003441 | -2.313 |
| FCMR | -2.099 | 1.635 | -3.076 | 0.004815 | 0.004815 | -2.153 |
| ZAK | -2.085 | 5.736 | -2.987 | 0.005989 | 0.005989 | -2.279 |
| SIRPD | -2.084 | -0.3318 | -2.837 | 0.00861 | 0.00861 | -2.836 |
| ACPP | -2.058 | 3.184 | -2.958 | 0.006426 | 0.006426 | -2.265 |
| C19orf38 | -2.046 | 3.221 | -2.93 | 0.006876 | 0.006876 | -2.319 |
| RIN2 | -2.042 | 1.635 | -2.885 | 0.007674 | 0.007674 | -2.489 |
| TNFSF10 | -1.975 | 4.917 | -4.38 | 0.000166 | 0.000166 | 0.8891 |
| LILRA6 | -1.973 | 0.7808 | -3.363 | 0.00235 | 0.00235 | -1.835 |
| SLC15A3 | -1.954 | 2.788 | -2.907 | 0.007276 | 0.007276 | -2.369 |
| FAM105A | -1.938 | 4.907 | -2.862 | 0.008101 | 0.008101 | -2.514 |
| SH3BP5 | -1.876 | 1.744 | -3.827 | 0.000713 | 0.000713 | -0.7956 |
| LII,RAS | -1.852 | 1.84 | -3.608 | 0.001257 | 0.001257 | -1.188 |
| C10orf105 | -1.818 | 1.662 | -2.97 | 0.006243 | 0.006243 | -2.354 |
| CMTM4 | -1.766 | 4.571 | -3.084 | 0.004725 | 0.004725 | -2.036 |
| NLRP1 | -1.747 | 5.498 | -3.766 | 0.000836 | 0.000836 | -0.5373 |
| SIGLEC17P | -1.738 | 3.446 | -3.094 | 0.004607 | 0.004607 | -1.993 |
| PRUNE2 | -1.737 | 2.806 | -3.255 | 0.003089 | 0.003089 | -1.709 |
| MLK7-AS1 | -1.727 | -0.2645 | -3.746 | 0.00088 | 0.00088 | -1.592 |
| TLR1 | -1.714 | 3.732 | -3.401 | 0.002137 | 0.002137 | -1.352 |
| CALHM2 | -1.703 | 3.788 | -2.934 | 0.006807 | 0.006807 | -2.316 |
| FAM72C | -1.681 | -0.6284 | -3.148 | 0.00403 | 0.00403 | -2.537 |
| POU2F2 | -1.622 | 5.57 | -2.988 | 0.005976 | 0.005976 | -2.274 |
| ZNF470 | 1.701 | 3.239 | 3.311 | 0.002678 | 0.002678 | -1.802 |
| LOC100996437 | 1.704 | 0.129 | 3.04 | 0.005257 | 0.005257 | -2.981 |
| FADS3 | 1.781 | 3.701 | 2.9 | 0.007392 | 0.007392 | -2.455 |
| SUGT1P1 | 1.818 | 1.809 | 3.307 | 0.002711 | 0.002711 | -2.178 |
| SYNE4 | 1.824 | -0.2189 | 3.542 | 0.001491 | 0.001491 | -2.63 |
| PRDM11 | 1.855 | 3.595 | 3.338 | 0.002508 | 0.002508 | -1.708 |
| ETV2 | 1.966 | -0.589 | 3.342 | 0.00248 | 0.00248 | -2.978 |
| HHIPL2 | 1.973 | -0.878 | 3.01 | 0.005656 | 0.005656 | -3.347 |
| PRICKLE1 | 2.017 | 3.028 | 2.899 | 0.007417 | 0.007417 | -2.543 |
| PRSS27 | 2.021 | 0.4384 | 3.355 | 0.002399 | 0.002399 | -2.619 |
| CLEC11A | 2.05 | 8.148 | 3.647 | 0.001137 | 0.001137 | -0.8159 |
| CBY3 | 2.05 | -0.9617 | 2.786 | 0.009716 | 0.009716 | -3.532 |
| LOC101929374 | 2.159 | -1.116 | 2.913 | 0.007177 | 0.007177 | -3.499 |
| DNAH14 | 2.194 | 2.85 | 2.78 | 0.009861 | 0.009861 | -2.776 |
| MIP | 2.414 | -1.219 | 3.043 | 0.005217 | 0.005217 | -3.454 |
| TSTD1 | 2.54 | 2.414 | 3.068 | 0.004911 | 0.004911 | -2.473 |
| ZNF219 | 2.575 | 4.234 | 4.003 | 0.00045 | 0.00045 | -0.4808 |
| PNMA6A | 2.702 | 0.9111 | 3.091 | 0.004641 | 0.004641 | -2.902 |
| PPP1R26 | 3.583 | 3.551 | 3.787 | 0.000792 | 0.000792 | -1.263 |

**Table 8. Gene enrichment analysis of differentially expressed genes in UC-764864 sensitive AML PDXs.**

| **GOID** | **GOTerm** | **Term PValue** | **Term PValue Corrected with Bonferroni step down** | **Group PValue** | **Group PValue Corrected with Bonferroni step down** | **GO Levels** | **% Associated Genes** | **Nr. Genes** | **Associated Genes Found** |
|---|---|---|---|---|---|---|---|---|---|
| R-HSA:198933^{1*} | Immunoregulatory interactions between a Lymphoid and a non-Lymphoid cell | 1.52E-10 | 9.14E-09 | 6.85E-20 | 6.85E-19 | [-1] | 6.02 | 8.00 | [CD1C, CD1D, CD300E, LILRA1, LILRA2, LILRA5, SIGLEC7, SIGLEC9] |
| G0:0032755^{1**} | positive regulation of interleukin-6 production | 9.36E-08 | 5.15E-06 | 6.85E-20 | 6.85E-19 | [4, 5, 6] | 5.04 | 6.00 | [IL1RN, LILRA2, LILRA5, LILRB2, TLR1, TLR8] |
| GO:0032611^{1**} | interleukin-1 beta production | 1.51E-07 | 8.16E-06 | 6.85E-20 | 6.85E-19 | [4] | 4.65 | 6.00 | [CX3CR1, LILRA2, LILRA5, NLRC4, NLRP1, TLR8] |
| GO:0050702^{1**} | interleukin-1 beta secretion | 3.05E-07 | 1.62E-05 | 6.85E-20 | 6.85E-19 | [5, 6, 8, 9, 10, 11] | 6.58 | 5.00 | [LILRA2, LILRA5, NLRC4, NLRP1, TLR8] |
| R-HSA:199043^{1***} | LILRs interact with MHC Class I | 3.92E-06 | 1.41E-04 | 6.85E-20 | 6.85E-19 | [-1] | 17.65 | 3.00 | [LILRA1, LILRA2, |
| | | | | | | | | | LILRA5] |
| R-HSA:5686938^{1*} | Regulation of TLR by endogenous ligand | 5.58E-06 | 1.90E-04 | 6.85E-20 | 6.85E-19 | [-1] | 15.79 | 3.00 | [CD14, S100A8, TLR1] |
| GO:0031663^{1**} | lipopolysaccharide-mediated signaling pathway | 1.80E-05 | 3.95E-04 | 6.85E-20 | 6.85E-19 | [4, 5, 6, 7, 9] | 4.76 | 4.00 | [CD14, LILRA2, MAP3K20, S100A8] |
| G0:0002755^{1**} | MyD88-dependent toll-like receptor signaling pathway | 6.41E-05 | 7.05E-04 | 6.85E-20 | 6.85E-19 | [7, 8, 9, 10, 11, 12] | 7.14 | 3.00 | [CD14, TLR1, TLR8] |
| GO:2000778^{1**} | positive regulation of interleukin-6 secretion | 7. 89E-05 | 7. 89E-04 | 6.85E-20 | 6.85E-19 | [5, 6, 7, 8, 9, 10, 11, 12] | 6.67 | 3.00 | [LILRA2, LILRA5, TLR8] |
| GO:0032757^{1**} | positive regulation of interleukin-8 production | 3.47E-04 | 6.94E-04 | 6.85E-20 | 6.85E-19 | [4, 5, 6] | 4.05 | 3.00 | [CD14, TLR1, TLR8] |
| R-HSA:8937654^{2***} | IL10 positively regulates plasma membrane-associated inflammatory mediators | 3.27E-07 | 1.70E-05 | 1.24E-16 | 1.12E-15 | [-1] | 37.50 | 3.00 | [CCR1, CCR2, IL1RN] |
| GO:0002709^{2**} | regulation of T cell mediated immunity | 5.67E-07 | 2.78E-05 | 1.24E-16 | 1.12E-15 | [6, 7] | 5.81 | 5.00 | [CCR2, CD1C, CD1D, IL1RN, MAP3K20] |
| R-HSA:380108^{2*} | Chemokine receptors bind chemokines | 1.91E-06 | 8.58E-05 | 1.24E-16 | 1.12E-15 | [-1] | 8.33 | 4.00 | [CCR1, CCR2, CX3CR1, CXCL10] |
| WP:2431²**** | Spinal Cord Injury | 2.95E-06 | 1.21E-04 | 1.24E-16 | 1.12E-15 | [-1] | 4.17 | 5.00 | [C1QB, CCR2, CXCL10, LILRB2, MMP9] |
| GO:0002821²** | positive regulation of adaptive immune response | 3.47E-06 | 1.32E-04 | 1.24E-16 | 1.12E-15 | [4, 5, 6] | 4.03 | 5.00 | [CCR2, CD1C, CD1D, IL1RN, MAP3K20] |
| GO:1903975^{2**} | regulation of glial cell migration | 4.70E-06 | 1.65E-04 | 1.24E-16 | 1.12E-15 | [5, 6, 7, 8, 9, 10] | 16.67 | 3.00 | [CCR2, CX3CR1, LRP1] |
| GO:0006968²** | cellular defense response | 6.09E-06 | 2.01E-04 | 1.24E-16 | 1.12E-15 | [4] | 6.25 | 4.00 | [CCR2, CX3CR1, FCMR, LILRB2] |
| GO:0090026²** | positive regulation of monocyte chemotaxis | 1.16E-05 | 3.12E-04 | 1.24E-16 | 1.12E-15 | [5, 6, 7, 8, 9, 10] | 12.50 | 3.00 | [CCR1, CCR2, CXCL10] |
| GO:0032729²** | positive regulation of interferon-gamma production | 1.34E-05 | 3.48E-04 | 1.24E-16 | 1.12E-15 | [4, 5, 6] | 5.13 | 4.00 | [CCR2, CD14, II,1RN, TLR8] |
| GO:0016493^{2**} | C-C chemokine receptor activity | 2.08E-05 | 4.36E-04 | 1.24E-16 | 1.12E-15 | [7, 8, 9, 10] | 10.34 | 3.00 | [CCR1, CCR2, CX3CR1] |
| GO:0071677²** | positive regulation of mononuclear cell migration | 2.30E-05 | 4.38E-04 | 1.24E-16 | 1.12E-15 | [4, 5, 6, 7, 8, 9] | 10.00 | 3.00 | [CCR1, CCR2, CXCL10] |
| GO:0001637²** | G protein-coupled chemoattractant receptor activity | 2.81E-05 | 4.49E-04 | 1.24E-16 | 1.12E-15 | [6, 7, 8] | 9.38 | 3.00 | [CCR1, CCR2, CX3CR1] |
| GO:0004950²** | chemokine receptor activity | 2.81E-05 | 4.49E-04 | 1.24E-16 | 1.12E-15 | [6, 7, 8, 9] | 9.38 | 3.00 | [CCR1, CCR2, CX3CR1] |
| GO:0042533²** | tumor necrosis factor biosynthetic process | 3.69E-05 | 5.16E-04 | 1.24E-16 | 1.12E-15 | [4, 5, 6, 7] | 8.57 | 3.00 | [CCR2, CX3CR1, TLR1] |
| GO:0002724²** | regulation of T cell cytokine production | 5.53E-05 | 6.64E-04 | 1.24E-16 | 1.12E-15 | [5, 6, 7, 8] | 7.50 | 3.00 | [CCR2, IL1RN, MAP3K20] |
| WP:24²**** | Peptide GPCRs | 3.61E-04 | 3.61E-04 | 1.24E-16 | 1.12E-15 | [-1] | 4.00 | 3.00 | [CCR1, CCR2, CX3CR1] |
| GO:1905517²** | macrophage migration | 3.61E-04 | 3.61E-04 | 1.24E-16 | 1.12E-15 | [4, 6, 7] | 4.00 | 3.00 | [CCR2, CX3CR1, S100A8] |
| GO:0005044²** | scavenger receptor activity | 3.61E-04 | 3.61E-04 | 1.24E-16 | 1.12E-15 | [8] | 4.00 | 3.00 | [CD 163, CX3CR1, LRP1] |
| GO:0071723³** | lipopeptide binding | 7.36E-09 | 4.27E-07 | 4.89E-13 | 3.91E-12 | [3, 4] | 30.77 | 4.00 | [CD 14, CD1C, CD1D, TLR1] |
| GO:0098883^{3**} | synapse pruning | 9.60E-07 | 4.61E-05 | 4.89E-13 | 3.91E-12 | [4] | 27.27 | 3.00 | [C1QB, C1QC, CX3CR1] |
| KEGG:04640³***** | Hematopoietic cell lineage | 1.03E-06 | 4.85E-05 | 4.89E-13 | 3.91E-12 | [-1] | 5.15 | 5.00 | [CD14, CD1C, CD1D, HLA-DMB, HLA-DQB1] |
| CORUM-FunCat:36251601³****** | innate immune response (invertebrates and vertebrates) | 5.58E-06 | 1.90E-04 | 4.89E-13 | 3.91E-12 | [3] | 15.79 | 3.00 | [C1QB, C1QC, S100A8] |
| KEGG:05150³***** | Staphylococcus aureus infection | 7. 76E-06 | 2.40E-04 | 4.89E-13 | 3.91E-12 | [-1] | 5.88 | 4.00 | [C1QB, C1QC, HLA-DMB, HLA-DQB1] |
| WP:2328³**** | Allograft Rejection | 2.36E-05 | 4.25E-04 | 4.89E-13 | 3.91E-12 | [-1] | 4.44 | 4.00 | [C1QB, C1QC, HLA-DMB, HLA-DQB1] |
| WP:3937³**** | Microglia Pathogen Phagocytosis Pathway | 5.53E-05 | 6.64E-04 | 4.89E-13 | 3.91E-12 | [-1] | 7.50 | 3.00 | [C1QB, C1QC, SIGLEC7] |
| KEGG:05321³***** | Inflammatory bowel disease (IBD) | 2.37E-04 | 9.46E-04 | 4.89E-13 | 3.91E-12 | [-1] | 4.62 | 3.00 | [HLA-DMB, HLA-DQB1, MAF] |
| GO:0050715⁴** | positive regulation of cytokine secretion | 2.70E-09 | 1.59E-07 | 9.03E-13 | 6.32E-12 | [4, 5, 6, 7, 8, 9, 10, 11] | 4.19 | 8.00 | [CD14, LILRA2, LILRA5, LRP1, NLRP1, S100A8, TLR1, TLR8] |
| GO:0032760⁴** | positive regulation of tumor necrosis factor production | 7. 24E-08 | 4.13E-06 | 9.03E-13 | 6.32E-12 | [5, 6, 7] | 5.26 | 6.00 | [CCR2, CD14, LILRA2, LILRA5, S100A8, TLR1] |
| R-HSA:6783783⁵* | Interleukin-10 signaling | 1.75E-06 | 8.05E-05 | 1.45E-12 | 8.72E-12 | [-1] | 8.51 | 4.00 | [CCR1, CCR2, CXCL10, IL1RN] |
| GO:0002711⁵** | positive regulation of T cell mediated immunity | 3.07E-06 | 1.23E-04 | 1.45E-12 | 8.72E-12 | [6, 7, 8] | 7.41 | 4.00 | [CD1C, CDID, IL1RN, MAP3K20] |
| GO:0032663⁵** | regulation of interleukin-2 production | 1.09E-05 | 3.15E-04 | 1.45E-12 | 8.72E-12 | [4, 5] | 5.41 | 4.00 | [CCR2, GBP1, MAP3K20, VSIG4] |
| GO:0045123⁵** | cellular extravasation | 3.20E-04 | 9.60E-04 | 1.45E-12 | 8.72E-12 | [3, 5, 6] | 4.17 | 3.00 | [CCR2, CX3CR1, IL1RN] |
| GO:0016045⁶** | detection of bacterium | 3.24E-06 | 1.26E-04 | 2.55E-12 | 1.28E-11 | [4, 6] | 18.75 | 3.00 | [CD1D, NLRC4, TLR1] |
| GO:0032330⁷** | regulation of chondrocyte differentiation | 1.69E-04 | 1.18E-03 | 1.69E-04 | 3.37E-04 | [4, 5, 6, 7, 8] | 5.17 | 3.00 | [MAF, TRPS1, ZNF219] |
| WP:3945⁸**** | TYROBP Causal Network | 2.16E-04 | 1.08E-03 | 2.16E-04 | 2.16E-04 | [-1] | 4.76 | 3.00 | [C1QC, MAF, SLC7A7] |

| | | | | | | | | | |
|---|---|---|---|---|---|---|---|---|---|
| GO Groups: ¹Group08; ²Group09; ³Group04; ⁴Group05; ⁵Group07; ⁶Group06; 7Group00; ⁸Group01 Ontology Source: *REACTOME_Pathways_27.02.2019; **GO_BiologicalProcess-EBI-UniProt-GOA_27.02.2019_00h00; ***REACTOME_Reactions_27.02.2019; ****WikiPathways_27.02.2019; *****KEGG_27.02.2019; ******CORUM_CORUM-FunCat-MIPS_04.09.2018. | | | | | | | | | |

The various methods and techniques described above provide a number of ways to carry out the invention. Of course, it is to be understood that not necessarily all objectives or advantages described can be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as taught or suggested herein. A variety of alternatives are mentioned herein. It is to be understood that some preferred embodiments specifically include one, another, or several features, while others specifically exclude one, another, or several features, while still others mitigate a particular feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be employed in various combinations by one of ordinary skill in this art to perform methods in accordance with the principles described herein. Among the various elements, features, and steps some will be specifically included and others specifically excluded in diverse embodiments.

Although the application has been disclosed in the context of certain embodiments and examples, it will be understood by those skilled in the art that the embodiments of the invention extend beyond the specifically disclosed embodiments to other alternative embodiments and/or uses and modifications and equivalents thereof.

In some embodiments, the numbers expressing quantities of ingredients, properties such as molecular weight, reaction conditions, and so forth, used to describe and claim certain embodiments of the application are to be understood as being modified in some instances by the term "about." Accordingly, in some embodiments, the numerical parameters set forth in the written description and attached claims are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In some embodiments, the terms "a" and "an" and "the" and similar references used in the context of describing a particular embodiment of the application (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (for example, "such as") provided with respect to certain embodiments herein is intended merely to better illuminate the application and does not pose a limitation on the scope of the application otherwise claimed. No language in the specification should be construed as indicating any non-claimed element essential to the practice of the application.

Preferred embodiments of this application are described herein. Variations on those preferred embodiments will become apparent to those of ordinary skill in the art upon reading the foregoing description. It is contemplated that skilled artisans can employ such variations as appropriate, and the application can be practiced otherwise than specifically described herein. Accordingly, many embodiments of this application include all modifications and equivalents of the subject matter recited in the claims appended hereto as permitted by applicable law. Moreover, any combination of the above-described elements in all possible variations thereof is encompassed by the application unless otherwise indicated herein or otherwise clearly contradicted by context.

All patents, patent applications, publications of patent applications, and other material, such as articles, books, specifications, publications, documents, things, and/or the like, referenced herein are hereby incorporated herein by this reference in their entirety for all purposes, excepting any prosecution file history associated with same, any of same that is inconsistent with or in conflict with the present document, or any of same that may have a limiting affect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the invention. Other modifications that can be employed can be within the scope of the application. Thus, by way of example, but not of limitation, alternative configurations of the embodiments of the application can be utilized in accordance with the teachings herein. Accordingly, embodiments of the present application are not limited to that precisely as shown and described.

### References

The references listed below are cited herein and incorporated by references herein in their entirety, and for all purposes.
1. Alessandrino, E.P., et al., WHO classification and WPSS predict posttransplantation outcome in patients with myelodysplastic syndrome: a study from the Gruppo Italiano Trapianto di Midollo Osseo (GITMO). Blood, 2008. 112(3): p. 895-902.
2. Pandolfi, A., L. Barreyro, and U. Steidl, Concise review: preleukemic stem cells: molecular biology and clinical implications of the precursors to leukemia stem cells. Stem Cells Transl Med, 2013. 2(2): p. 143-50.
3. Shastri, A., et al., Stem and progenitor cell alterations in myelodysplastic syndromes. Blood, 2017. 129(12): p. 1586-1594.
4. Ye, H., et al., Leukemic Stem Cells Evade Chemotherapy by Metabolic Adaptation to an Adipose Tissue Niche. Cell Stem Cell, 2016. 19(1): p. 23-37.
5. Hemmati, S., T. Haque, and K. Gritsman, Inflammatory Signaling Pathways in Preleukemic and Leukemic Stem Cells. Front Oncol, 2017. 7: p. 265.
6. Varney, M.E., et al., Deconstructing innate immune signaling in myelodysplastic syndromes. Exp Hematol, 2015. 43(8): p. 587-98.
7. Thaiss, C.A., et al., Integration of Innate Immune Signaling. Trends Immunol, 2016. 37(2): p. 84-101.
8. Rhyasen, G.W., et al., Targeting IRAK1 as a therapeutic approach for myelodysplastic syndrome. Cancer Cell, 2013. 24(1): p. 90-104.
9. Fang, J., et al., Ubiquitination of hnRNPA1 by TRAF6 links chronic innate immune signaling with myelodysplasia. Nat Immunol, 2017. 18(2): p. 236-245.
10. Varney, M.E., et al., Loss of Tifab, a del(5q) MDS gene, alters hematopoiesis through derepression of Toll-like receptor-TRAF6 signaling. J Exp Med, 2015. 212(11): p. 1967-85.
11. Ågerstam, H., et al., Antibodies targeting human IL1RAP (IL1R3) show therapeutic effects in xenograft models of acute myeloid leukemia. Proceedings of the National Academy of Sciences, 2015. 112(34): p. 10786-10791.
12. Askmyr, M., et al., Selective killing of candidate AML stem cells by antibody targeting of IL1RAP. Blood, 2013. 121(18): p. 3709-3713.
13. Fang, J., et al., Cytotoxic effects of bortezomib in myelodysplastic syndrome/acute myeloid leukemia depend on autophagy-mediated lysosomal degradation of TRAF6 and repression of PSMA1. Blood, 2012. 120(4): p. 858-67.
14. Barreyro, L., et al., Overexpression of IL-1 receptor accessory protein in stem and progenitor cells and outcome correlation in AML and MDS. Blood, 2012. 120(6): p. 1290-8.
15. Wang, T., et al., Gene Essentiality Profiling Reveals Gene Networks and Synthetic Lethal Interactions with Oncogenic Ras. Cell, 2017. 168(5): p. 890-903.e15.
16. Hodge, C.D., et al., Covalent Inhibition of Ubc13 Affects Ubiquitin Signaling and Reveals Active Site Elements Important for Targeting. ACS Chem Biol, 2015. 10(7): p. 1718-28.
17. Strickson, S., et al., The anti-inflammatory drug BAY 11-7082 suppresses the MyD88-dependent signalling network by targeting the ubiquitin system. Biochem J, 2013. 451(3): p. 427-37.
18. Eddins, M.J., et al., Mms2-Ubc13 covalently bound to ubiquitin reveals the structural basis of linkage-specific polyubiquitin chain formation. Nat Struct Mol Biol, 2006. 13(10): p. 915-20.
19. Klomsiri, C., P.A. Karplus, and L.B. Poole, Cysteine-based redox switches in enzymes. Antioxid Redox Signal, 2011. 14(6): p. 1065-77.
20. Gombodorj, N., et al., Inhibition of Ubiquitin-conjugating Enzyme E2 May Activate the Degradation of Hypoxia-inducible Factors and, thus, Overcome Cellular Resistance to Radiation in Colorectal Cancer. Anticancer Res, 2017. 37(5): p. 2425-2436.
21. Cheng, J., et al., A small-molecule inhibitor of UBE2N induces neuroblastoma cell death via activation of p53 and JNK pathways. Cell Death Dis, 2014. 5: p. e1079.
22. Pulvino, M., et al., Inhibition of proliferation and survival of diffuse large B-cell lymphoma cells by a small-molecule inhibitor of the ubiquitin-conjugating enzyme Ubc13-Uev1A. Blood, 2012. 120(8): p. 1668-77.
23. Ritorto, M.S., et al., Screening of DUB activity and specificity by MALDI-TOF mass spectrometry. Nat Commun, 2014. 5: p. 4763.
24. Lee, J., et al., BAY 11-7082 is a broad-spectrum inhibitor with anti-inflammatory activity against multiple targets. Mediators Inflamm, 2012. 2012: p. 416036.
25. Krishnan, N., et al., The anti-inflammatory compound BAY-11-7082 is a potent inhibitor of protein tyrosine phosphatases. Febs j, 2013. 280(12): p. 2830-41.
26. M. Vass*, K.H., M. Franek, Nitrofuran antibiotics: a review on the application, prohibition and residual analysis. 2008: Veterinarni Medicina. p. 469-500.
27. Jackson, P.A., et al., Covalent Modifiers: A Chemical Perspective on the Reactivity of alpha, beta-Unsaturated Carbonyls with Thiols via Hetero-Michael Addition Reactions. J Med Chem, 2017. 60(3): p. 839-885.
28. McKenna, S., et al., Noncovalent interaction between ubiquitin and the human DNA repair protein Mms2 is required for Ubc13-mediated polyubiquitination. J Biol Chem, 2001. 276(43): p. 40120-6.
29. Hodge, C.D., L. Spyracopoulos, and J.N. Glover, Ubc13: the Lys63 ubiquitin chain building machine. Oncotarget, 2016. 7(39): p. 64471-64504.
30. Breccia, M. and G. Alimena, NF-kappaB as a potential therapeutic target in myelodysplastic syndromes and acute myeloid leukemia. Expert Opin Ther Targets, 2010. 14(11): p. 1157-76.
31. Pellagatti, A., et al., Gene expression profiles of CD34+ cells in myelodysplastic syndromes: involvement of interferon-stimulated genes and correlation to FAB subtype and karyotype. Blood, 2006. 108(1): p. 337-45.
32. Zhou, J., et al., Enhanced activation of STAT pathways and overexpression of survivin confer resistance to FLT3 inhibitors and could be therapeutic targets in AML. Blood, 2009. 113(17): p. 4052-62.
33. Xiang, Z., et al., Identification of somatic JAK1 mutations in patients with acute myeloid leukemia. Blood, 2008. 111(9): p. 4809-12.
34. Sato, T., et al., Interferon regulatory factor-2 protects quiescent hematopoietic stem cells from type I interferon-dependent exhaustion. Nat Med, 2009. 15(6): p. 696-700.
35. Essers, M.A., et al., IFNalpha activates dormant haematopoietic stem cells in vivo. Nature, 2009. 458(7240): p. 904-8.
36. Matatall, K.A., et al., Type II interferon promotes differentiation of myeloid-biased hematopoietic stem cells. Stem Cells, 2014. 32(11): p. 3023-30.
37. Li, Y., et al., Inflammatory signaling regulates embryonic hematopoietic stem and progenitor cell production. Genes Dev, 2014. 28(23): p. 2597-612.
38. Sawamiphak, S., Z. Kontarakis, and D.Y. Stainier, Interferon gamma signaling positively regulates hematopoietic stem cell emergence. Dev Cell, 2014. 31(5): p. 640-53.
39. Matatall, K.A., et al., Chronic Infection Depletes Hematopoietic Stem Cells through Stress-Induced Terminal Differentiation. Cell Rep, 2016. 17(10): p. 2584-2595.
40. Baldridge, M.T., et al., Quiescent haematopoietic stem cells are activated by IFN-gamma in response to chronic infection. Nature, 2010. 465(7299): p. 793-7.
41. MacNamara, K.C., et al., Infection-induced myelopoiesis during intracellular bacterial infection is critically dependent upon IFN-gamma signaling. J Immunol, 2011. 186(2): p. 1032-43.
42. Schurch, C.M., C. Riether, and A.F. Ochsenbein, Cytotoxic CD8+ T cells stimulate hematopoietic progenitors by promoting cytokine release from bone marrow mesenchymal stromal cells. Cell Stem Cell, 2014. 14(4): p. 460-72.
43. Chen, J., et al., IFN-gamma-mediated hematopoietic cell destruction in murine models of immune-mediated bone marrow failure. Blood, 2015. 126(24): p. 2621-31.
44. Fang, J., et al., TRAF6 Mediates Basal Activation of NF-kappaB Necessary for Hematopoietic Stem Cell Homeostasis. Cell Rep, 2018. 22(5): p. 1250-1262.
45. Benjamin, R., et al., Continuous delivery of human type I interferons (alpha/beta) has significant activity against acute myeloid leukemia cells in vitro and in a xenograft model. Blood, 2007. 109(3): p. 1244-7.
46. He, Y.F., et al., Sustained low-level expression of interferon-gamma promotes tumor development: potential insights in tumor prevention and tumor immunotherapy. Cancer Immunol Immunother, 2005. 54(9): p. 8917.
47. Aqbi, H.F., et al., IFN-gamma orchestrates tumor elimination, tumor dormancy, tumor escape, and progression. J Leukoc Biol, 2018.
48. Vuorela, M., K. Pylkas, and R. Winqvist, Mutation screening of the RNF8, UBC13 and MMS2 genes in Northern Finnish breast cancer families. BMC Med Genet, 2011. 12: p. 98.
49. Vallabhaneni, K.C., et al., Stromal cell extracellular vesicular cargo mediated regulation of breast cancer cell metastasis via ubiquitin conjugating enzyme E2 N pathway. Oncotarget, 2017. 8(66): p. 109861-109876.
50. Wu, Z., et al., Ubiquitin-conjugating enzyme complex Uev1A-Ubc13 promotes breast cancer metastasis through nuclear factor-small ka, CyrillicB mediated matrix metalloproteinase-1 gene regulation. Breast Cancer Res, 2014. 16(4): p. R75.
51. Wu, X., et al., Ubiquitin-conjugating enzyme Ubc13 controls breast cancer metastasis through a TAK1-p38 MAP kinase cascade. Proc Natl Acad Sci USA, 2014. 111(38): p. 13870-5.
52. Wu, Z., et al., Uev1A-Ubc13 promotes colorectal cancer metastasis through regulating CXCL1 expression via NF-small ka, CyrillicB activation. Oncotarget, 2018. 9(22): p. 15952-15967.
53. Zhang, E., et al., MicroRNA miR-147b promotes tumor growth via targeting UBE2N in hepatocellular carcinoma. Oncotarget, 2017. 8(69): p. 114072-114080.
54. Zhang, X., et al., The inhibition of UBC13 expression and blockage of the DNMTI-CHFR-Aurora A pathway contribute to paclitaxel resistance in ovarian cancer. Cell Death Dis, 2018. 9(2): p. 93.
55. Chang, J.H., et al., Ubc13 maintains the suppressive function of regulatory T cells and prevents their conversion into effector-like T cells. Nat Immunol, 2012. 13(5): p. 481-90.
56. Fukushima, T., et al., Ubiquitin-conjugating enzyme Ubc13 is a critical component of TNF receptor-associated factor (TRAF)-mediated inflammatory responses. Proc Natl Acad Sci U S A, 2007. 104(15): p. 6371-6.
57. Joo, E., et al., Ubc13 haploinsufficiency protects against age-related insulin resistance and high-fat diet-induced obesity. Sci Rep, 2016. 6: p. 35983.
58. Zhang, H., et al., Genetic rescue of lineage-balanced blood cell production reveals a crucial role for STAT3 antiinflammatory activity in hematopoiesis. Proc Natl Acad Sci USA, 2018. 115(10): p. E2311-e2319.
59. Baell, J. and M.A. Walters, Chemistry: Chemical con artists foil drug discovery. Nature, 2014. 513(7519): p. 481-3.
60. Matsuoka, A., et al., Lenalidomide induces cell death in an MDS-derived cell line with deletion of chromosome 5q by inhibition of cytokinesis. Leukemia, 2010. 24(4): p. 748-55.
61. Rhyasen, G.W., et al., An MDS xenograft model utilizing a patient-derived cell line. Leukemia, 2014. 28(5): p. 1142-5.
62. Kanehisa, M., et al., New approach for understanding genome variations in KEGG. Nucleic Acids Res, 2019. 47(D1): p. D590-d595.
63. Kanehisa, M., et al., KEGG: new perspectives on genomes, pathways, diseases and drugs. Nucleic Acids Res, 2017. 45(D1): p. D353-d361.
64. Kanehisa, M. and S. Goto, KEGG: kyoto encyclopedia of genes and genomes. Nucleic Acids Res, 2000. 28(1): p. 27-30.
65. Pfaffl, M.W., A new mathematical model for relative quantification in real-time RT-PCR. Nucleic Acids Research, 2001. 29(9).
66. Moffat, J., et al., A lentiviral RNAi library for human and mouse genes applied to an arrayed viral high-content screen. Cell, 2006. 124(6): p. 1283-98.
67. Schneider, C.A., W.S. Rasband, and K.W. Eliceiri, NIH Image to Imaged: 25 years of image analysis. Nat Methods, 2012. 9(7): p. 671-5.
68. Dobin, A., et al., STAR: ultrafast universal RNA-seq aligner. Bioinformatics, 2013. 29(1): p. 15-21.
69. Harrow, J., et al., GENCODE: the reference human genome annotation for The ENCODE Project. Genome Res, 2012. 22(9): p. 1760-74.
70. Liao, Y., G.K. Smyth, and W. Shi, featureCounts: an efficient general purpose program for assigning sequence reads to genomic features. Bioinformatics, 2014. 30(7): p. 923-30.
71. Li, B., et al., RNA-Seq gene expression estimation with read mapping uncertainty. Bioinformatics, 2010. 26(4): p. 493-500.
72. Love, M.I., W. Huber, and S. Anders, Moderated estimation of fold change and dispersion for RNA-seq data with DESeq2. Genome Biol, 2014. 15(12): p. 550.
73. Subramanian, A., et al., Gene set enrichment analysis: a knowledge-based approach for interpreting genome-wide expression profiles. Proc Natl Acad Sci U S A, 2005. 102(43): p. 15545-50.
74. Huang da, W., B.T. Sherman, and R.A. Lempicki, Systematic and integrative analysis of large gene lists using DAVID bioinformatics resources. Nat Protoc, 2009. 4(1): p. 44-57.
75. Huang da, W., B.T. Sherman, and R.A. Lempicki, Bioinformatics enrichment tools: paths toward the comprehensive functional analysis of large gene lists. Nucleic Acids Res, 2009. 37(1): p. 1-13.
76. Shannon, P., et al., Cytoscape: a software environment for integrated models of biomolecular interaction networks. Genome Res, 2003. 13(11): p. 2498-504.
77. Merico, D., et al., Enrichment map: a network-based method for gene-set enrichment visualization and interpretation. PLoS One, 2010. 5(11): p. e13984.
78. Kim, D., B. Langmead, and S.L. Salzberg, HISAT: a fast spliced aligner with low memory requirements. Nat Methods, 2015. 12(4): p. 357-60.
79. O'Leary, N.A., et al., Reference sequence (RefSeq) database at NCBI: current status, taxonomic expansion, and functional annotation. Nucleic Acids Res, 2016. 44(D1): p. D733-45.
80. Karolchik, D., et al., The UCSC Table Browser data retrieval tool. Nucleic Acids Res, 2004. 32(Database issue): p. D493-6.
81. Robinson, M.D., D.J. McCarthy, and G.K. Smyth, edgeR: a Bioconductor package for differential expression analysis of digital gene expression data. Bioinformatics, 2010. 26(1): p. 139-40.
82. McCarthy, D.J., Y. Chen, and G.K. Smyth, Differential expression analysis of multifactor RNA-Seq experiments with respect to biological variation. Nucleic Acids Res, 2012. 40(10): p. 4288-97.
83. Maza, E., In Papyro Comparison of TMM (edgeR), RLE (DESeq2), and MRN Normalization Methods for a Simple Two-Conditions-Without-Replicates RNA-Seq Experimental Design. Front Genet, 2016. 7: p. 164.
84. Maza, E., et al., Comparison of normalization methods for differential gene expression analysis in RNA-Seq experiments: A matter of relative size of studied transcriptomes. Commun Integr Biol, 2013. 6(6): p. e25849.
85. Will, B., et al., Stem and progenitor cells in myelodysplastic syndromes show aberrant stage-specific expansion and harbor genetic and epigenetic alterations, in Blood. 2012: United States. p. 2076-86.
86. Chutipongtanate, S. and K.D. Greis, Multiplex Biomarker Screening Assay for Urinary Extracellular Vesicles Study: A Targeted Label-Free Proteomic Approach. Sci Rep, 2018. 8(1): p. 15039.
87. Ritchie, M.E., et al., limma powers differential expression analyses for RNA-sequencing and microarray studies. Nucleic Acids Res, 2015. 43(7): p. e47.
88. Bindea, G., et al., ClueGO: a Cytoscape plug-in to decipher functionally grouped gene ontology and pathway annotation networks. Bioinformatics, 2009. 25(8): p. 1091-3.
89. Barreyro, L. et al., Inhibition of UBE2N As a Therapeutic Approach in Myelodysplastic Syndromes (MDS) and Acute Myeloid Leukemia (AML). Blood, 2016. 128(22):579.
90. Barreyro, L. et al., Therapeutic Targeting of the Ubiquitin Conjugating Enzyme UBE2N in Myeloid Malignancies. Blood, 2018. 132:4050.

Aspects of the invention will now be described with reference to the following numbered clauses:
1. A method of treating a subtype of acute myeloid leukemia (AML) responsive to UBE2N inhibition, the method comprising:
   identifying a subject having one or more subtype of AML responsive to UBE2N inhibition, wherein the AML subtype comprises acute myelomonocytic leukemia (AML-M4) and/or acute monocytic leukemia (AML-M5); and
   providing to the subject one or more administrations of one or more compositions comprising a UBE2N inhibitor; and
   wherein administration of the UBE2N inhibitor results in treating the subtype of acute myeloid leukemia (AML) responsive to UBE2N inhibition in the subject.
2. The method of clause 1, wherein treating comprises modulating UBE2N-mediated immune signaling.
3. The method of clause 1, wherein the AML subtype comprises AML-M4.
4. The method of clause 1, wherein the AML subtype comprises AML-M5.
5. The method of any of clauses 1-4, wherein the UBE2N inhibitor is a small molecule.
6. The method of any of clauses 1-5, wherein the UBE2N inhibitor is at least one selected from the group consisting of NSC697923 (2-(4-methylphenyl)sulfonyl-5-nitrofuran), UC-764864 (1-(4-ethylphenyl)-3-[(6-methyl-1H-benzimidazol-2-yl) sulfanyl]prop-2-en-1-one), UC-764865 (1-(4-methoxyphenyl)-3-[(6-methyl-1H-benzimidazol-2-yl) sulfanyl]prop-2-en-1-one), and UC-764865 (1-(4-methylphenyl)-3-[(6-methyl-1H-benzimidazol-2-yl) sulfanyl]prop-2-en-1-one), and pharmaceutically-acceptable salts, cocrystals, hydrates, solvates, optical isomers, geometric isomers, salts of isomers, prodrugs, and derivatives thereof.
7. The method of any of clauses 1-6, wherein administration of the UBE2N inhibitor to the subject decreases the incidence of one or more symptoms associated with AML-M4 and/or AML-M5 or decreases one or more markers of viability of AMI,-M4 and/or AML-M5 cells.
8. The method of clause 7, wherein the one or more symptoms associated with AML-M4 and/or AML-M5 comprises decreasing marrow failure, immune dysfunction, transformation to overt leukemia, or a combination thereof in the subject, or wherein the marker of viability of AML-M4 and/or AML-M5 cells comprises survival over time, proliferation, growth, migration, formation of colonies, chromatic assembly, DNA binding, RNA metabolism, cell migration, cell adhesion, inflammation, or a combination of two or more thereof.
9. The method of any of clauses 1-8, wherein the method further comprises administration of a composition comprising a BCL2 inhibitor.
10. The method of clause 9, wherein the BCL2 inhibitor comprises venetoclax, or a salt, isomer, derivative or analog thereof.
11. The method of any of clauses 9-10, wherein the administration of a composition comprising a BCL2 inhibitor occurs concurrently with or after administration of the UBE2N inhibitor.
12. The method of any of clauses 1-11, wherein the subject has been treated previously with one or more BCL2 inhibitor.
13. The method of any of clauses 1-12, wherein administration of the UBE2N inhibitor resensitizes the subject to the BCL2 inhibitor and/or otherwise enhances the effectiveness of the administration of the BCL2 inhibitor.
14. The method of any of clauses 1-13, wherein the method further comprises administration of one or more chemotherapy, and/or one or more apoptotic agent, immune modulating agent, and/or epigenetic modifying agent.
15. The method of clause 14, wherein the chemotherapy comprises one or more selected from the group consisting of a taxane, a platinum-based agent, an anthracycline, an alkylating agent, a vinca alkaloid, an epothilone, a histone deacetylase inhibitor, a topoisomerase I and II inhibitor, a kinase inhibitor, a nucleotide analog, a precursor analog, a peptide antibiotic, and combinations thereof.
16. The method of any of clauses 1-15, wherein the method further comprises administration of a CUL4-CRBN E3 ligase complex inhibitor.
17. The method of clause 16, wherein the CUL4-CRBN E3 ligase complex inhibitor comprises lenalidomide.
18. The method of any of clauses 1-17, wherein at least one of the one or more administrations comprises parenteral administration, a mucosal administration, intravenous administration, subcutaneous administration, topical administration, intradermal administration, oral administration, sublingual administration, intranasal administration, or intramuscular administration.
19. The method of any of clauses 1-18, wherein if there is more than one administration at least one composition used for at least one administration is different from the composition of at least one other administration.
20. The method of any of clauses 1-19, wherein the compound of at least one of the one or more compositions is administered to the subject in an amount of from about 0.005 mg/kg animal body weight to about 50 mg /kg animal body weight.
21. The method of any of clauses 1-20, wherein the subject is a mammal, preferably wherein the subject is a human, a rodent, or a primate.
22. The method of any of clauses 1-21, wherein the subject is enrolled in a clinical trial.
23. A method of identifying a subject having acute myeloid leukemia (AML) suitable for treatment with a UBE2N inhibitor, the method comprising:
   determining whether the subject has one or more subtype of AML responsive to UBE2N inhibition, wherein the AML subtype comprises acute myelomonocytic leukemia (AML-M4) and/or acute monocytic leukemia (AML-M5);
   assigning the subject to a first treatment cohort where the subject has an AML subtype comprising AML-M4 and/or AMI,-M5, wherein the first treatment cohort is treatable by administration of an UBE2N inhibitor, or assigning the subject to a second treatment cohort where the subject does not have an AML subtype comprising AML-M4 and/or AML-M5, wherein the second treatment cohort is not treatable, or is less effectively treatable by administration of an UBE2N inhibitor.
24. The method of clause 23, wherein determining whether the subject has one or more subtype of AML responsive to UBE2N inhibition comprises obtaining a sample from the subject, and analyzing the sample to determine whether the subject has AML-M4 or AML-M5.
25. The method of clause 23 or 24, wherein the AML subtype comprises AML-M4.
26. The method of clause 23 or 24, wherein the AML subtype comprises AML-M5.
27. The method of any of clauses 23-26, further comprising treating the subject with a UBE2N inhibitor if the subject has an AML subtype comprising AML-M4 and/or AML-M5, or treating the subject with a therapy excluding a UBE2N inhibitor if the subject does not have an AML subtype comprising AML-M4 and/or AML-M5.
28. The method of any of clauses 23-27, wherein the UBE2N inhibitor is a small molecule.
29. The method of any of clauses 23-28, wherein the UBE2N inhibitor is at least one selected from the group consisting of NSC697923 (2-(4-methylphenyl)sulfonyl-5-nitrofuran), UC-764864 (1-(4-ethylphenyl)-3-[(6-methyl-1H-benzimidazol-2-yl) sulfanyl]prop-2-en-1-one), UC-764865 (1-(4-methoxyphenyl)-3-[(6-methyl-1H-benzimidazol-2-yl) sulfanyl]prop-2-en-1-one), and UC-764865 (1-(4-methylphenyl)-3-[(6-methyl-1H-benzimidazol-2-yl) sulfanyl]prop-2-en-1-one), and pharmaceutically-acceptable salts, cocrystals, hydrates, solvates, optical isomers, geometric isomers, salts of isomers, prodrugs, and derivatives thereof.
30. The method of any of clauses 23-29, wherein administration of the UBE2N inhibitor to the subject decreases the incidence of one or more symptoms associated with AML-M4 and/or AML-M5 or decreases one or more markers of viability of AML-M4 and/or AML-M5 cells.
31. The method of clause 30, wherein the one or more symptoms associated with AML-M4 and/or AML-M5 comprises decreasing marrow failure, immune dysfunction, transformation to overt leukemia, or a combination thereof in the subject, or wherein the marker of viability of AML-M4 and/or AML-M5 cells comprises survival over time, proliferation, growth, migration, formation of colonies, chromatic assembly, DNA binding, RNA metabolism, cell migration, cell adhesion, inflammation, or a combination of two or more thereof.
32. The method of any of clauses 23-31, wherein the method further comprises administration of a composition comprising a BCL2 inhibitor.
33. The method of clause 32, wherein the BCL2 inhibitor comprises venetoclax, or a salt, isomer, derivative or analog thereof.
34. The method of any of clauses 32-33, wherein the administration of a composition comprising a BCL2 inhibitor occurs concurrently with or after administration of the UBE2N inhibitor.
35. The method of any of clauses 23-34, wherein the subject has been treated previously with one or more BCL2 inhibitor.
36. The method of any of clauses 23-35, wherein administration of the UBE2N inhibitor resensitizes the subject to the BCL2 inhibitor and/or otherwise enhances the effectiveness of the administration of the BCL2 inhibitor.
37. The method of any of clauses 23-36, wherein the method further comprises administration of one or more chemotherapy, and/or one or more apoptotic agent, immune modulating agent, and/or epigenetic modifying agent.
38. The method of clause 37, wherein the chemotherapy comprises one or more selected from the group consisting of a taxane, a platinum-based agent, an anthracycline, an alkylating agent, a vinca alkaloid, an epothilone, a histone deacetylase inhibitor, a topoisomerase I and II inhibitor, a kinase inhibitor, a nucleotide analog, a precursor analog, a peptide antibiotic, and combinations thereof.
39. The method of any of clauses 23-38, wherein the method further comprises administration of a CUL4-CRBN E3 ligase complex inhibitor.
40. The method of clause 16, wherein the CUL4-CRBN E3 ligase complex inhibitor comprises lenalidomide.
41. The method of any of clauses 23-40, wherein at least one of the one or more administrations comprises parenteral administration, a mucosal administration, intravenous administration, subcutaneous administration, topical administration, intradermal administration, oral administration, sublingual administration, intranasal administration, or intramuscular administration.
42. The method of any of clauses 23-41, wherein if there is more than one administration at least one composition used for at least one administration is different from the composition of at least one other administration.
43. The method of any of clauses 23-42, wherein the compound of at least one of the one or more compositions is administered to the subject in an amount of from about 0.005 mg/kg animal body weight to about 50 mg /kg animal body weight.
44. The method of any of clauses 23-43, wherein the subject is a mammal, preferably wherein the subject is a human, a rodent, or a primate.
45. The method of any of clauses 23-44, wherein the subject is enrolled in a clinical trial.
46. A method of treating a chronic inflammatory condition responsive to UBE2N inhibition, the method comprising:
   identifying a subject having one or more chronic inflammatory condition responsive to UBE2N inhibition; and
   providing to the subject one or more administrations of one or more compositions comprising a UBE2N inhibitor; and
   wherein administration of the UBE2N inhibitor results in treating the chronic inflammatory condition responsive to UBE2N inhibition in the subject.
47. A method of treating a hematologic malignancy and/or solid tumor responsive to UBE2N inhibition, the method comprising:
   identifying a subject having a hematologic malignancy and/or solid tumor responsive to UBE2N inhibition; and
   providing to the subject one or more administrations of one or more compositions comprising a UBE2N inhibitor; and
   wherein administration of the UBE2N inhibitor results in treating the hematologic malignancy and/or solid tumor responsive to UBE2N inhibition in the subject.
48. The method of clause 47, wherein the disease or disorder comprises diffuse large B cell lymphoma, neuroblastoma, breast cancer, metastatic colorectal cancer, hepatocarcinoma, ovarian cancer, breast cancer, cervical cancer, colorectal cancer, endometrial cancer, glioma, head and neck cancer, liver cancer, melanoma, ovarian cancer, pancreatic cancer, prostate cancer, renal cancer, stomach cancer, testicular cancer, thyroid cancer, or urothelial cancer, or a combination of two or more thereof.

## Claims

1. One or more compositions comprising a UBE2N inhibitor for use in a method of treating a hematologic malignancy and/or solid tumor responsive to UBE2N inhibition or a chronic inflammatory condition responsive to UBE2N inhibition, the method comprising:
identifying a subject having a hematologic malignancy and/or solid tumor responsive to UBE2N inhibition or a chronic inflammatory condition responsive to UBE2N inhibition or; and
providing to the subject one or more administrations of the one or more compositions comprising a UBE2N inhibitor;
wherein administration of the UBE2N inhibitor results in treating the hematologic malignancy and/or solid tumor responsive to UBE2N inhibition or the chronic inflammatory condition responsive to UBE2N inhibition in the subject; and
wherein the UBE2N inhibitor comprises an RNAi sufficient to inhibit UBE2N expression or is at least one selected from the group consisting of NSC697923 (2-(4-methylphenyl)sulfonyl-5-nitrofuran), UC-764864 (l-(4-ethylphenyl)-3-[(6-methyl- lH-benzimidazol-2-yl) sulfanyl]prop-2-en-lone), UC-764865 (l-(4-methoxyphenyl)-3-[(6-methyl-lH-benzimidazol-2-yl) sulfanyl]prop-2-en-lone), and l-(4-methylphenyl)-3-[(6-methyl-lH-benzimidazol-2-yl) sulfanyl]prop-2-en-l-one, and pharmaceutically-acceptable salts, cocrystals, hydrates, solvates, optical isomers, geometric isomers, and salts of isomers thereof.

2. The one or more compositions comprising a UBE2N inhibitor for use of claim 1, wherein the hematologic malignancy and/or solid tumor comprises diffuse large B cell lymphoma, neuroblastoma, breast cancer, metastatic colorectal cancer, hepatocarcinoma, ovarian cancer, breast cancer, cervical cancer, colorectal cancer, endometrial cancer, glioma, head and neck cancer, liver cancer, melanoma, ovarian cancer, pancreatic cancer, prostate cancer, renal cancer, stomach cancer, testicular cancer, thyroid cancer, or urothelial cancer, or a combination of two or more thereof.

3. The one or more compositions for use of claim 1 or 2, wherein the hematologic malignancy and/or solid tumor is diffuse large B cell lymphoma.

4. The one or more compositions for use of any of claims 1-3, wherein the UBE2N inhibitor is at least one selected from the group consisting of NSC697923 (2-(4-methylphenyl)sulfonyl-5-nitrofuran), UC-764864 (l-(4-ethylphenyl)-3-[(6-methyl- lH-benzimidazol-2-yl) sulfanyl]prop-2-en-lone), UC-764865 (l-(4-methoxyphenyl)-3-[(6-methyl-lH-benzimidazol-2-yl) sulfanyl]prop-2-en-lone), and l-(4-methylphenyl)-3-[(6-methyl-lH-benzimidazol-2-yl) sulfanyl]prop-2-en-l-one, and pharmaceutically-acceptable salts, cocrystals, hydrates, solvates, optical isomers, geometric isomers, and salts of isomers thereof.

5. The one or more compositions for use of any of claims 1-4, wherein the UBE2N inhibitor is UC-764864 (l-(4-ethylphenyl)-3-[(6-methyl-lH-benzimidazol-2-yl) sulfanyl]prop-2-en-l-one) or UC-764865 (l-(4-methoxyphenyl)-3-[(6-methyl-lH-benzimidazol-2-yl) sulfanyl]prop-2-en-l-one).

6. The one or more compositions for use of any of claims 1-5, wherein at least one of the one or more administrations comprises parenteral administration, a mucosal administration, intravenous administration, subcutaneous administration, topical administration, intradermal administration, oral administration, sublingual administration, intranasal administration, or intramuscular administration.

7. The one or more compositions for use of any of claims 1-6, wherein if there is more than one administration at least one composition used for at least one administration is different from the composition of at least one other administration.

8. The one or more compositions for use of any of claims 1-7, wherein the compound of at least one of the one or more compositions is administered to the subject in an amount of from about 0.005 mg/kg animal body weight to about 50 mg/kg animal body weight.

9. The one or more compositions for use of any of claims 1-8, wherein the subject is a mammal, preferably wherein the subject is a human, a rodent, or a primate.

10. The one or more compositions for use of any of claims 1-9, wherein the subject is enrolled in a clinical trial.

11. The one or more compositions for use of any of claims 1-10, wherein the method further includes administration of one or more chemotherapy, and/or one or more apoptotic agent, immune modulating agent, and/or epigenetic modifying agent.

12. The one or more compositions for use of claim 11, wherein the chemotherapy includes one or more selected from the group consisting of a taxane, a platinum-based agent, an anthracycline, an alkylating agent, a vinca alkaloid, an epothilone, a histone deacetylase inhibitor, a topoisomerase I and II inhibitor, a kinase inhibitor, a nucleotide analog, a precursor analog, a peptide antibiotic, and combinations thereof.


SEQUENCE LISTING

<110> Starczynowski, Daniel
      Barreyro, Laura
      Seibel, William

<120> Rational Therapeutic Targeting of Oncogenic Immune Signaling
States in Myeloid Malignancies via the Ubiquitin Conjugating Enzyme
UBE2N

<130> 0088544-048WO0

<150> 62861711
<151> 2019-06-14

<160> 34

<170> FastSEQ for Windows Version 4.0

<210> 1
<211> 22
<212> RNA
<213> Artificial Sequence

<220>
<223> UBE2N forward primer sequence

<400> 1
tgatgtagcg gagcagtgga ag                                          22

<210> 2
<211> 22
<212> RNA
<213> Artificial Sequence

<220>
<223> UBE2N reverse primer sequence

<400> 2
ggaggaagtc ttggcagaac ag                                                22

<210> 3
<211> 21
<212> RNA
<213> Artificial Sequence

<220>
<223> FOS forward primer sequence

<400> 3
ccggggatag cctctcttac t                                                 21

<210> 4
<211> 19
<212> RNA
<213> Artificial Sequence

<220>
<223> FOS reverse primer sequence

<400> 4
ccaggtccgt gcagaagtc                                                    19

<210> 5
<211> 21
<212> RNA
<213> Atificial Sequence

<220>
<223> JUN forward primer sequence

<400> 5
tccaagtgcc gaaaaaggaa g                                                 21

<210> 6
<211> 21

<212> RNA
<213> Artificial Sequence

<220>
<223> JUN reverse primer sequence

<400> 6
cgagttctga gctttcaagg t                                                21

<210> 7
<211> 21
<212> RNA
<213> Artificial Sequence

<220>
<223> JUNB forward primer sequence

<400> 7
acaaactcct gaaaccgagc c                                                21

<210> 8
<211> 21
<212> RNA
<213> Artificial Sequence

<220>
<223> JUNB reverse primer sequence

<400> 8
cgagccctga ccagaaaagt a                                                21

<210> 9
<211> 21
<212> RNA
<213> Artificial Sequence

<220>
<223> IL6 forward primer sequence

<400> 9
ggtacatcct cgacggcatc t                                                21

<210> 10
<211> 21
<212> RNA
<213> Artificial Sequence

<220>
<223> IL6 reverse primer sequence

<400> 10
gtgcctcttt gctgctttca c                                                21

<210> 11
<211> 23
<212> RNA
<213> Artificial Sequence

<220>
<223> IL1B forward primer sequence

<400> 11
aatctgtacc tgtcctgcgt gtt                                              23

<210> 12
<211> 26
<212> RNA
<213> Artificial Sequence

<220>
<223> IL1B reverse primer sequence

<400> 12
tgggtaattt ttgggatcta cactct                                           26

<210> 13

<211> 20

<212> RNA

<213> Artificial Sequence

<220>

<223> TNF forward primer sequence

<400> 13

tcttctcgaa ccccgagtga                                                    20

<210> 14

<211> 19

<212> RNA

<213> rArtificial Sequence

<220>

<223> TNF reverse primer sequence

<400> 14

cctctgatgg caccaccag                                                     19

<210> 15

<211> 20

<212> RNA

<213> Artificial Sequence

<220>

<223> Actin forward primer sequence

<400> 15

ctcttccagc cttccttcct                                                    20

<210> 16

<211> 20

<212> RNA

<213> Artificial Sequence

<220>

<223> Actin reverse primer sequence

<400> 16
agcactgtgt tggcgtacag                                                    20

<210> 17
<211> 58
<212> RNA
<213> Artificial Sequence

<220>
<223> UBE2N template oligonucleotide shUBE2N-2

<400> 17
ccggctaggc tatatgccat gaatactcga gtattcatgg catatagcct agtttttg   58

<210> 18
<211> 58
<212> RNA
<213> Artificial Sequence

<220>
<223> UBE2N template oligonucleotide shUBE2N-3

<400> 18
ccggagacaa gttgggaaga atatgctcga gcatattctt cccaacttgt ctttttg   58

<210> 19
<211> 57
<212> RNA
<213> Artificial Sequence

<220>
<223> UBE2N template oligonucleotide shUBE2N-1

<400> 19
ccgggctgag gcatttgtga gtcttctcga gaagactcac aaatgcctca gcttttt   57

<210> 20

<211> 57

<212> RNA

<213> Artificial Sequence

<220>

<223> Non-silencing control shControl

<400> 20

ccggcaacaa gatgaagagc accaactcga gttggtgctc ttcatcttgt tgttttt    57

<210> 21

<211> 11

<212> PRT

<213> Artificial Sequence

<220>

<223>  UBE2N  Ub  conjugation  peptide  with  ubiquinated  diglycine  (UGG)
motif

<400> 21

Ile Tyr His Pro Asn Val Asp Lys Leu Gly Arg
 1               5                   10

<210> 22

<211> 12

<212> PRT

<213> Artificial Sequence

<220>

<223>  UBE2N  Ub  conjugation  peptide  with  ubiquinated  diglycine  (UGG)
motif

<400> 22

Phe Met Thr Lys Ile Tyr His Pro Asn Val Asp Lys
 1               5                   10

<210> 23
<211> 10
<212> PRT
<213> Artificial Sequence

<220>
<223> UBE2N Ub conjugation peptide with ubiquinated diglycine (UGG)
motif

<400> 23
Asp Lys Trp Ser Pro Ala Leu Gln Ile Arg
1               5                   10

<210> 24
<211> 12
<212> PRT
<213> Artificial Sequence

<220>
<223> UBE2N Ub conjugation peptide with ubiquinated diglycine (UGG)
motif

<400> 24
Ile Tyr His Pro Asn Val Asp Lys Leu Gly Arg Ile
1               5                   10

<210> 25
<211> 12
<212> PRT
<213> Artificial Sequence

<220>
<223> UBE2N Ub conjugation peptide with ubiquinated diglycine (UGG)
motif

<400> 25
Phe Met Thr Lys Ile Tyr His Pro Asn Val Asp Lys
1               5                   10

<210> 26
<211> 12
<212> PRT
<213> Artificial Sequence

<220>
<223> UBE2N Ub conjugation peptide with ubiquinated diglycine (UGG)
motif

<400> 26
Val Ser Phe Glu Leu Phe Ala Asp Lys Val Pro Lys
1               5                   10

<210> 27
<211> 13
<212> PRT
<213> Artificial Sequence

<220>
<223> UBE2N Ub conserved site peptide with ubiquinated diglycine (UGG)
motif

<400> 27
Cys Cys Leu Thr Tyr Cys Phe Asn Lys Pro Glu Asp Lys
1               5                   10

<210> 28
<211> 16
<212> PRT
<213> Artificial Sequence

<220>
<223> UBE2N Ub conserved site peptide with ubiquinated diglycine (UGG)
motif

<400> 28
Thr Leu Glu Val Glu Pro Ser Asp Thr Ile Glu Asn Val Lys Ala Lys
1               5                   10                  15

<210> 29
<211> 13
<212> PRT
<213> Artificial Sequence

<220>
<223> UBE2N Ub conserved site peptide with ubiquinated diglycine (UGG)
motif

<400> 29
Leu Lys Tyr Tyr Lys Val Asp Glu Asn Gly Lys Ser Arg
1               5                   10

<210> 30
<211> 13
<212> PRT
<213> Artificial Sequence

<220>
<223> UBE2N Ub-protein transferase activity peptide with ubiquinated
diglycine (UGG) motif

<400> 30
Leu Val Cys Pro Asp Glu Gly Phe Tyr Lys Ser Gly Lys
1               5                   10

<210> 31

<211> 9
<212> PRT
<213> Artificial Sequence

<220>
<223> UBE2N Ub-like protein ligase binding site with ubiquinated
diglycine (UGG) motif

<400> 31
His Thr Pro Leu Ser Lys Leu Met Lys
1               5

<210> 32
<211> 18
<212> PRT
<213> Artificial Sequence

<220>
<223> UBE2N Ub-like protein ligase binding site with ubiquinated
diglycine (UGG) motif

<400> 32
Leu Ile Tyr Ser Gly Lys Gln Met Asn Asp Glu Lys Thr Ala Ala Asp
1               5                   10                  15
Tyr Lys

<210> 33
<211> 18
<212> PRT
<213> Artificial Sequence

<220>
<223> UBE2N Ub-like protein ligase binding site with ubiquinated
diglycine (UGG) motif

<400> 33

Leu Ile Tyr Ser Gly Lys Gln Met Asn Asp Glu Lys Thr Ala Ala Asp
1               5                   10                  15
Tyr Lys

<210> 34
<211> 11
<212> PRT
<213> Artificial Sequence

<220>
<223> UBE2N Ub interacting motif with ubiquinated diglycine (UGG)
motif

<400> 34
Cys Asn Tyr Lys Glu His Trp Phe Thr Val Arg
1               5                   10